# EUROPEAN PATENT SPECIFICATION

(11) **EP 4 291 679 B1**
(45) Date of publication and mention of the grant of the patent: **04.02.2026**
(21) Application number: 22707955.5
(22) Date of filing: 11.02.2022
(51) Int. Cl.: C12Q 1/6827, C12Q 1/6844

(54) **METHODS FOR DETECTING NUCLEIC ACID VARIANTS**
VERFAHREN ZUM NACHWEIS VON NUKLEINSÄUREVARIANTEN
PROCÉDÉS POUR LA DÉTECTION DE VARIANTS D'ACIDE NUCLÉIQUE

(30) Priority: 12.02.2021 US 202163149182 P
(43) Date of publication of application: 20.12.2023
(60) Divisional application: 25223710.2
(73) Proprietor: Guardant Health, Inc., Palo Alto, CA 94304 (US)
(72) Inventor: KENNEDY, Andrew, Redwood City, California 94063 (US)
(74) Representative: J A Kemp LLP
(86) International application number: PCT/US2022/016224
(87) International publication number: WO 2022/174109

(56) References cited:
- WO-A1-2008/144841
- WO-A1-2018/005983
- METZLER M ET AL: "Asymmetric multiplex-polymerase chain reaction - a high throughput method for detection and sequencing genomic fusion sites in t(4;11)", BRITISH JOURNAL OF HAEMATOLOGY, JOHN WILEY, HOBOKEN, USA, vol. 124, no. 1, 11 December 2003 (2003-12-11), pages 47 - 54, XP071119992, ISSN: 0007-1048, DOI: 10.1046/J.1365-2141.2003.04740.X

## Description

### FIELD OF THE INVENTION

The present disclosure provides methods related to analyzing DNA, such as cell-free DNA. In some embodiments, the DNA is from a subject having or suspected of having cancer and/or the DNA includes DNA from cancer cells. In some embodiments, DNA rearrangements, such as translocations, are detected using a method comprising primer annealing and primer extension by an 5' to 3' exonuclease negative, strand displacement negative DNA polymerase, and the primer-extended products are identified.

### INTRODUCTION

Cancer is responsible for millions of deaths per year worldwide. Early detection of cancer may result in improved outcomes because early-stage cancer tends to be more susceptible to treatment.

Improperly controlled cell growth is a hallmark of cancer. Cancer is usually caused by the accumulation of mutations within an individual's normal cells, at least some of which result in improperly regulated cell division. Such mutations commonly include single nucleotide variations (SNVs), gene fusions, insertions and deletions (indels), transversions, translocations, and inversions. Cancers may also exhibit an accumulation of epigenetic changes, such as including modification of cytosine (e.g., 5-methylcytosine, 5-hydroxymethylcytosine, and other more oxidized forms) and association of DNA with chromatin proteins and transcription factors.

Biopsies represent a traditional approach for detecting or diagnosing cancer in which cells or tissue are extracted from a possible site of cancer and analyzed for relevant phenotypic and/or genotypic features. Biopsies have the drawback of being invasive.

Detection of cancer based on analysis of body fluids ("liquid biopsies"), such as blood, is an intriguing alternative based on the observation that DNA from cancer cells is released into body fluids. A liquid biopsy is noninvasive (sometimes requiring only a blood draw). However, it has been challenging to develop accurate and sensitive methods for analyzing liquid biopsy material because the amount of nucleic acids released into body fluids is low and variable as is recovery of nucleic acids from such fluids in analyzable form. These sources of variation can obscure predictive value of mutations (e.g., rearrangements, such as translocations and indels) among samples. Such mutations may include biomarkers that can be used to evaluate whether a subject diagnosed with, or suspected of having signs of, a cancer will benefit from a specific type of cancer therapy, such as Immuno-Oncology (I-O) therapy. Isolating and processing cell-free DNA useful for further analysis in liquid biopsy procedures is an important part of these methods. Accordingly, there is a need for improved methods and compositions for analyzing cell-free DNA, e.g., in liquid biopsies.

WO 2018/005983 discloses compositions and methods for detection of nucleic acid mutations. WO 2008/144841 discloses a method for DNA breakpoint analysis. Metzler, M., et al. (British journal of haematology 124.1 (2004): 47-54) discloses asymmetric multiplex-polymerase chain reaction as a high throughput method for detection and sequencing specific genomic fusion sites.

### SUMMARY OF THE INVENTION

DNA breakpoints can vary; therefore, effective methods of detecting rearrangements in DNA should capture as many breakpoints as possible. Existing methods may not be selective for rearrangements and may result in significant detection of wild type DNA and thus poor signal to noise ratios. The methods herein comprise selective extension of primers annealed to DNA comprising a rearrangement and detection of the primer-extended products. Thus, unlike existing methods, methods according to this disclosure can provide selective detection of DNA rearrangements over wild type DNA and specific detection of rearrangements in a target region of the genome.

The methods herein can also provide combined information about DNA rearrangements and other modifications, including but not limited to sequence variations.

The present disclosure aims to meet the need for improved analysis of DNA comprising a rearrangement, such as cell-free DNA.
Specifically the invention provides a method of analyzing DNA in a sample, the DNA comprising intronic regions, the method comprising:
a) contacting the DNA with a plurality of primers, wherein the plurality of primers comprises at least two primers that anneal in a parallel orientation to a target region, thereby producing primer-annealed DNA; and
b) contacting the primer-annealed DNA with a DNA polymerase and deoxynucleoside triphosphates, wherein the DNA polymerase is a 5' to 3' exonuclease negative, strand displacement negative DNA polymerase, thereby producing a primer-extended sample, wherein for at least half of the primers, when annealed to a wild type target region, extension is blocked by a downstream primer; and
c) detecting a presence or absence of a DNA molecule that comprises a structural variation, wherein the detecting comprises sequencing DNA in the primer-extended sample.

### BRIEF DESCRIPTION OF THE DRAWINGS

FIG. 1A illustrates an exemplary workflow according to certain embodiments of the disclosure beginning with cfDNA isolated from a sample and ligated to adapters; then contacted with primers that anneal to a target region, a 5' to 3' exonuclease negative and strand displacement negative DNA polymerase, and deoxynucleoside triphosphates comprising a low percentage of a biotinylated deoxynucleoside triphosphate; then contacted with streptavidin coated magnetic beads. The workflow results in an enriched sample of cfDNA comprising a structural variation breakpoint.

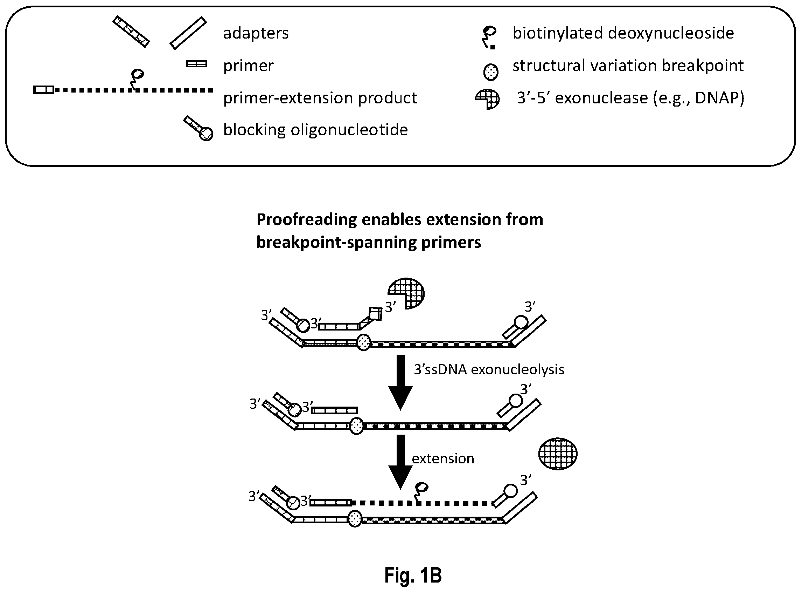

FIG. 1B illustrates an exemplary workflow according to certain embodiments of the disclosure wherein a primer anneals to a sequence comprising a structural variation breakpoint such that the 3' terminus of the primer is not complementary and is a substrate for exonucleolysis by a 3'-5' exonuclease, e.g., of a DNA polymerase. Following exonucleolysis, the primer is extended and a biotinylated deoxynucleoside is incorporated, facilitating capture. Blocking oligonucleotides are annealed to the 3' adapter to protect it from degradation and to the 5' adapter to prevent extension of a primer across the adapter sequence.

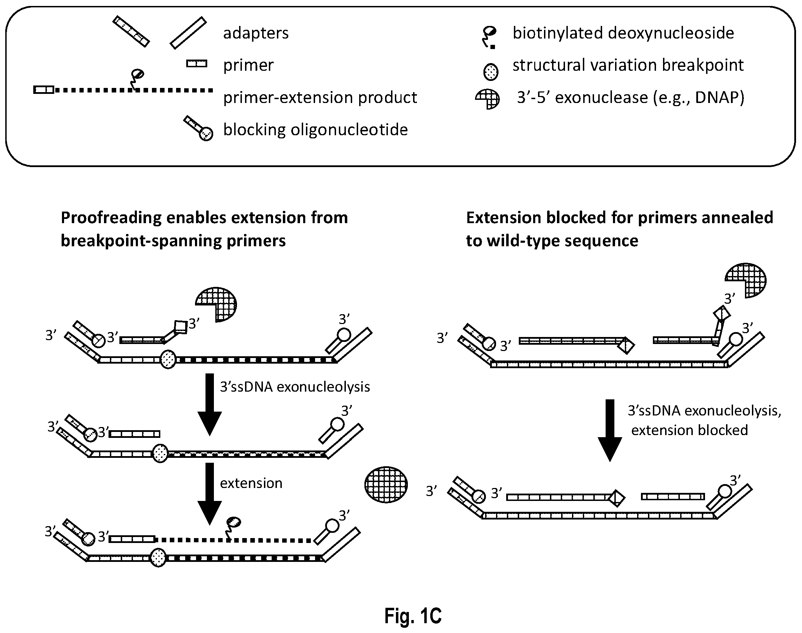

FIG. 1C illustrates an exemplary workflow according to certain embodiments of the disclosure wherein a primer anneals to a sequence comprising a structural variation breakpoint such that the 3' terminus of the primer is not complementary and is a substrate for exonucleolysis by a 3'-5' exonuclease, e.g., of a DNA polymerase. Following exonucleolysis, the primer is extended and a biotinylated deoxynucleoside is incorporated, facilitating capture. Blocking oligonucleotides are annealed to the 3' adapter to protect it from degradation and to the 5' adapter to prevent extension of a primer across the adapter sequence. A primer that anneals to a wild-type sequence near the 5' adapter may undergo 3' exonucleolysis but extension is blocked by the blocking oligonucleotide.
FIG. 2 is a schematic diagram of an example of a system suitable for use with some embodiments of the disclosure.
FIG. 3 is an exemplary workflow according to certain embodiments of the disclosure.
FIG. 4 illustrates primer and blocking oligonucleotide design according to certain embodiments of the disclosure.
FIG. 5 illustrates exemplary steps according to certain embodiments of the disclosure.

### DETAILED DESCRIPTION OF CERTAIN EMBODIMENTS

The technical information set out below may in some respects go beyond the disclosure of the invention, which is defined exclusively by the appended claims. The additional technical information is provided to place the actual invention in a broader technical context and to illustrate possible related technical developments. Such additional technical information which does not fall within the scope of the appended claims, is not part of the invention.

Reference will now be made in detail to certain embodiments of the invention. While the invention will be described in conjunction with such embodiments, it will be understood that they are not intended to limit the invention to those embodiments. The invention as defined by the appended claims.

Before describing the present teachings in detail, it is to be understood that the disclosure is not limited to specific compositions or process steps, as such may vary. It should be noted that, as used in this specification and the appended claims, the singular form "a", "an" and "the" include plural references unless the context clearly dictates otherwise. Thus, for example, reference to "a nucleic acid" includes a plurality of nucleic acids, reference to "a cell" includes a plurality of cells, and the like.

Numeric ranges are inclusive of the numbers defining the range. Measured and measurable values are understood to be approximate, taking into account significant digits and the error associated with the measurement. Also, the use of "comprise", "comprises", "comprising", "contain", "contains", "containing", "include", "includes", and "including" are not intended to be limiting. It is to be understood that both the foregoing general description and detailed description are exemplary and explanatory only and are not restrictive of the teachings.

Unless specifically noted in the above specification, embodiments in the specification that recite "comprising" various components are also contemplated as "consisting of" or "consisting essentially of" the recited components; embodiments in the specification that recite "consisting of" various components are also contemplated as "comprising" or "consisting essentially of" the recited components; and embodiments in the specification that recite "consisting essentially of" various components are also contemplated as "consisting of" or "comprising" the recited components (this interchangeability does not apply to the use of these terms in the claims).

The section headings used herein are for organizational purposes and are not to be construed as limiting the disclosed subject matter in any way. In the event that any document or other material referenced contradicts any explicit content of this specification, including definitions, this specification controls.

### I. Definitions

"Cell-free DNA," "cfDNA molecules," or simply "cfDNA" include DNA molecules that naturally occur in a subject in extracellular form (e.g., in blood, serum, plasma, or other bodily fluids such as lymph, cerebrospinal fluid, urine, or sputum). While the cfDNA previously existed in a cell or cells in a large complex biological organism, e.g., a mammal, it has undergone release from the cell(s) into a fluid found in the organism, and may be obtained from a sample of the fluid without the need to perform an in vitro cell lysis step. cfDNA molecules may occur as DNA fragments.

As used herein, "primer-annealed DNA," when referring to primers that anneal to at least one target region, means DNA molecules annealed to primers, wherein the DNA molecules comprise at least one target region. In some embodiments, the target region of primer-annealed DNA is tiled with primers such that there are no gaps between the primers annealed to the target region or such that the gaps are small enough to prevent significant primer extension by a DNA polymerase, wherein the target region consists of a wild type sequence. In some embodiments, the target region of primer-annealed DNA has a gap between primers large enough to permit primer extension by a DNA polymerase, such as a 5' to 3' exonuclease negative, strand displacement negative DNA polymerase, wherein the target region comprises a rearrangement (e.g., rearrangement breakpoint).

As used herein, a DNA polymerase that is "5' to 3' exonuclease negative" does not have significant 5' to 3' exonuclease activity. A DNA polymerase that is "strand displacement negative" does not have significant helicase activity to displace a strand, such as a primer, annealed to DNA. In some embodiments, a 5' to 3' exonuclease negative, strand displacement negative DNA polymerase is used for primer extension in order to prevent extension of primers annealed to wild type target regions.

As used herein, a "primer-extended product," when referring to primers that anneal to at least one target region, means a nucleic acid strand formed by extension of a primer annealed to a DNA target region. In some embodiments, a primer-extended product is a significant primer-extended product or is formed by significant primer extension, meaning that the resulting nucleic acid strand has sufficient additional length (e.g., at least 10, 15, 20, 30, 40, 50, 60, 75, or 100 nucleotides in addition to the length of the original primer) to be detected and/or identified using methods described herein. In some embodiments, significant primer extension results in primer-extended products comprising a capture moiety present at a low percentage in the deoxynucleoside triphosphate mixture. In some embodiments, primer extension that results in no primer-extended products or only short primer-extended products that do not comprise the capture moiety occurs on target regions comprising a completely tiled primer-annealed target region.

As used herein, a "blocking oligonucleotide" is an oligonucleotide having a sequence that is complementary to a portion of a DNA molecule that is outside of a target region of the DNA molecule, or to a portion of an intron within or adjacent to an exonic target region, or to a portion of an exon adjacent to an intronic target region. Blocking oligonucleotides can be used in addition to primers, which are generally complementary to sequence within a target region. In some embodiments, blocking oligonucleotides help prevent significant primer extension of primers annealed to a wild type sequence within a DNA molecule. For example, blocking may occur after a short primer-extended product of up to 50 nucleotides (e.g., 1, 2, 5, 10, 15, 20, 30, 40, or 50 nucleotides) in addition to the length of the primer has formed. In some embodiments, blocking oligonucleotides help prevent degradation or exonucleolysis of DNA molecules originally isolated from a sample. In some embodiments, blocking oligonucleotides anneal to adapters ligated to DNA molecules. In some embodiments, blocking oligonucleotides anneal to a portion of a wild type exon adjacent to an intron in an intronic target region. In some embodiments, blocking oligonucleotides anneal to a portion of a wild type intron adjacent to an exon in an exonic target region.

As used herein, "adjacent" nucleosides or oligonucleotides are nucleosides or oligonucleotides that are next to each other, with no intervening nucleosides. For example, "adjacent" nucleosides may be covalently linked together within a nucleic acid or oligonucleotide, or they may be unlinked but are next to each other because they are annealed to or hybridized to adjacent linked nucleosides of a nucleic acid. "Adjacent" oligonucleotides may likewise be linked together or unlinked to each other but annealed to or hybridized to adjacent, linked portions of a nucleic acid.

As used herein, "partitioning" of nucleic acids, such as DNA molecules, means separating, fractionating, sorting, or enriching a sample or population of nucleic acids into a plurality of subsamples or subpopulations of nucleic acids based on one or more modifications or features that is in different proportions in each of the plurality of subsamples or subpopulations. Partitioning may include physically partitioning nucleic acid molecules based on the presence or absence of one or more methylated nucleobases. A sample or population may be partitioned into one or more partitioned subsamples or subpopulations based on a characteristic that is indicative of a genetic or epigenetic change or a disease state.

As used herein, a modification or other feature is present in "a greater proportion" in a first sample or population of nucleic acid than in a second sample or population when the fraction of nucleotides with the modification or other feature is higher in the first sample or population than in the second population. For example, if in a first sample, one tenth of the nucleotides are mC, and in a second sample, one twentieth of the nucleotides are mC, then the first sample comprises the cytosine modification of 5-methylation in a greater proportion than the second sample.

As used herein, the form of the "originally isolated" sample refers to the composition or chemical structure of a sample at the time it was isolated and before undergoing any procedure that changes the chemical structure of the isolated sample. Similarly, a feature that is "originally present" in DNA molecules refers to a feature present in "original DNA molecules" or in DNA molecules "originally comprising" the feature before the DNA molecules undergo a procedure that changes the chemical structure of DNA molecules.

As used herein, "without substantially altering base pairing specificity" of a given nucleobase means that a majority of molecules comprising that nucleobase that can be sequenced do not have alterations of the base pairing specificity of the given nucleobase relative to its base pairing specificity as it was in the originally isolated sample. In some embodiments, 75%, 90%, 95%, or 99% of molecules comprising that nucleobase that can be sequenced do not have alterations of the base pairing specificity relative to its base pairing specificity as it was in the originally isolated sample. As used herein, "altered base pairing specificity" of a given nucleobase means that a majority of molecules comprising that nucleobase that can be sequenced have a base pairing specificity at that nucleobase relative to its base pairing specificity in the originally isolated sample.

As used herein, "base pairing specificity" refers to the standard DNA base (A, C, G, or T) for which a given base most preferentially pairs. For example, unmodified cytosine and 5-methylcytosine have the same base pairing specificity (i.e., specificity for G) whereas uracil and cytosine have different base pairing specificity because uracil has base pairing specificity for A while cytosine has base pairing specificity for G. The ability of uracil to form a wobble pair with G is irrelevant because uracil nonetheless most preferentially pairs with A among the four standard DNA bases.

As used herein, a "combination" comprising a plurality of members refers to either of a single composition comprising the members or a set of compositions in proximity, e.g., in separate containers or compartments within a larger container, such as a multiwell plate, tube rack, refrigerator, freezer, incubator, water bath, ice bucket, machine, or other form of storage.

The "capture yield" of a collection of probes for a given target set refers to the amount (e.g., amount relative to another target set or an absolute amount) of nucleic acid corresponding to the target set that the collection of probes captures under typical conditions. Exemplary typical capture conditions are an incubation of the sample nucleic acid and probes at 65°C for 10-18 hours in a small reaction volume (about 20 µL) containing stringent hybridization buffer. The capture yield may be expressed in absolute terms or, for a plurality of collections of probes, relative terms. When capture yields for a plurality of sets of target regions are compared, they are normalized for the footprint size of the target region set (e.g., on a per-kilobase basis). Thus, for example, if the footprint sizes of first and second target regions are 50 kb and 500 kb, respectively (giving a normalization factor of 0.1), then the DNA corresponding to the first target region set is captured with a higher yield than DNA corresponding to the second target region set when the mass per volume concentration of the captured DNA corresponding to the first target region set is more than 0.1 times the mass per volume concentration of the captured DNA corresponding to the second target region set. As a further example, using the same footprint sizes, if the captured DNA corresponding to the first target region set has a mass per volume concentration of 0.2 times the mass per volume concentration of the captured DNA corresponding to the second target region set, then the DNA corresponding to the first target region set was captured with a two-fold greater capture yield than the DNA corresponding to the second target region set.

"Capturing" one or more target nucleic acids or one or more nucleic acids comprising at least one target region refers to preferentially isolating or separating the one or more target nucleic acids or one or more nucleic acids comprising at least one target region from non-target nucleic acids or from nucleic acids that do not comprise at least one target region.

A "captured set" of nucleic acids or "captured" nucleic acids refers to nucleic acids that have undergone capture.

As used herein, a "capture moiety" is a molecule that allows affinity separation of molecules, such as nucleic acids, linked to the capture moiety from molecules lacking the capture moiety. Exemplary capture moieties include biotin, which allows affinity separation by binding to streptavidin linked or linkable to a solid phase or an oligonucleotide, which allows affinity separation through binding to a complementary oligonucleotide linked or linkable to a solid phase.

A "target region" refers to a genomic locus targeted for identification and/or capture, for example, by using primers and/or probes (e.g., through sequence complementarity). A "target region set" or "set of target regions" refers to a plurality of genomic loci targeted for identification and/or capture, for example, by using a set of primers and/or probes (e.g., through sequence complementarity). In some embodiments, a target region is an intronic region. In some embodiments, a target region is an exonic region. In some embodiments, a target region is an exon-exon junction region. In some embodiments, a target region comprises a rearrangement, such as a rearrangement associated with cancer.

An "intronic region," of DNA herein encodes an intron or a portion thereof. Intronic regions include regions that encode introns removed during post-transcriptional splicing of pre-mRNA to generate mRNA and also "J intronic regions," which are sequences that intervene between germline J gene segments (e.g., in immunoglobulin or T cell receptor loci) and are removed during somatic V(D)J recombination. An "exonic region" of DNA herein encodes at least one exon or a portion thereof in pre-mRNA or mRNA. In some embodiments, an exonic region is a VDJ exonic region, meaning that it encodes one or more V, D, or J exons in pre-mRNA or mRNA. An "exon-exon junction region" of DNA herein encodes at least one exon-exon junction in pre-mRNA (e.g., immunoglobulin or T cell receptor pre-mRNA) before any introns are removed by splicing. Exon-exon junction regions can be formed by V(D)J recombination, e.g., when a J segment is joined to a D or V segment or a V segment is joined to a D or J segment.

As used herein, a DNA "structural variation" is a mutation comprising a DNA sequence not present in the wild-type genome other than a point mutation (e.g., in which at least 5, 10, 20, or 50 contiguous nucleotides are different relative to the wild type sequence at the corresponding locus). Examples of DNA structural variations include rearrangements, such as translocations, insertions, deletions, duplications, copy-number variants, and inversions. As used herein, a DNA "rearrangement" is a structural variation, wherein the DNA sequence comprises two adjacent sequence portions that are not adjacent to each other in the germline genomic DNA. In some embodiments, a rearrangement is a translocation, gene fusion, insertion, deletion, or inversion. Exemplary rearrangements include products of a translocation, gene fusion, and VDJ recombination. In some embodiments, the rearrangement is the product of a translocation comprising fusion of two intronic regions. In some embodiments, the rearrangement is a product of VDJ recombination comprising adjacent J exonic regions. A molecule comprising a structural variation may be referred to as a structural variant. In some embodiments, an insertion is an insertion of at least 5, 10, 20, 30, 40, 50, 60, 70, 80, 90, or 100 nucleotides. In some embodiments, a deletion affects sequence spanning the end of the target region, so as to result in a primer being unblocked and undergoing extension in a method described herein.

"Corresponding to a target region set" means that a nucleic acid, such as cfDNA, originated from a locus in the target region set or specifically binds one or more primers or probes for the target region set.

"Specifically binds" in the context of a primer, a probe, or other oligonucleotide and a target sequence means that under appropriate hybridization conditions, the primer, oligonucleotide, or probe hybridizes to its target sequence, or replicates thereof, to form a stable hybrid, while at the same time formation of stable non-target hybrids is minimized. Thus, a primer or probe hybridizes to a target sequence or replicate thereof to a sufficiently greater extent than to a non-target sequence, to ultimately enable capture or detection of the target sequence. Appropriate hybridization conditions are well-known in the art, may be predicted based on sequence composition, or can be determined by using routine testing methods (see, e.g., Sambrook et al., Molecular Cloning, A Laboratory Manual, 2nd ed. (Cold Spring Harbor Laboratory Press, Cold Spring Harbor, NY, 1989) at §§ 1.90-1.91, 7.37-7.57, 9.47-9.51 and 11.47-11.57, particularly §§ 9.50-9.51, 11.12-11.13, 11.45-11.47 and 11.55-11.57).

"Sequence-variable target regions" refer to target regions that may exhibit changes in sequence such as nucleotide substitutions (i.e., single nucleotide variations), insertions, deletions, or gene fusions or transpositions in neoplastic cells (e.g., tumor cells and cancer cells) relative to normal cells. A sequence-variable target region set is a set of sequence-variable target regions. In some embodiments, the sequence-variable target regions are target regions that may exhibit changes that affect less than or equal to 50 contiguous nucleotides, e.g., less than or equal to 40, 30, 20, 10, 5, 4, 3, 2, or 1 nucleotides.

"Epigenetic target regions" refers to target regions that may show sequence-independent changes in different tissue types or in neoplastic cells (e.g., tumor cells and cancer cells) relative to normal cells; or that may show sequence-independent changes in cfDNA from subjects having cancer relative to cfDNA from healthy subjects or in DNA originating from different tissue types (e.g., tissues that ordinarily do not substantially contribute to cfDNA (e.g., lung, colon, etc.) relative to background cfDNA (e.g., cfDNA that originated from hematopoietic cells). Examples of sequence-independent changes include, but are not limited to, changes in methylation (increases or decreases), nucleosome distribution, cfDNA fragmentation patterns, CCCTC-binding factor ("CTCF") binding, transcription start sites, and regulatory protein binding regions. Epigenetic target region sets thus include, but are not limited to, hypermethylation variable target region sets, hypomethylation variable target region sets, and fragmentation variable target region sets, such as CTCF binding sites and transcription start sites. For present purposes, loci susceptible to neoplasia-, tumor-, or cancer-associated focal amplifications and/or gene fusions may also be included in an epigenetic target region set because detection of a change in copy number by sequencing or a fused sequence that maps to more than one locus in a reference genome tends to be more similar to detection of exemplary epigenetic changes discussed above than detection of nucleotide substitutions, insertions, or deletions, e.g., in that the focal amplifications and/or gene fusions can be detected at a relatively shallow depth of sequencing because their detection does not depend on the accuracy of base calls at one or a few individual positions. An epigenetic target region set is a set of epigenetic target regions.

As used herein, a "differentially methylated region" refers to a region having a detectably different degree of methylation in at least one type of tissue relative to the degree of methylation in cell-free DNA from a healthy subject. In some embodiments, a differentially methylated region has a detectably higher degree of methylation in at least one type of tissue relative to the degree of methylation in cell-free DNA from a healthy subject. In some embodiments, a differentially methylated region has a detectably lower degree of methylation in at least one type of tissue relative to the degree of methylation in cell-free DNA from a healthy subject.

A nucleic acid is "produced by a tumor" or is "circulating tumor DNA" ("ctDNA") if it originated from a tumor cell. Tumor cells are neoplastic cells that originated from a tumor, regardless of whether they remain in the tumor or become separated from the tumor (as in the cases, e.g., of metastatic cancer cells and circulating tumor cells).

The term "methylation" or "DNA methylation" refers to addition of a methyl group to a nucleobase in a nucleic acid molecule. In some embodiments, methylation refers to addition of a methyl group to a cytosine at a CpG site (cytosine-phosphate-guanine site (i.e., a cytosine followed by a guanine in a 5' → 3' direction of the nucleic acid sequence). In some embodiments, DNA methylation refers to addition of a methyl group to adenine, such as in N⁶-methyladenine. In some embodiments, DNA methylation is 5-methylation (modification of the 5th carbon of the 6-carbon ring of cytosine). In some embodiments, 5-methylation refers to addition of a methyl group to the 5C position of the cytosine to create 5-methylcytosine (5mC). In some embodiments, methylation comprises a derivative of 5mC. Derivatives of 5mC include, but are not limited to, 5-hydroxymethylcytosine (5-hmC), 5-formylcytosine (5-fC), and 5-caryboxylcytosine (5-caC). In some embodiments, DNA methylation is 3C methylation (modification of the 3rd carbon of the 6-carbon ring of cytosine). In some embodiments, 3C methylation comprises addition of a methyl group to the 3C position of the cytosine to generate 3-methylcytosine (3mC). Methylation can also occur at non CpG sites, for example, methylation can occur at a CpA, CpT, or CpC site. DNA methylation can change the activity of methylated DNA region. For example, when DNA in a promoter region is methylated, transcription of the gene may be repressed. DNA methylation is critical for normal development and abnormality in methylation may disrupt epigenetic regulation. The disruption, e.g., repression, in epigenetic regulation may cause diseases, such as cancer. Promoter methylation in DNA may be indicative of cancer

The term "hypermethylation" refers to an increased level or degree of methylation of nucleic acid molecule(s) relative to the other nucleic acid molecules within a population (e.g., sample) of nucleic acid molecules. In some embodiments, hypermethylated DNA can include DNA molecules comprising at least 1 methylated residue, at least 2 methylated residues, at least 3 methylated residues, at least 5 methylated residues, or at least 10 methylated residues.

The term "hypomethylation" refers to a decreased level or degree of methylation of nucleic acid molecule(s) relative to the other nucleic acid molecules within a population (e.g., sample) of nucleic acid molecules. In some embodiments, hypomethylated DNA includes unmethylated DNA molecules. In some embodiments, hypomethylated DNA can include DNA molecules comprising 0 methylated residues, at most 1 methylated residue, at most 2 methylated residues, at most 3 methylated residues, at most 4 methylated residues, or at most 5 methylated residues.

The terms "agent that recognizes a modified nucleobase in DNA" refers to a molecule or reagent that binds to or detects one or more modified nucleobases in DNA. A "modified nucleobase" is a nucleobase that comprises a difference in chemical structure from an unmodified nucleobase. In the case of DNA, an unmodified nucleobase is adenine, cytosine, guanine, or thymine. In some embodiments, a modified nucleobase is a modified cytosine. In some embodiments, a modified nucleobase is a methylated nucleobase. In some embodiments, a modified cytosine is a methyl cytosine, e.g., a 5-methyl cytosine. In such embodiments, the cytosine modification is a methyl. Agents that recognize a methyl cytosine in DNA include but are not limited to "methyl binding reagents," which refer herein to reagents that bind to a methyl cytosine. Methyl binding reagents include but are not limited to methyl binding domains (MBDs) and methyl binding proteins (MBPs) and antibodies specific for methyl cytosine. In some embodiments, such antibodies bind to 5-methyl cytosine in DNA. In some such embodiments, the DNA may be single-stranded or double-stranded.

The terms "or a combination thereof" and "or combinations thereof" as used herein refers to any and all permutations and combinations of the listed terms preceding the term. For example, "A, B, C, or combinations thereof" is intended to include at least one of: A, B, C, AB, AC, BC, or ABC, and if order is important in a particular context, also BA, CA, CB, ACB, CBA, BCA, BAC, or CAB. Continuing with this example, expressly included are combinations that contain repeats of one or more item or term, such as BB, AAA, AAB, BBC, AAABCCCC, CBBAAA, CABABB, and so forth. The skilled artisan will understand that typically there is no limit on the number of items or terms in any combination, unless otherwise apparent from the context.

"Or" is used in the inclusive sense, i.e., equivalent to "and/or," unless the context requires otherwise.

### II. Exemplary methods

### A. Primer extension procedures

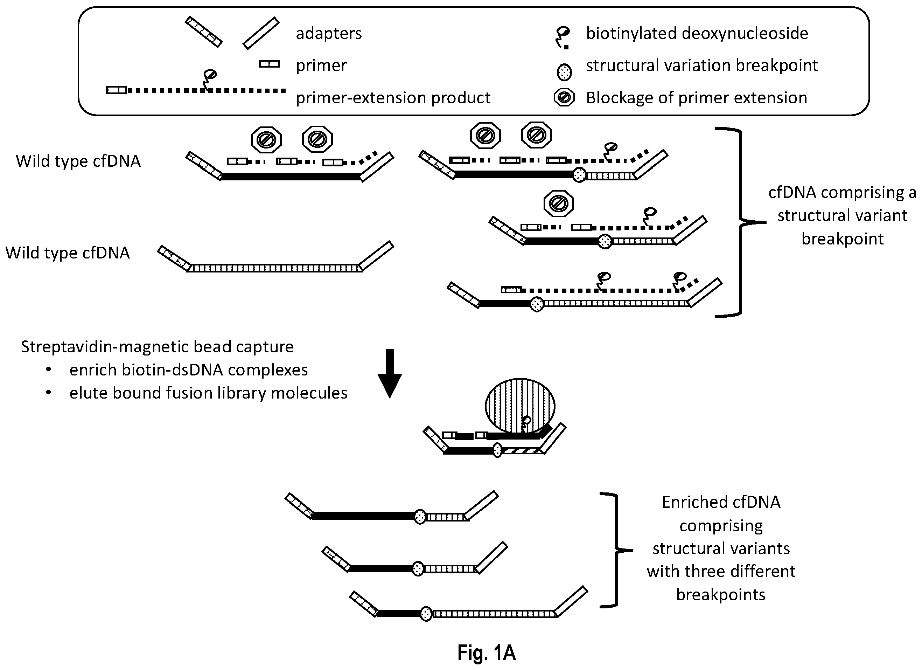

Methods disclosed herein comprise steps of contacting the DNA with a plurality of primers comprising at least two primers that anneal in a parallel orientation to a target region, thereby producing primer-annealed DNA, and contacting the primer-annealed DNA with a 5' to 3' exonuclease negative, strand displacement negative DNA polymerase and deoxynucleoside triphosphates, thereby producing a primer-extended sample, wherein for at least half of the primers, when annealed to a wild type target region, extension is blocked by a downstream primer. For example, as illustrated in Fig. 1A, a plurality of primers can be provided that anneals at various sites along a target region. When annealed to wild-type sequence, extension of one primer is blocked by the next primer. Primers near an adapter may be extended into the adapter or may be blocked by a blocking oligonucleotide that anneals to the adapter and is non-extendable (not shown). When annealed to a target region comprising a structural variation, the primer closest to the breakpoint of the structural variation can be extended because there is no annealed downstream primer in a position to block it. This can result in incorporation of a labeled (e.g., biotinylated) nucleotide, and complexes of such extended primers and the molecules to which they are annealed can then be captured or enriched, e.g., for further analysis such as sequencing.

In some embodiments, one or more target regions are amplified, e.g., as part of sequencing library preparation (e.g., LP-PCR) and/or adapters are ligated to the DNA prior to annealing the DNA to primers used in primer extension. In some embodiments, the primers used in primer extension are shorter than 100 nucleosides in length and at least 20 nucleotides in length. In some embodiments, the primers used in primer extension are 20-60, 25-60, or 30-40 nucleotides in length. In some embodiments, a majority (e.g., at least 60%, 70%, 80%, 90%, or all) of the primers used in primer extension anneal to their corresponding target regions in a parallel orientation. In such embodiments, the 3' end of each primer points toward the 5' end of another primer and/or the 5' end of each primer points toward the 3' end of another primer when they are annealed to the same DNA strand. Thus, in such embodiments, only one strand of DNA in target region anneals to any of the plurality of primers. In some embodiments, a majority (e.g., at least 60%, 70%, 80%, 90%, or all) of the primers anneal to a site separated from the site to which another primer anneals by less than or equal to about 50 nucleotides (e.g., less than or equal to 40, 30, 20, 10, 5, 4, 3, 2, or 1 nucleotides).

In some embodiments, a majority (e.g., at least 60%, 70%, 80%, 90%, or 95%) of the primers annealed to a wild type target region are blocked by a downstream primer. This means of the distinct species of primer in the plurality, the specified portion can anneal to wild-type sequence upstream of another primer so as to be blocked from extension (a small amount of extension may be permitted depending on the amount of separation between primers, as discussed above). For any given target region, there will be one last primer that is not upstream of another primer and thus is not blocked by a downstream primer; this primer may be extended or may be blocked by a non-extendable blocking oligonucleotide, as discussed elsewhere herein.

In some embodiments, the plurality of primers have approximately uniform melting temperatures with respect to annealing to their complementary sequences, e.g., so that substiantially all of the primers are capable of annealing under the same condition. For example, the plurality of primers may each anneal to their respective complementary sequence with a melting temperature within a range of 10°C, 9°C, 8°C, 7°C, 6°C, 5°C, 4°C, 3°C, 2°C, or 1°C. Variation of parameters such as primer length, GC content, and nucleotide modifications (e.g., methylation, base analogs, or LNA modifications) are known approaches for adjusting primer melting temperatures to desired values.

Extension being blocked by a downstream primer when the primers are annealed to a wild-type sequence means that the 5' to 3' exonuclease negative, strand displacement negative DNA polymerase is unable to continue extending a primer when it encounters the 5' end of another primer. This may occur after a short amount of extension, such as up to 50 nucleotides, e.g., up to 40, 30, 20, 10, 5, 4, 3, 2, or 1 nucleotides. The amount of permitted extension before becoming blocked will depend on the distance between the primers when annealed to the template sequence. In some embodiments, at least 60%, 70%, 80%, 90%, or 95% of the primers are blocked in this way when annealed to wild-type sequence. In some embodiments, the sample comprises a mixture of DNA having a wild-type sequence and a structural variation mutant sequence, and extension of at least one primer is blocked on the wild-type sequence but not on the structural variation mutant sequence. Thus, such methods can preferentially generate products in a primer-extended sample complementary to the structural variation mutant sequence. This can facilitate detection of such sequences because they are more common in the primer-extended sample than in the initial sample.

In some embodiments, the methods comprise contacting the DNA with a plurality of primers. In some embodiments, each of the plurality of primers anneal to different portions of the same target region. In some embodiments, two or more of the plurality of primers anneal to a first target region and two or more other primers of the plurality of primers anneal to a second target region. In some such embodiments, the plurality of primers comprises primers that each anneal to one of two, three, four, five, 10, 20, 30, 40, 50, 100, or more different target regions. For example, the DNA may comprise a plurality of target regions, and the plurality of primers may comprise at least two, three, four, five, 10, 20, 30, 40, 50, 100, or more primers configured to anneal to each of the plurality of target regions, resulting in each of the plurality of target regions annealing to at least two primers comprising two different sequences.

In some embodiments, the methods comprise contacting the DNA with blocking oligonucleotides that anneal to an exon adjacent to an intron of the target region in wild type sequences. In some embodiments, the methods comprise contacting the DNA with blocking oligonucleotides that anneal to an intron adjacent to an exon of the target region in wild type regions. In some embodiments, such as embodiments in which possible breakpoints in the target region are unknown, the primers used in the primer extension step are configured so that the entire wild type sequence of the target region is tiled with primers but that the sequence will not be completely tiled with primers if it comprises a rearrangement. In some embodiments, such as embodiments in which possible breakpoints in the target region are known, the primers used in the primer extension step are configured to be adjacent to a possible breakpoint when annealed to the DNA. In some such embodiments, the methods comprise contacting the DNA with blocking oligonucleotides that anneal to the wild type sequence adjacent to the breakpoint such that the blocking oligonucleotide and primer are adjacent when annealed to a wild type sequence but the blocking oligonucleotide does not anneal to DNA comprising the rearrangement.

In some embodiments, the deoxynucleoside triphosphates comprise a non-zero percentage of a modified deoxynucleoside triphosphate (e.g., a modified deoxycytidine triphosphate, deoxyadenosine triphosphate, or deoxyuridine triphosphate). The modification can facilitate capture of molecules into which the modified deoxynucleoside triphosphate has been incorporated. In some embodiments, the modified deoxynucleoside triphosphate comprises a capture moiety, which may be linked to the nucleobase of the dNTP. In some embodiments, the capture moiety is biotin. In any of the foregoing embodiments, the modified deoxynucleoside triphosphate may be a modified deoxycytidine triphosphate. In some embodiments, the percentage of deoxynucleoside triphosphate comprising a capture moiety minimizes the number of short primer-extended products produced on a wild type DNA molecule template that comprise the capture moiety and/or maximizes the number of primer-extended products produced on a DNA molecule comprising a rearrangement that comprise the capture moiety. In some embodiments, the ratio of the modified:unmodified versions of the modified deoxynucleoside triphosphate is from 1:1 to 1:100, e.g., from 1:9 to 1:19.

In some embodiments, the 5' to 3' exonuclease negative, strand displacement negative DNA polymerase is also a 3' to 5' exonuclease positive DNA polymerase that degrades any unannealed 3' primer ends. See Fig. 1B. In some such embodiments, the methods comprise contacting the DNA with blocking oligonucleotides that anneal to the 3' end adapter of the DNA to prevent degradation of the 3' end of the DNA molecule isolated from the sample, and/or contacting the DNA with blocking oligonucleotides that anneal to the 5' end adapter of the DNA to block extension of primers that anneal at or near the junction between a wild-type intron (or portion thereof) and the adapter. See Fig. 1C. In some embodiments, the blocking oligonucleotides comprise a modified nucleoside at the 3' end of the blocking oligonucleotide. In some such embodiments, the modified nucleoside comprises an inverted nucleobase, such as inverted thymine. In some embodiments, the modified nucleoside is an abasic nucleoside.

In some embodiments, the primer-extended products are enriched and/or captured using a solid support linked to a binding partner of the capture moiety, thereby also enriching and/or capturing the DNA molecules isolated from the sample that are hybridized to the primer-extended products. In some embodiments, the DNA molecules are denatured from their corresponding primer-extended products and sequenced in order to determine if they comprise a rearrangement and, if so, the breakpoint(s) of the rearrangement and further analysis as described herein. In some embodiments, the DNA molecules isolated from the sample that are hybridized to the primer-extended products comprise adapters and/or barcodes. In some embodiments, the adapters are used to amplify such molecules after denaturation. The barcodes can be used to identify sequence reads originating from the same molecule. When blocking oligonucleotides that anneal to adapters are present during production of the primer-extended sample, these can prevent incorporation of adapter and/or barcode sequences into the primer-extended products, e.g., so that only or substantially only the original DNA molecules and not the primer-extended products are amplified and sequenced.

In some embodiments, the primers that anneal to the target region of the DNA for use in a primer extension procedure do not comprise a tail that does not bind to a target region. In some embodiments, the primers that anneal to the target region of the DNA for use in a primer extension procedure do comprise a tail that does not bind to a target region but is short enough to allow the primers to anneal to the target region. In some such embodiments, the primer tail is at the 5' end of the primer. In such embodiments, the primer tail binds to the 5' end of the adapter ligated to the 3' end of the DNA molecule. In some embodiments, the primer tail comprises a capture moiety and the deoxynucleoside triphosphates used in primer extension may not comprise a capture moiety. In other embodiments, the primer tail does not comprise a capture moiety and the deoxynucleoside triphosphates used in primer extension comprise a capture moiety. The primer-extended products are captured using a solid support linked to a binding partner of the capture moiety and amplified on the solid support using PCR primers that anneal to a sequence within the primer tail and to the adapter ligated to the 5' end of the DNA. Alternatively, the primer tail comprises a modification at the 5' end that protects the primer from exonuclease activity, such as phosphorothioate internucleoside linkages. In some such embodiments, primer-extended products are enriched by contacting the primer-extended sample with a 5' to 3' exonuclease to degrade wild type sequences. The remaining sequences are amplified by PCR. In some embodiments, TdT ddATP tailing is performed prior to amplification in order to prevent truncated sequences from acting as primers in the PCR.

### B. Adapter ligation

In some embodiments, adapters are added to the nucleic acids. This may be done concurrently with an amplification procedure, e.g., by providing the adapters in a 5' portion of a primer (where PCR is used, this can be referred to as library prep-PCR or LP-PCR). In some embodiments, adapters are added by other approaches, such as ligation. In some such methods, prior to primer annealing, first adapters are added to the nucleic acids by ligation to the 3' ends thereof, which may include ligation to single-stranded DNA. The adapter can be used as a priming site for second-strand synthesis, e.g., using a universal primer and a DNA polymerase. A second adapter can then be ligated to at least the 3' end of the second strand of the now double-stranded molecule. In some embodiments, the first adapter comprises an affinity tag, such as biotin, and nucleic acid ligated to the first adapter is bound to a solid support (e.g., bead), which may comprise a binding partner for the affinity tag such as streptavidin. For further discussion of a related procedure, see Gansauge et al., Nature Protocols 8:737-748 (2013). Commercial kits for sequencing library preparation compatible with single-stranded nucleic acids are available, e.g., the Accel-NGS^{®} Methyl-Seq DNA Library Kit from Swift Biosciences. In some embodiments, after adapter ligation, nucleic acids are amplified.

Preferably, the adapters include different tags of sufficient numbers that the number of combinations of tags results in a low probability e.g., 95, 99 or 99.9% of two nucleic acids with the same start and stop points receiving the same combination of tags. Adapters, whether bearing the same or different tags, can include the same or different primer binding sites, but preferably adapters include the same primer binding site.

In some embodiments, following attachment of adapters, the nucleic acids are subject to amplification. The amplification can use, e.g., universal primers that recognize primer binding sites in the adapters.

In some embodiments, following attachment of adapters, the nucleic acids are contacted with an agent that preferentially binds to nucleic acids bearing an epigenetic modification. The nucleic acids are partitioned into at least two subsamples differing in the extent to which the nucleic acids bear the modification from binding to the agents. For example, if the agent has affinity for nucleic acids bearing the modification, nucleic acids overrepresented in the modification (compared with median representation in the population) preferentially bind to the agent, whereas nucleic acids underrepresented for the modification do not bind or are more easily eluted from the agent. The nucleic acids are then amplified from primers binding to the primer binding sites within the adapters. Partitioning may be performed instead before adapter attachment, in which case the adapters may comprise differential tags that include a component that identifies which partition a molecule occurred in.

In some embodiments, the nucleic acids are linked at both ends to Y-shaped adapters including primer binding sites and tags. The molecules are amplified.

### C. Tagging

"Tagging" DNA molecules is a procedure in which a tag is attached to or associated with the DNA molecules. Tags can be molecules, such as nucleic acids, containing information that indicates a feature of the molecule with which the tag is associated. Tags can allow one to differentiate molecules from which sequence reads originated. For example, molecules can bear a sample tag (which distinguishes molecules in one sample from those in a different sample) or a molecular tag/molecular barcode/barcode (which distinguishes different molecules from one another (in both unique and non-unique tagging scenarios). For methods that involve an optional partitioning step, a partition tag (which distinguishes molecules in one partition from those in a different partition) may be included. In some embodiments, adapters added to DNA molecules comprise tags. In certain embodiments, a tag can comprise one or a combination of barcodes. As used herein, the term "barcode" refers to a nucleic acid molecule having a particular nucleotide sequence, or to the nucleotide sequence, itself, depending on context. A barcode can have, for example, between 10 and 100 nucleotides. A collection of barcodes can have degenerate sequences or can have sequences having a certain hamming distance, as desired for the specific purpose. So, for example, a molecular barcode can be comprised of one barcode or a combination of two barcodes, each attached to different ends of a molecule. Additionally or alternatively, for different partitions and/or samples, different sets of molecular barcodes, or molecular tags can be used such that the barcodes serve as a molecular tag through their individual sequences and also serve to identify the partition and/or sample to which they correspond based the set of which they are a member. Tags comprising barcodes can be incorporated into or otherwise joined to adapters. Tags can be incorporated by ligation, overlap extension PCR among other methods.

Tagging strategies can be divided into unique tagging and non-unique tagging strategies. In unique tagging, all or substantially all of the molecules in a sample bear a different tag, so that reads can be assigned to original molecules based on tag information alone. Tags used in such methods are sometimes referred to as "unique tags". In non-unique tagging, different molecules in the same sample can bear the same tag, so that other information in addition to tag information is used to assign a sequence read to an original molecule. Such information may include start and stop coordinate, coordinate to which the molecule maps, start or stop coordinate alone, etc. Tags used in such methods are sometimes referred to as "non-unique tags". Accordingly, it is not necessary to uniquely tag every molecule in a sample. It suffices to uniquely tag molecules falling within an identifiable class within a sample. Thus, molecules in different identifiable families can bear the same tag without loss of information about the identity of the tagged molecule.

In certain embodiments of non-unique tagging, the number of different tags used can be sufficient that there is a very high likelihood (e.g., at least 99%, at least 99.9%, at least 99.99% or at least 99.999% that all molecules of a particular group bear a different tag. It is to be noted that when barcodes are used as tags, and when barcodes are attached, e.g., randomly, to both ends of a molecule, the combination of barcodes, together, can constitute a tag. This number, in term, is a function of the number of molecules falling into the calls. For example, the class may be all molecules mapping to the same start-stop position on a reference genome. The class may be all molecules mapping across a particular genetic locus, e.g., a particular base or a particular region (e.g., up to 100 bases or a gene or an exon of a gene). In certain embodiments, the number of different tags used to uniquely identify a number of molecules, z, in a class can be between any of 2*z, 3*z, 4*z, 5*z, 6*z, 7*z, 8*z, 9*z, 10*z, 11 *z, 12*z, 13*z, 14*z, 15*z, 16*z, 17*z, 18*z, 19*z, 20*z or 100*z (e.g., lower limit) and any of 100,000*z, 10,000*z, 1000*z or 100*z (e.g., upper limit).

For example, in a sample of about 5 ng to 30 ng of cell free DNA, one expects around 3000 molecules to map to a particular nucleotide coordinate, and between about 3 and 10 molecules having any start coordinate to share the same stop coordinate. Accordingly, about 50 to about 50,000 different tags (e.g., between about 6 and 220 barcode combinations) can suffice to uniquely tag all such molecules. To uniquely tag all 3000 molecules mapping across a nucleotide coordinate, about 1 million to about 20 million different tags would be required.

Generally, assignment of unique or non-unique tags barcodes in reactions follows methods and systems described by US patent applications 20010053519, 20030152490, 20110160078, and U.S. Pat. No. 6,582,908 and U.S. Pat. No. 7,537,898 and US Pat. No. 9,598,731. Tags can be linked to sample nucleic acids randomly or non-randomly.

In some embodiments, the tagged nucleic acids are sequenced after loading into a microwell plate. The microwell plate can have 96, 384, or 1536 microwells. In some cases, they are introduced at an expected ratio of unique tags to microwells. For example, the unique tags may be loaded so that more than about 1, 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 unique tags are loaded per genome sample. In some cases, the unique tags may be loaded so that less than about 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 unique tags are loaded per genome sample. In some cases, the average number of unique tags loaded per sample genome is less than, or greater than, about 1, 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 unique tags per genome sample.

A preferred format uses 20-50 different tags (e.g., barcodes) ligated to both ends of target nucleic acids. For example 35 different tags (e.g., barcodes) ligated to both ends of target molecules creating 35 x 35 permutations, which equals 1225 for 35 tags. Such numbers of tags are sufficient so that different molecules having the same start and stop points have a high probability (e.g., at least 94%, 99.5%, 99.99%, 99.999%) of receiving different combinations of tags. Other barcode combinations include any number between 10 and 500, e.g., about 15x15, about 35x35, about 75x75, about 100x100, about 250x250, about 500x500.

In some cases, unique tags may be predetermined or random or semi-random sequence oligonucleotides. In other cases, a plurality of barcodes may be used such that barcodes are not necessarily unique to one another in the plurality. In this example, barcodes may be ligated to individual molecules such that the combination of the barcode and the sequence it may be ligated to creates a unique sequence that may be individually tracked. As described herein, detection of non-unique barcodes in combination with sequence data of beginning (start) and end (stop) portions of sequence reads may allow assignment of a unique identity to a particular molecule. The length or number of base pairs, of an individual sequence read may also be used to assign a unique identity to such a molecule. As described herein, fragments from a single strand of nucleic acid having been assigned a unique identity, may thereby permit subsequent identification of fragments from the parent strand.

In some embodiments, two or more partitions, e.g., each partition, is/are differentially tagged. Tags can be used to label the individual polynucleotide population partitions so as to correlate the tag (or tags) with a specific partition. Alternatively, tags can be used in embodiments of the invention that do not employ a partitioning step. In some embodiments, a single tag can be used to label a specific partition. In some embodiments, multiple different tags can be used to label a specific partition. In embodiments employing multiple different tags to label a specific partition, the set of tags used to label one partition can be readily differentiated for the set of tags used to label other partitions. In some embodiments, the tags may have additional functions, for example the tags can be used to index sample sources or used as unique molecular identifiers (which can be used to improve the quality of sequencing data by differentiating sequencing errors from mutations, for example as in Kinde et al., Proc Nat'l Acad Sci USA 108: 9530-9535 (2011), Kou et al., PLoS ONE,11: e0146638 (2016)) or used as non-unique molecule identifiers, for example as described in US Pat. No. 9,598,731. Similarly, in some embodiments, the tags may have additional functions, for example the tags can be used to index sample sources or used as non-unique molecular identifiers (which can be used to improve the quality of sequencing data by differentiating sequencing errors from mutations).

In one embodiment, partition tagging comprises tagging molecules in each partition with a partition tag. After re-combining partitions (e.g., to reduce the number of sequencing runs needed and avoid unnecessary cost) and sequencing molecules, the partition tags identify the source partition. In another embodiment, different partitions are tagged with different sets of molecular tags, e.g., comprised of a pair of barcodes. In this way, each molecular barcode indicates the source partition as well as being useful to distinguish molecules within a partition. For example, a first set of 35 barcodes can be used to tag molecules in a first partition, while a second set of 35 barcodes can be used tag molecules in a second partition.

In some embodiments, after partitioning and tagging with partition tags, the molecules may be pooled for sequencing in a single run. In some embodiments, a sample tag is added to the molecules, e.g., in a step subsequent to addition of partition tags and pooling. Sample tags can facilitate pooling material generated from multiple samples for sequencing in a single sequencing run.

Alternatively, in some embodiments, partition tags may be correlated to the sample as well as the partition. As a simple example, a first tag can indicate a first partition of a first sample; a second tag can indicate a second partition of the first sample; a third tag can indicate a first partition of a second sample; and a fourth tag can indicate a second partition of the second sample.

While tags may be attached to molecules already partitioned based on one or more characteristics, the final tagged molecules in the library may no longer possess that characteristic. For example, while single stranded DNA molecules may be partitioned and tagged, the final tagged molecules in the library are likely to be double stranded. Similarly, while DNA may be subject to partition based on different levels of methylation, in the final library, tagged molecules derived from these molecules are likely to be unmethylated. Accordingly, the tag attached to molecule in the library typically indicates the characteristic of the "parent molecule" from which the ultimate tagged molecule is derived, not necessarily to characteristic of the tagged molecule, itself.

As an example, barcodes 1, 2, 3, 4, etc. are used to tag and label molecules in the first partition; barcodes A, B, C, D, etc. are used to tag and label molecules in the second partition; and barcodes a, b, c, d, etc. are used to tag and label molecules in the third partition. Differentially tagged partitions can be pooled prior to sequencing. Differentially tagged partitions can be separately sequenced or sequenced together concurrently, e.g., in the same flow cell of an Illumina sequencer.

After sequencing, analysis of reads to detect genetic variants can be performed on a partition-by-partition level, as well as a whole nucleic acid population level. Tags are used to sort reads from different partitions. Analysis can include in silico analysis to determine genetic and epigenetic variation (one or more of methylation, chromatin structure, etc.) using sequence information, genomic coordinates length, coverage, and/or copy number. In some embodiments, higher coverage can correlate with higher nucleosome occupancy in genomic region while lower coverage can correlate with lower nucleosome occupancy or a nucleosome depleted region (NDR).

Partitioning procedures may result in imperfect sorting of DNA molecules among the subsamples. For example, a minority of the molecules in the second subsample may be highly modified (e.g., hypermethylated), and/or a minority of the molecules in the first subsample may be unmodified or mostly unmodified (e.g., unmethylated or mostly unmethylated). Highly modified molecules in the second subsample and unmodified or mostly unmodified molecules in the first subsample are considered nonspecifically partitioned. The methods described herein comprise steps that can reduce technical noise from nonspecifically partitioned DNA, e.g., by converting certain bases such that nonspecifically partitioned DNA can be identified following sequencing and/or by degrading it. Thus, the methods described herein can provide improved sensitivity and/or streamlined analysis.

### D. Enriching/Capturing step; amplification

Methods disclosed herein comprise detecting DNA, such as cfDNA target regions. In some embodiments, the detecting comprises enriching for or capturing one or more primer-extended products and target regions hybridized to the one or more primer-extended products. Enrichment or capture may be performed on any sample or subsample described herein using any suitable approach known in the art.

In some embodiments, the primer-extended products comprise a capture moiety that facilitates the enrichment or capture of the primer-extended products and DNA hybridized to the primer-extended products. The capture moiety may be provided as part of the primer or incorporated during extension as part of a modified deoxyribonucleotide triphosphate as discussed in detail elsewhere herein. In some embodiments, the capture moiety is biotin. In some such embodiments, streptavidin attached to a solid support, such as magnetic beads, is used to bind to the biotin. Nonspecifically bound DNA that does not comprise a target region is washed away from the captured primer-extended products and DNA hybridized to them. Where the capture moiety is incorporated during extension, nonspecifically bound DNA can include short primer-extended products that do not comprise a capture moiety. In some embodiments, DNA is then dissociated or denatured from the primer-extended products and eluted from the solid support using salt washes or buffers comprising another DNA denaturing agent. In some embodiments, the primer-extended products are also eluted from the solid support by, e.g., disrupting the biotin-streptavidin interaction. In some embodiments, captured DNA is amplified following elution from the solid support. In some such embodiments, DNA comprising adapters is amplified using PCR primers that anneal to the adapters. In some embodiments, captured DNA is amplified while attached to the solid support. In some such embodiments, the amplification comprises use of a PCR primer that anneals to a sequence within an adapter and a PCR primer that anneals to a sequence within a primer annealed to the target region of the DNA. In some embodiments, e.g., where the capture moiety is provided as part of the primer, the presence of short primer-extended products and unextended primers can be minimized by performing an amplification subsequent to capture that is dependent on the presence of adapter sequences at both ends of the DNA for exponential amplification, e.g., such that short primer-extended products (which resulted from extension that was blocked before reaching an adapter sequence in the template molecule) are not exponentially amplified or are amplified to a lesser extent than primer-extended products that comprise adapter sequences at both ends.

In some embodiments, the methods herein comprise enriching for or capturing DNA comprising epigenetic and/or sequence-variable target regions. Such regions may be captured from an aliquot of a sample (e.g., a sample that has undergone attachment of adapters and amplification), while the steps of contacting the DNA with a plurality of primers that anneal to a target region and contacting the primer-annealed DNA with a DNA polymerase are performed on a separate aliquot of the sample. Enriching for or capturing DNA comprising epigenetic and/or sequence-variable target regions may comprise contacting the DNA with a set of target-specific probes. The set of target-specific probes may have any of the features described herein for sets of target-specific probes, including but not limited to in the embodiments set forth above and the sections relating to probes below. Capturing may be performed on one or more subsamples prepared during methods disclosed herein. In some embodiments, DNA is captured from the first subsample or the second subsample, e.g., the first subsample and the second subsample. In some embodiments, the subsamples are differentially tagged (e.g., as described herein) and then pooled before undergoing capture.

The capturing step may be performed using conditions suitable for specific nucleic acid hybridization, which generally depend to some extent on features of the probes such as length, base composition, etc. Those skilled in the art will be familiar with appropriate conditions given general knowledge in the art regarding nucleic acid hybridization. In some embodiments, complexes of target-specific probes and DNA are formed.

In some embodiments, methods described herein comprise capturing a plurality of sets of target regions of cfDNA obtained from a test subject. The target regions comprise intronic regions or VDJ regions that may comprise rearrangements, epigenetic target regions, which may show differences in methylation levels and/or fragmentation patterns depending on whether they originated from a tumor or from healthy cells, and sequence-variable regions, which may show differences in sequence, other than rearrangements, depending on whether they originated from a tumor or from healthy cells. The capturing step produces a captured set of cfDNA molecules. In some embodiments, the cfDNA molecules corresponding to the sequence-variable target region set are captured at a greater capture yield in the captured set of cfDNA molecules than cfDNA molecules corresponding to the epigenetic target region set. In some embodiments, a method described herein comprises contacting cfDNA obtained from a test subject with a set of target-specific probes, wherein the set of target-specific probes is configured to capture cfDNA corresponding to the sequence-variable target region set at a greater capture yield than cfDNA corresponding to the epigenetic target region set. For additional discussion of capturing steps, capture yields, and related aspects, see WO2020/160414.

It can be beneficial to capture cfDNA corresponding to the sequence-variable target region set at a greater capture yield than cfDNA corresponding to the epigenetic target region set because a greater depth of sequencing may be necessary to analyze the sequence-variable target regions with sufficient confidence or accuracy than may be necessary to analyze the epigenetic target regions. The volume of data needed to determine fragmentation patterns (e.g., to test for perturbation of transcription start sites or CTCF binding sites) or fragment abundance (e.g., in hypermethylated and hypomethylated partitions) is generally less than the volume of data needed to determine the presence or absence of cancer-related sequence mutations. Capturing the target region sets at different yields can facilitate sequencing the target regions to different depths of sequencing in the same sequencing run (e.g., using a pooled mixture and/or in the same sequencing cell).

In some embodiments, the DNA is amplified. In some embodiments, amplification is performed before the capturing step. In some embodiments, amplification is performed after the capturing step. In some embodiments, amplification is performed before and after the capturing step. In various embodiments, the methods further comprise sequencing the captured cfDNA, e.g., to different degrees of sequencing depth for the epigenetic and sequence-variable target region sets and for rearrangements, consistent with the discussion herein.

In some embodiments, complexes of primer-extended products and DNA are separated from DNA not bound to primer-extended products. For example, where complexes comprising primer-extended products are bound covalently or noncovalently to a solid support, a washing or aspiration step can be used to separate unbound material. Alternatively, where the complexes have chromatographic properties distinct from unbound material (e.g., where the primer-extended products comprise a ligand that binds a chromatographic resin), chromatography can be used. In some embodiments, complexes of target-specific probes and DNA are separated from DNA not bound to target-specific probes. For example, where target-specific probes are bound covalently or noncovalently to a solid support, a washing or aspiration step can be used to separate unbound material. Alternatively, where the complexes have chromatographic properties distinct from unbound material (e.g., where the probes comprise a ligand that binds a chromatographic resin), chromatography can be used.

In some embodiments, a capturing step is performed with probes for a sequence-variable target region set and probes for an epigenetic target region set in the same vessel at the same time, e.g., the probes for the sequence-variable and epigenetic target region sets are in the same composition. This approach provides a relatively streamlined workflow. In some embodiments, the concentration of the probes for the sequence-variable target region set is greater that the concentration of the probes for the epigenetic target region set.

Alternatively, a capturing step is performed with a sequence-variable target region probe set in a first vessel and with an epigenetic target region probe set in a second vessel, or a contacting step is performed with a sequence-variable target region probe set at a first time and a first vessel and an epigenetic target region probe set at a second time before or after the first time. This approach allows for preparation of separate first and second compositions comprising captured DNA corresponding to a sequence-variable target region set and captured DNA corresponding to an epigenetic target region set. The compositions can be processed separately as desired (e.g., to partition based on methylation as described herein) and pooled in appropriate proportions to provide material for further processing and analysis such as sequencing.

In some embodiments, adapters are included in the DNA as described herein. In some embodiments, tags, which may be or include barcodes, are included in the DNA. In some embodiments, such tags are included in adapters. Tags can facilitate identification of the origin of a nucleic acid. For example, barcodes can be used to allow the origin (e.g., subject) whence the DNA came to be identified following pooling of a plurality of samples for parallel sequencing. This may be done concurrently with an amplification procedure, e.g., by providing the barcodes in a 5' portion of a primer, e.g., as described herein. In some embodiments, adapters and tags/barcodes are provided by the same primer or primer set. For example, the barcode may be located 3' of the adapter and 5' of the target-hybridizing portion of the primer. Alternatively, barcodes can be added by other approaches, such as ligation, optionally together with adapters in the same ligation substrate.

Additional details regarding amplification, tags, and barcodes are discussed herein, which can be combined to the extent practicable with any of these embodiments.

### E. Procedures that affect a first nucleobase in the DNA differently from a second nucleobase in the DNA

In some embodiments, methods disclosed herein are useful for analyzing epigenetic modifications in DNA, e.g., in addition to detecting structural variations. Some such embodiments comprise a step of subjecting DNA to a procedure that affects a first nucleobase in the DNA differently from a second nucleobase in the DNA, wherein the first nucleobase is a modified or unmodified nucleobase, the second nucleobase is a modified or unmodified nucleobase different from the first nucleobase, and the first nucleobase and the second nucleobase have the same base pairing specificity. In some embodiments, the procedure chemically converts the first or second nucleobase such that the base pairing specificity of the converted nucleobase is altered. In some embodiments, if the first nucleobase is a modified or unmodified adenine, then the second nucleobase is a modified or unmodified adenine; if the first nucleobase is a modified or unmodified cytosine, then the second nucleobase is a modified or unmodified cytosine; if the first nucleobase is a modified or unmodified guanine, then the second nucleobase is a modified or unmodified guanine; and if the first nucleobase is a modified or unmodified thymine, then the second nucleobase is a modified or unmodified thymine (where modified and unmodified uracil are encompassed within modified thymine for the purpose of this step).

In some embodiments, the first nucleobase is a modified or unmodified cytosine, then the second nucleobase is a modified or unmodified cytosine. For example, first nucleobase may comprise unmodified cytosine (C) and the second nucleobase may comprise one or more of 5-methylcytosine (mC) and 5-hydroxymethylcytosine (hmC). Alternatively, the second nucleobase may comprise C and the first nucleobase may comprise one or more of mC and hmC. Other combinations are also possible, as indicated, e.g., in the Summary above and the following discussion, such as where one of the first and second nucleobases comprises mC and the other comprises hmC.

In some embodiments, the procedure that affects a first nucleobase in the DNA differently from a second nucleobase in the DNA comprises bisulfite conversion. Treatment with bisulfite converts unmodified cytosine and certain modified cytosines (e.g. 5-formyl cytosine (fC) or 5-carboxylcytosine (caC)) to uracil whereas other modified cytosines (e.g., 5-methylcytosine, 5-hydroxylmethylcystosine) are not converted. Thus, where bisulfite conversion is used, the first nucleobase comprises or consists of one or more of unmodified cytosine, 5-formyl cytosine, 5-carboxylcytosine, or other cytosine forms affected by bisulfite, and the second nucleobase may comprise one or more of mC and hmC, such as mC and optionally hmC. Sequencing of bisulfite-treated DNA identifies positions that are read as cytosine as being mC or hmC positions. Meanwhile, positions that are read as T are identified as being T or a bisulfite-susceptible form of C, such as unmodified cytosine, 5-formyl cytosine, or 5-carboxylcytosine. Performing bisulfite conversion as described herein thus facilitates identifying positions containing mC or hmC using the sequence reads. For an exemplary description of bisulfite conversion, *see, e.g.,* Moss et al., Nat Commun. 2018; 9: 5068.

In some embodiments, the procedure that affects a first nucleobase in the DNA differently from a second nucleobase in the DNA comprises oxidative bisulfite (Ox-BS) conversion. This procedure first converts hmC to fC, which is bisulfite susceptible, followed by bisulfite conversion. Thus, when oxidative bisulfite conversion is used, the first nucleobase comprises one or more of unmodified cytosine, fC, caC, hmC, or other cytosine forms affected by bisulfite, and the second nucleobase comprises mC. Sequencing of Ox-BS converted DNA identifies positions that are read as cytosine as being mC positions. Meanwhile, positions that are read as T are identified as being T, hmC, or a bisulfite-susceptible form of C, such as unmodified cytosine, fC, or hmC. Performing Ox-BS conversion as described herein thus facilitates identifying positions containing mC using the sequence reads. For an exemplary description of oxidative bisulfite conversion, *see, e.g.,* Booth et al., Science 2012; 336: 934-937.

In some embodiments, the procedure that affects a first nucleobase in the DNA differently from a second nucleobase in the DNA comprises Tet-assisted bisulfite (TAB) conversion. In TAB conversion, hmC is protected from conversion and mC is oxidized in advance of bisulfite treatment, so that positions originally occupied by mC are converted to U while positions originally occupied by hmC remain as a protected form of cytosine. For example, as described in Yu et al., Cell 2012; 149: 1368-80, β-glucosyl transferase can be used to protect hmC (forming 5-glucosylhydroxymethylcytosine (ghmC)), then a TET protein such as mTet1 can be used to convert mC to caC, and then bisulfite treatment can be used to convert C and caC to U while ghmC remains unaffected. Thus, when TAB conversion is used, the first nucleobase comprises one or more of unmodified cytosine, fC, caC, mC, or other cytosine forms affected by bisulfite, and the second nucleobase comprises hmC. Sequencing of TAB-converted DNA identifies positions that are read as cytosine as being hmC positions. Meanwhile, positions that are read as T are identified as being T, mC, or a bisulfite-susceptible form of C, such as unmodified cytosine, fC, or caC. Performing TAB conversion as described herein thus facilitates identifying positions containing hmC using the sequence reads.

In some embodiments, the procedure that affects a first nucleobase in the DNA differently from a second nucleobase in the DNA comprises Tet-assisted conversion with a substituted borane reducing agent, optionally wherein the substituted borane reducing agent is 2-picoline borane, borane pyridine, tert-butylamine borane, or ammonia borane. In Tet-assisted pic-borane conversion with a substituted borane reducing agent conversion, a TET protein is used to convert mC and hmC to caC, without affecting unmodified C. caC, and fC if present, are then converted to dihydrouracil (DHU) by treatment with 2-picoline borane (pic-borane) or another substituted borane reducing agent such as borane pyridine, tert-butylamine borane, or ammonia borane, also without affecting unmodified C. *See, e.g.,* Liu et al., Nature Biotechnology 2019; 37:424-429 (e.g., at Supplementary Fig. 1 and Supplementary Note 7). DHU is read as a T in sequencing. Thus, when this type of conversion is used, the first nucleobase comprises one or more of mC, fC, caC, or hmC, and the second nucleobase comprises unmodified cytosine. Sequencing of the converted DNA identifies positions that are read as cytosine as being unmodified C positions. Meanwhile, positions that are read as T are identified as being T, mC, fC, caC, or hmC. Performing TAP conversion as described herein thus facilitates identifying positions containing unmodified C using the sequence reads. This procedure encompasses Tet-assisted pyridine borane sequencing (TAPS), described in further detail in Liu et al. 2019, supra.

Alternatively, protection of hmC (e.g., using βGT) can be combined with Tet-assisted conversion with a substituted borane reducing agent. hmC can be protected as noted above through glucosylation using βGT, forming ghmC. Treatment with a TET protein such as mTet1 then converts mC to caC but does not convert C or ghmC. caC is then converted to DHU by treatment with pic-borane or another substituted borane reducing agent such as borane pyridine, tert-butylamine borane, or ammonia borane, also without affecting unmodified C or ghmC. Thus, when Tet-assisted conversion with a substituted borane reducing agent is used, the first nucleobase comprises mC, and the second nucleobase comprises one or more of unmodified cytosine or hmC, such as unmodified cytosine and optionally hmC, fC, and/or caC. Sequencing of the converted DNA identifies positions that are read as cytosine as being either hmC or unmodified C positions. Meanwhile, positions that are read as T are identified as being T, fC, caC, or mC. Performing TAPSβ conversion as described herein thus facilitates distinguishing positions containing unmodified C or hmC on the one hand from positions containing mC using the sequence reads. For an exemplary description of this type of conversion, *see, e.g.,* Liu et al., Nature Biotechnology 2019; 37:424-429.

In some embodiments, the procedure that affects a first nucleobase in the DNA differently from a second nucleobase in the DNA comprises chemical-assisted conversion with a substituted borane reducing agent, optionally wherein the substituted borane reducing agent is 2-picoline borane, borane pyridine, tert-butylamine borane, or ammonia borane. In chemical-assisted conversion with a substituted borane reducing agent, an oxidizing agent such as potassium perruthenate (KRuO₄) (also suitable for use in ox-BS conversion) is used to specifically oxidize hmC to fC. Treatment with pic-borane or another substituted borane reducing agent such as borane pyridine, tert-butylamine borane, or ammonia borane converts fC and caC to DHU but does not affect mC or unmodified C. Thus, when this type of conversion is used, the first nucleobase comprises one or more of hmC, fC, and caC, and the second nucleobase comprises one or more of unmodified cytosine or mC, such as unmodified cytosine and optionally mC. Sequencing of the converted DNA identifies positions that are read as cytosine as being either mC or unmodified C positions. Meanwhile, positions that are read as T are identified as being T, fC, caC, or hmC. Performing this type of conversion as described herein thus facilitates distinguishing positions containing unmodified C or mC on the one hand from positions containing hmC using the sequence reads. For an exemplary description of this type of conversion, *see, e.g.,* Liu et al., Nature Biotechnology 2019; 37:424-429.

In some embodiments, the procedure that affects a first nucleobase in the DNA differently from a second nucleobase in the DNA comprises APOBEC-coupled epigenetic (ACE) conversion. In ACE conversion, an AID/APOBEC family DNA deaminase enzyme such as APOBEC3A (A3A) is used to deaminate unmodified cytosine and mC without deaminating hmC, fC, or caC. Thus, when ACE conversion is used, the first nucleobase comprises unmodified C and/or mC (e.g., unmodified C and optionally mC), and the second nucleobase comprises hmC. Sequencing of ACE-converted DNA identifies positions that are read as cytosine as being hmC, fC, or caC positions. Meanwhile, positions that are read as T are identified as being T, unmodified C, or mC. Performing ACE conversion as described herein thus facilitates distinguishing positions containing hmC from positions containing mC or unmodified C using the sequence reads. For an exemplary description of ACE conversion, *see, e.g.,* Schutsky et al., Nature Biotechnology 2018; 36: 1083-1090.

In some embodiments, procedure that affects a first nucleobase in the DNA differently from a second nucleobase in the DNA comprises enzymatic conversion of the first nucleobase, e.g., as in EM-Seq. See, e.g., Vaisvila R, et al. (2019) EM-seq: Detection of DNA methylation at single base resolution from picograms of DNA. bioRxiv; DOI: 10.1101/2019.12.20.884692, available at www.biorxiv.org/content/10.1101/2019.12.20.884692v1. For example, TET2 and optionally T4-βGT can be used to convert 5mC and 5hmC into substrates that cannot be deaminated by a deaminase (e.g., APOBEC3A), and then a deaminase (e.g., APOBEC3A) can be used to deaminate unmodified cytosines converting them to uracils. In some embodiments, 5mC and/or 5hmC are converted to 5caC (e.g., using Tet2), and then unmodified C is converted to U (e.g., using APOBEC3A). 5caC-containing DNA (generated from DNA originally containing 5mC and/or 5hmC) can then be partitioned, e.g., using an anti-caC antibody.

In some embodiments, the first nucleobase is a modified or unmodified adenine, and the second nucleobase is a modified or unmodified adenine. In some embodiments, the modified adenine is N⁶-methyladenine (mA). In some embodiments, the modified adenine is one or more of N⁶-methyladenine (mA), N⁶-hydroxymethyladenine (hmA), or N⁶-formyladenine (fA).

Techniques comprising methylated DNA immunoprecipitation (MeDIP) can be used to separate DNA containing modified bases such as mC, mA, caC (which may be generated by oxidation of mC or hmC with Tet2, e.g., before enzymatic conversion of unmodified C to U, e.g., using a deaminase such as APOBEC3A), or dihydrouracil from other DNA. *See, e.g.,* Kumar et al., Frontiers Genet. 2018; 9: 640; Greer et al., Cell 2015; 161: 868-878. An antibody specific for mA is described in Sun et al., Bioessays 2015; 37:1155-62. Antibodies for various modified nucleobases, such as mC, caC, and forms of thymine/uracil including dihydrouracil or halogenated forms such as 5-bromouracil, are commercially available. Various modified bases can also be detected based on alterations in their base pairing specificity. For example, hypoxanthine is a modified form of adenine that can result from deamination and is read in sequencing as a G. *See, e.g.,* US Patent 8,486,630; Brown, Genomes, 2nd Ed., John Wiley & Sons, Inc., New York, N.Y., 2002, chapter 14, "Mutation, Repair, and Recombination."

### F. Partitioning the sample into a plurality of subsamples

In some embodiments described herein, different forms of nucleic acids (e.g., hypermethylated and hypomethylated DNA) are physically partitioned based on one or more characteristics of the nucleic acids. This approach can be used to determine, for example, whether certain sequences are hypermethylated or hypomethylated. In some embodiments, the partitioning is completed after a procedure that affects first and second nucleobases differently and is based on different forms of the nucleic acids that were originally present in the sample before it was subjected to the procedure. In some embodiments, the partitioning is based on different forms of the nucleic acids that were produced by the procedure that differently affects a first nucleobase and a second nucleobase in the nucleic acids of the sample. In some embodiments, the partitioning is completed before a procedure that affects first and second nucleobases differently.

Methylation profiling can involve determining methylation patterns across different regions of the genome. For example, after partitioning molecules based on extent of methylation (e.g., relative number of methylated nucleobases per molecule) and sequencing, the sequences of molecules in the different partitions can be mapped to a reference genome. This can show regions of the genome that, compared with other regions, are more highly methylated or are less highly methylated. In this way, genomic regions, in contrast to individual molecules, may differ in their extent of methylation.

Partitioning nucleic acid molecules in a sample can increase a rare signal, e.g., by enriching rare nucleic acid molecules that are more prevalent in one partition of the sample. For example, a genetic variation present in hypermethylated DNA but less (or not) present in hypomethylated DNA can be more easily detected by partitioning a sample into hypermethylated and hypomethylated nucleic acid molecules. By analyzing multiple partitions of a sample, a multi-dimensional analysis of a single molecule can be performed and hence, greater sensitivity can be achieved. Partitioning may include physically partitioning nucleic acid molecules into partitions or subsamples based on the presence or absence of one or more methylated nucleobases. A sample may be partitioned into partitions or subsamples based on a characteristic that is indicative of differential gene expression or a disease state. A sample may be partitioned based on a characteristic, or combination thereof that provides a difference in signal between a normal and diseased state during analysis of nucleic acids, e.g., cell free DNA (cfDNA), non-cfDNA, tumor DNA, circulating tumor DNA (ctDNA) and cell free nucleic acids (cfNA).

In some embodiments, hypermethylation variable epigenetic target regions are analyzed to determine whether they show hypermethylation characteristic of tumor cells or cells of a type that does not normally contribute to the DNA sample being analyzed (such as cfDNA), and/or hypomethylation variable epigenetic target regions are analyzed to determine whether they show hypomethylation characteristic of tumor cells or cells of a type that does not normally contribute to the DNA sample being analyzed (such as cfDNA).

In some instances, a heterogeneous nucleic acid sample is partitioned into two or more partitions (e.g., at least 3, 4, 5, 6 or 7 partitions). In some embodiments, each partition is differentially tagged. Tagged partitions can then be pooled together for collective sample prep and/or sequencing. The partitioning-tagging-pooling steps can occur more than once, with each round of partitioning occurring based on a different characteristics (examples provided herein), and tagged using differential tags that are distinguished from other partitions and partitioning means. In other instances, the differentially tagged partitions are separately sequenced.

In some embodiments, sequence reads from differentially tagged and pooled nucleic acids are obtained and analyzed in silico. Tags are used to sort reads from different partitions. Analysis to detect genetic variants can be performed on a partition-by-partition level, as well as whole nucleic acid population level. For example, analysis can include in silico analysis to determine genetic variants, such as CNV, SNV, indel, fusion in nucleic acids in each partition. In some instances, in silico analysis can include determining chromatin structure. For example, coverage of sequence reads can be used to determine nucleosome positioning in chromatin. Higher coverage can correlate with higher nucleosome occupancy in genomic region while lower coverage can correlate with lower nucleosome occupancy or nucleosome depleted region (NDR).

Examples of characteristics that can be used for partitioning include sequence length, methylation level, nucleosome binding, sequence mismatch, immunoprecipitation, and/or proteins that bind to DNA. Resulting partitions can include one or more of the following nucleic acid forms: single-stranded DNA (ssDNA), double-stranded DNA (dsDNA), shorter DNA fragments and longer DNA fragments. In some embodiments, partitioning based on a cytosine modification (e.g., cytosine methylation) or methylation generally is performed and is optionally combined with at least one additional partitioning step, which may be based on any of the foregoing characteristics or forms of DNA. In some embodiments, a heterogeneous population of nucleic acids is partitioned into nucleic acids with one or more epigenetic modifications and without the one or more epigenetic modifications. Examples of epigenetic modifications include presence or absence of methylation; level of methylation; type of methylation (e.g., 5-methylcytosine versus other types of methylation, such as adenine methylation and/or cytosine hydroxymethylation); and association and level of association with one or more proteins, such as histones. Alternatively or additionally, a heterogeneous population of nucleic acids can be partitioned into nucleic acid molecules associated with nucleosomes and nucleic acid molecules devoid of nucleosomes. Alternatively or additionally, a heterogeneous population of nucleic acids may be partitioned into single-stranded DNA (ssDNA) and double-stranded DNA (dsDNA). Alternatively, or additionally, a heterogeneous population of nucleic acids may be partitioned based on nucleic acid length (e.g., molecules of up to 160 bp and molecules having a length of greater than 160 bp).

The agents used to partition populations of nucleic acids within a sample can be affinity agents, such as antibodies with the desired specificity, natural binding partners or variants thereof (Bock et al., Nat Biotech 28: 1106-1114 (2010); Song et al., Nat Biotech 29: 68-72

(2011)), or artificial peptides selected e.g., by phage display to have specificity to a given target. In some embodiments, the agent used in the partitioning is an agent that recognizes a modified nucleobase. In some embodiments, the modified nucleobase recognized by the agent is a modified cytosine, such as a methylcytosine (e.g., 5-methylcytosine). In some embodiments, the modified nucleobase recognized by the agent is a product of a procedure that affects the first nucleobase in the DNA differently from the second nucleobase in the DNA of the sample. In some embodiments, the modified nucleobase may be a "converted nucleobase," meaning that its base pairing specificity was changed by the procedure. For example, certain procedures convert unmethylated or unmodified cytosine to dihydrouracil, or more generally, at least one modified or unmodified form of cytosine undergoes deamination, resulting in uracil (considered a modified nucleobase in the context of DNA) or a further modified form of uracil. Examples of partitioning agents include antibodies, such as antibodies that recognize a modified nucleobase, which may be a modified cytosine, such as a methylcytosine (e.g., 5-methylcytosine). In some embodiments, the partitioning agent is an antibody that recognizes a modified cytosine other than 5-methylcytosine, such as 5-carboxylcytosine (5caC). Alternative partitioning agents include methyl binding domain (MBDs such as MBD2) and methyl binding proteins (MBPs) as described herein, including proteins such as MeCP2.

Additional, non-limiting examples of partitioning agents are histone binding proteins which can separate nucleic acids bound to histones from free or unbound nucleic acids. Examples of histone binding proteins that can be used in the methods disclosed herein include RBBP4, RbAp48 and SANT domain peptides.

The binding of partitioning agents to particular nucleic acids and the partitioning of the nucleic acids into subsamples may occur to a certain extent or may occur in an essentially binary manner. In some instances, nucleic acids comprising a greater proportion of a certain modification bind to the agent at a greater extent than nucleic acids comprising a lesser proportion of the modification. Similarly, the partitioning may produce subsamples comprising greater and lesser proportions of nucleic acids comprising a certain modification. Alternatively, the partitioning may produce subsamples comprising essentially all or none of the nucleic acids comprising the modification. In all instances, various levels of modifications may be sequentially eluted from the partitioning agent.

In some embodiments, partitioning can comprise both binary partitioning and partitioning based on degree/level of modifications. For example, methylated fragments can be partitioned by methylated DNA immunoprecipitation (MeDIP), or all methylated fragments can be partitioned from unmethylated fragments using methyl binding domain proteins (e.g., MethylMinder Methylated DNA Enrichment Kit (ThermoFisher Scientific). Subsequently, additional partitioning may involve eluting fragments having different levels of methylation by adjusting the salt concentration in a solution with the methyl binding domain and bound fragments. As salt concentration increases, fragments having greater methylation levels are eluted.

In some instances, the final partitions are enriched in nucleic acids having different extents of modifications (overrepresentative or underrepresentative of modifications). Overrepresentation and underrepresentation can be defined by the number of modifications born by a nucleic acid relative to the median number of modifications per strand in a population. For example, if the median number of 5-methylcytosine residues in nucleic acid in a sample is 2, a nucleic acid including more than two 5-methylcytosine residues is overrepresented in this modification and a nucleic acid with 1 or zero 5-methylcytosine residues is underrepresented. The effect of the affinity separation is to enrich for nucleic acids overrepresented in a modification in a bound phase and for nucleic acids underrepresented in a modification in an unbound phase (i.e. in solution). The nucleic acids in the bound phase can be eluted before subsequent processing.

When using MeDIP or MethylMiner^{®}Methylated DNA Enrichment Kit (ThermoFisher Scientific) various levels of methylation can be partitioned using sequential elutions. For example, a hypomethylated partition (no methylation) can be separated from a methylated partition by contacting the nucleic acid population with the MBD from the kit, which is attached to magnetic beads. The beads are used to separate out the methylated nucleic acids from the non-methylated nucleic acids. Subsequently, one or more elution steps are performed sequentially to elute nucleic acids having different levels of methylation. For example, a first set of methylated nucleic acids can be eluted at a salt concentration of 160 mM or higher, e.g., at least 150 mM, at least 200 mM, 300 mM, 400 mM, 500 mM, 600 mM, 700 mM, 800 mM, 900 mM, 1000 mM, or 2000 mM. After such methylated nucleic acids are eluted, magnetic separation is once again used to separate higher level of methylated nucleic acids from those with lower level of methylation. The elution and magnetic separation steps can be repeated to create various partitions such as a hypomethylated partition (enriched in nucleic acids comprising no methylation), a methylated partition (enriched in nucleic acids comprising low levels of methylation), and a hyper methylated partition (enriched in nucleic acids comprising high levels of methylation).

In some methods, nucleic acids bound to an agent used for affinity separation based partitioning are subjected to a wash step. The wash step washes off nucleic acids weakly bound to the affinity agent. Such nucleic acids can be enriched in nucleic acids having the modification to an extent close to the mean or median (i.e., intermediate between nucleic acids remaining bound to the solid phase and nucleic acids not binding to the solid phase on initial contacting of the sample with the agent).

The affinity separation results in at least two, and sometimes three or more partitions of nucleic acids with different extents of a modification. While the partitions are still separate, the nucleic acids of at least one partition, and usually two or three (or more) partitions are linked to nucleic acid tags, usually provided as components of adapters, with the nucleic acids in different partitions receiving different tags that distinguish members of one partition from another. The tags linked to nucleic acid molecules of the same partition can be the same or different from one another. But if different from one another, the tags may have part of their code in common so as to identify the molecules to which they are attached as being of a particular partition.

For further details regarding portioning nucleic acid samples based on characteristics such as methylation, see WO2018/119452.

In some embodiments, the nucleic acid molecules can be fractionated into different partitions based on the nucleic acid molecules that are bound to a specific protein or a fragment thereof and those that are not bound to that specific protein or fragment thereof.

Nucleic acid molecules can be fractionated based on DNA-protein binding. Protein-DNA complexes can be fractionated based on a specific property of a protein. Examples of such properties include various epitopes, modifications (e.g., histone methylation or acetylation) or enzymatic activity. Examples of proteins which may bind to DNA and serve as a basis for fractionation may include, but are not limited to, protein A and protein G. Any suitable method can be used to fractionate the nucleic acid molecules based on protein bound regions. Examples of methods used to fractionate nucleic acid molecules based on protein bound regions include, but are not limited to, SDS-PAGE, chromatin-immuno-precipitation (ChIP), heparin chromatography, and asymmetrical field flow fractionation (AF4).

In some embodiments, the partitioning of the sample into a plurality of subsamples is performed by contacting the nucleic acids with an antibody that recognizes a modified nucleobase in the DNA, which may be is a modified cytosine or a product of the procedure that affects the first nucleobase in the DNA differently from the second nucleobase in the DNA of the sample. In some embodiments, the modified nucleobase is 5mC. In some embodiments, the modified nucleobase is 5caC. In some embodiments, the modified nucleobase is dihydrouracil (DHU). In some embodiments, the antibody that recognizes a modified nucleobase in the DNA is used to partition single-stranded DNA.

In some embodiments, the partitioning is performed by contacting the nucleic acids with a methyl binding domain ("MBD") of a methyl binding protein ("MBP"). In some such embodiments, the nucleic acids are contacted with an entire MBP. In some embodiments, an MBD binds to 5-methylcytosine (5mC), and an MBP comprises an MBD and is referred to interchangeably herein as a methyl binding protein or a methyl binding domain protein. In some embodiments, MBD is coupled to paramagnetic beads, such as Dynabeads^{®} M-280 Streptavidin via a biotin linker. Partitioning into fractions with different extents of methylation can be performed by eluting fractions by increasing the NaCl concentration.

In some embodiments, bound DNA is eluted by contacting the antibody or MBD with a protease, such as proteinase K. This may be performed instead of or in addition to elution steps using NaCl as discussed above.

Examples of agents that recognize a modified nucleobase contemplated herein include, but are not limited to:
(a) MeCP2 and MBD2 are a proteins that preferentially binds to 5-methyl-cytosine over unmodified cytosine.
(b) RPL26, PRP8 and the DNA mismatch repair protein MHS6 preferentially bind to 5-hydroxymethyl-cytosine over unmodified cytosine.
(c) FOXK1, FOXK2, FOXP1, FOXP4 and FOXI3 preferably bind to 5-formyl-cytosine over unmodified cytosine (Iurlaro et al., Genome Biol. 14: R119 (2013)).
(d) Antibodies specific to one or more methylated or modified nucleobases or conversion products thereof, such as 5mC, 5caC, or DHU.

In general, elution is a function of the number of modifications, such as the number of methylated sites per molecule, with molecules having more methylation eluting under increased salt concentrations. To elute the DNA into distinct populations based on the extent of methylation, one can use a series of elution buffers of increasing NaCl concentration. Salt concentration can range from about 100 nm to about 2500 mM NaCl. In one embodiment, the process results in three (3) partitions. Molecules are contacted with a solution at a first salt concentration and comprising a molecule comprising an agent that recognizes a modified nucleobase, which molecule can be attached to a capture moiety, such as streptavidin. At the first salt concentration a population of molecules will bind to the agent and a population will remain unbound. The unbound population can be separated as a "hypomethylated" population. For example, a first partition enriched in hypomethylated form of DNA is that which remains unbound at a low salt concentration, e.g., 100 mM or 160 mM. A second partition enriched in intermediate methylated DNA is eluted using an intermediate salt concentration, e.g., between 100 mM and 2000 mM concentration. This is also separated from the sample. A third partition enriched in hypermethylated form of DNA is eluted using a high salt concentration, e.g., at least about 2000 mM.

In some embodiments, a monoclonal antibody raised against 5-methylcytidine (5mC) is used to purify methylated DNA. DNA is denatured, e.g., at 95°C in order to yield single-stranded DNA fragments. Protein G coupled to standard or magnetic beads as well as washes following incubation with the anti-5mC antibody are used to immunoprecipitate DNA bound to the antibody. Such DNA may then be eluted. Partitions may comprise unprecipitated DNA and one or more partitions eluted from the beads.

In some embodiments, sample DNA (e.g., between 5 and 200 ng) is mixed with methyl binding domain (MBD) buffer and magnetic beads conjugated with MBD proteins and incubated overnight. Methylated DNA (hypermethylated DNA) binds the MBD protein on the magnetic beads during this incubation. Non-methylated (hypomethylated DNA) or less methylated DNA (intermediately methylated) is washed away from the beads with buffers containing increasing concentrations of salt. For example, one, two, or more fractions containing non-methylated, hypomethylated, and/or intermediately methylated DNA may be obtained from such washes. Finally, a high salt buffer is used to elute the heavily methylated DNA (hypermethylated DNA) from the MBD protein. In some embodiments, these washes result in three partitions (hypomethylated partition, intermediately methylated fraction and hypermethylated partition) of DNA having increasing levels of methylation.

In some embodiments, the partitions of DNA are desalted and concentrated in preparation for the enzymatic steps of library preparation.

Sequences that comprise structural variations may tend to be hypomethylated. Accordingly, in some embodiments, the DNA contacted with a plurality of primers comprising at least two primers that anneal in a parallel orientation to a target region is DNA from or comprising at least a portion of a hypomethylated partition. The DNA from or comprising a at least a portion of hypomethylated partition may or may not be combined with DNA from or comprising at least a portion of one or more other partitions, such as an intermediate partition or a hypermethylated partition.

### G. Captured set; target regions

In some embodiments, nucleic acids captured or enriched using a method described here comprise one or more captured sets of DNA. In some embodiments, a captured set of DNA comprises a target region set comprising a structural variation, e.g., a rearrangement. In some embodiments, one or more target regions to which a primer anneals comprises a structural variation, e.g., a rearrangement. In some embodiments, the rearrangement is in an intronic target region. In some embodiments, the structural variation is not present in healthy cells. In some embodiments, the structural variation is only present in cancer cells, or is only present in aberrant cells (e.g., hyperplastic, metaplastic, or neoplastic cells). In some embodiments, the structural variation is in an exonic VDJ region. In some such embodiments, the structural variation is due to aberrant VDJ recombination that occurs at a greater frequency in cancer cells than in healthy cells. In some embodiments, the structural variation is a duplication or copy number variation (CNV).

A plurality of primers for detecting a duplication or copy number variation can anneal to a region encompassing a 3' end of a gene (e.g., a stop codon or transcription termination site) or the complement thereof. Where a duplication or copy number variation has occurred, there will generally be a breakpoint after the 3' end beyond which sequence introduced by the duplication or CNV commences. The breakpoint will permit extension of the primer annealing closest to the breakpoint, e.g., essentially as shown in Fig. 1A. In some embodiments, a primer that spans the breakpoint undergoes 3' exonucleolysis and extension, e.g., essentially as shown in Fig. 1B.

In some embodiments, the structural variation is in a cDNA molecule; for example, the structural variation may be a gene fusion. cDNA may be prepared by reverse transcription of mRNA, e.g., from a cell or tissue sample. A plurality of primers for detecting a structural variation in a cDNA, such as a gene fusion, can anneal to the coding sequence. In some embodiments, a blocking oligonucleotide is used to prevent extension of a primer into the 3' untranslated region of the cDNA. Where there is a structural variation, such as a gene fusion, in the cDNA, there will generally be a breakpoint within the coding sequence beyond which sequence introduced by the duplication or CNV commences. The breakpoint will permit extension of the primer annealing closest to the breakpoint, e.g., essentially as shown in Fig. 1A. In some embodiments, a primer that spans the breakpoint undergoes 3' exonucleolysis and extension, e.g., essentially as shown in Fig. 1B.

In some embodiments, a first captured epigenetic target region set captured from a sample or first subsample comprises hypermethylation variable target regions. In some embodiments, the hypermethylation variable target regions show lower methylation in healthy cfDNA than in at least one other tissue type. Without wishing to be bound by any particular theory, cancer cells may shed more DNA into the bloodstream than healthy cells of the same tissue type. As such, the distribution of tissue of origin of cfDNA may change upon carcinogenesis. Thus, an increase in the level of hypermethylation variable target regions in the first subsample can be an indicator of the presence (or recurrence, depending on the history of the subject) of cancer.

In some embodiments, a second captured epigenetic target region set captured from a sample or second subsample comprises hypomethylation variable target regions. In some embodiments, the hypomethylation variable target regions show higher methylation in healthy cfDNA than in at least one other tissue type. Without wishing to be bound by any particular theory, cancer cells may shed more DNA into the bloodstream than healthy cells of the same tissue type. As such, the distribution of tissue of origin of cfDNA may change upon carcinogenesis. Thus, an increase in the level of hypomethylation variable target regions in the second subsample can be an indicator of the presence (or recurrence, depending on the history of the subject) of cancer.

Alternatively, first and second captured target region sets may be provided, comprising, respectively, DNA corresponding to a sequence-variable target region set and DNA corresponding to an epigenetic target region set. The first and second captured sets may be combined to provide a combined captured set. The first and second captured sets may be further combined with captured sets comprising intronic or exonic VDJ target regions to provide a combined captured set.

In some embodiments in which a captured set comprising DNA corresponding to the sequence-variable target region set and the epigenetic target region set includes a combined captured set as discussed above, the DNA corresponding to the sequence-variable target region set may be present at a greater concentration than the DNA corresponding to the epigenetic target region set, e.g., a 1.1 to 1.2-fold greater concentration, a 1.2- to 1.4-fold greater concentration, a 1.4- to 1.6-fold greater concentration, a 1.6- to 1.8-fold greater concentration, a 1.8- to 2.0-fold greater concentration, a 2.0- to 2.2-fold greater concentration, a 2.2- to 2.4-fold greater concentration a 2.4- to 2.6-fold greater concentration, a 2.6- to 2.8-fold greater concentration, a 2.8- to 3.0-fold greater concentration, a 3.0- to 3.5-fold greater concentration, a 3.5- to 4.0, a 4.0- to 4.5-fold greater concentration, a 4.5- to 5.0-fold greater concentration, a 5.0-to 5.5-fold greater concentration, a 5.5- to 6.0-fold greater concentration, a 6.0- to 6.5-fold greater concentration, a 6.5- to 7.0-fold greater, a 7.0- to 7.5-fold greater concentration, a 7.5- to 8.0-fold greater concentration, an 8.0- to 8.5-fold greater concentration, an 8.5- to 9.0-fold greater concentration, a 9.0- to 9.5-fold greater concentration, 9.5- to 10.0-fold greater concentration, a 10- to 11-fold greater concentration, an 11- to 12-fold greater concentration a 12- to 13-fold greater concentration, a 13- to 14-fold greater concentration, a 14- to 15-fold greater concentration, a 15- to 16-fold greater concentration, a 16- to 17-fold greater concentration, a 17- to 18-fold greater concentration, an 18- to 19-fold greater concentration, a 19- to 20-fold greater concentration, a 20- to 30-fold greater concentration, a 30- to 40-fold greater concentration, a 40- to 50-fold greater concentration, a 50- to 60-fold greater concentration, a 60- to 70-fold greater concentration, a 70- to 80-fold greater concentration, a 80-to 90-fold greater concentration, or a 90- to 100-fold greater concentration. The degree of difference in concentrations accounts for normalization for the footprint sizes of the target regions, as discussed in the definition section.

### 1. Intronic target regions

In some embodiments, the captured set of DNA comprises an intronic target region. In some embodiments, the intronic target region comprises one or more introns likely to differentiate DNA from neoplastic (e.g., tumor or cancer) cells and from healthy cells, e.g., non-neoplastic circulating cells. For example, an intron comprising a rearrangement known to be present in some neoplastic cells and absent from healthy cells can be used to differentiate DNA from neoplastic (e.g., tumor or cancer) cells and from healthy cells. In some embodiments, the rearrangement is a translocation. The methods herein facilitate detection of intronic target regions comprising a rearrangement regardless of the location of the breakpoint within the intronic target region; thus, the location of the rearrangement within the intronic target region need not be known prior to carrying out the method. Furthermore, in some embodiments, the methods herein can be used to identify the location of the breakpoint of a rearrangement.

In some embodiments, the intronic target region captured set has a footprint of at least 30 bp, e.g., at least 100 bp, at least 200 bp, at least 500 bp, at least 1 kb, at least 2 kb, at least 5 kb, at least 10 kb, at least 20 kb, at least 50 kb, at least 200 kb, at least 300 kb, or at least 400 kb. In some embodimebps, the ibpronic target region set has a footpribp in the range of 30 bp-1000 kb, e.g., 30 bp-100 bp, 100 bp-200 bp, 200 bp-500 bp, 500 bp-1kb, 1 kb-2 kb, 2 kb-5 kb, 5 kb-10 kb, 10 kb-20 kb, 20 kb-50 kb, 50 kb-100 kb, 100-200 kb, 200-300 kb, 300-400 kb, 400-500 kb, 500-600 kb, 600-700 kb, 700-800 kb, 800-900 kb, and 900-1,000 kb.

Exemplary rearrangements, such as intronic translocations that can be detected using the methods described herein include but are not limited to translocations wherein at least one of the two genes involved in the translocation is a receptor tyrosine kinase. Exemplary translocation products are the BCR-ABL fusion. and fusions comprising any of ALK, FGFR2, FGFR3, NTRK1, RET, or ROS1.

### 2. Exonic VDJ target regions

In some embodiments, the captured set of DNA comprises an exonic VDJ target region. In some embodiments, the exonic VDJ target region is likely to differentiate DNA from proliferating immune cells (e.g., immune cells proliferating in response to a tumor or cancer, or hematological cancer cells such as leukemia, lymphoma, or myeloma cells) relative to DNA from healthy non-immune cells, e.g., non-neoplastic myeloid cells. For example, the presence of an exonic VDJ target region comprising exon-exon fusion at levels higher than the level thereof in DNA extracted from a comparable sample from a healthy subject can be used as an indicator of the likelihood of the presence of a cancer. The methods herein can facilitate detection of exonic VDJ target regions comprising a VDJ recombination rearrangement regardless of the sequence fused to the terminus of a J exon within the target region; thus, the sequence fused to the J exon within the exonic VDJ target region need not be known prior to carrying out the method. Furthermore, in some embodiments, the methods herein can be used to identify the sequence recombined with the J exon.

In some embodiments, the exonic VDJ target region comprises a CDR3, at least in part. In some embodiments, the exonic VDJ target region comprises a J exon. In some embodiments, the exonic VDJ target region is part of a T cell receptor (TCR) coding sequence.

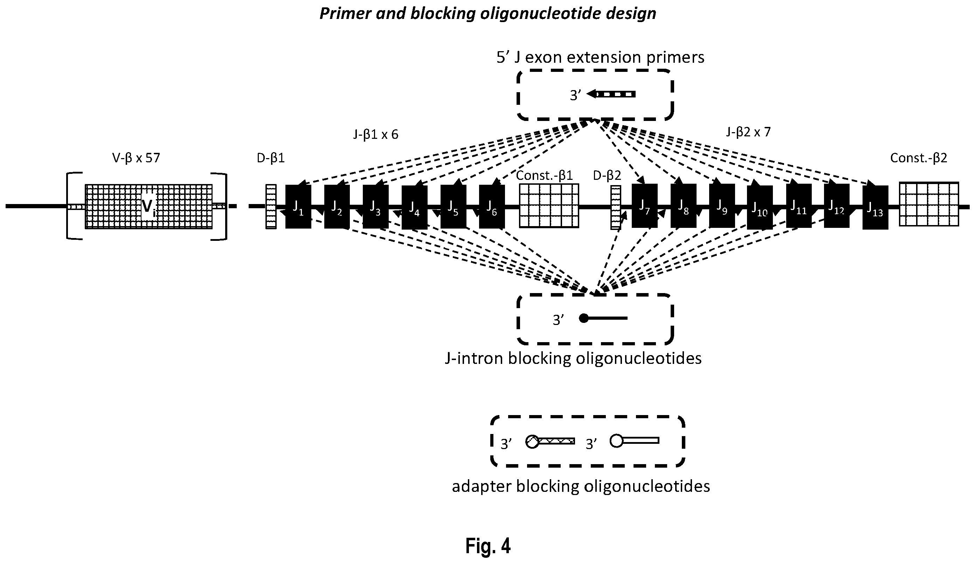

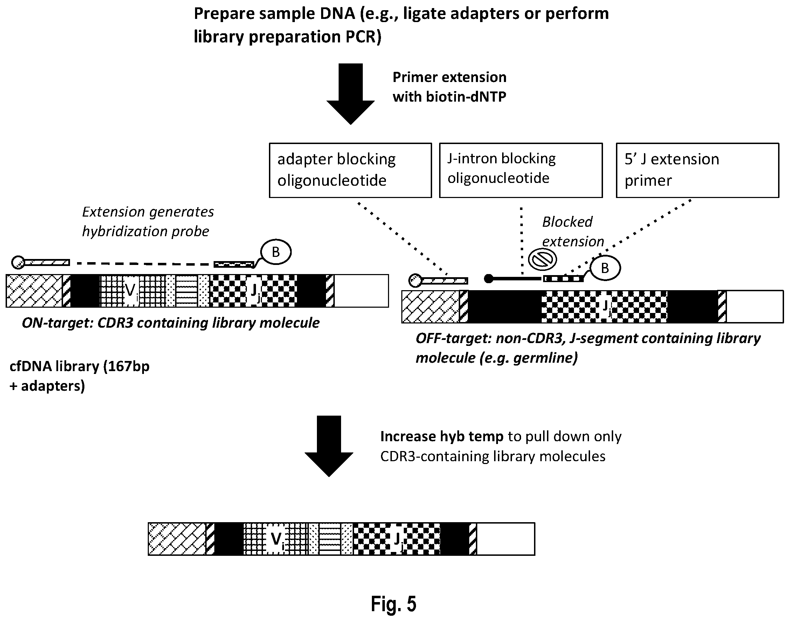

Where an exonic VDJ target region is being analyzed, blocking oligonucleotides can be provided that bind to introns between the germline sequence. For example, where the primers anneal to J exons, blocking oligonucleotides can be provided that bind to the introns between the J exons. See, e.g., Fig. 4. In the exemplary embodiment of Fig. 4, primers are provided that target each J exon, along with blocking oligonucleotides that are non-extendable and that block extension of the primers along germline sequence. Fig. 5 illustrates, on the right, a molecule comprising a J exon that has not been rearranged and that does not comprise a CDR3 (e.g., a germline sequence), where the blocking oligonucleotide blocks extension of a biotinylated primer annealed to the J exon. An adapter-blocking oligonucleotide can also be provided to block extension of the primer annealed to the J exon in case the intron is absent or truncated in the context of a fragment, such as a cfDNA fragment, which comprises an adapter sequence. On the left. Fig. 5 illustrates a CDR3-containing molecule along which the biotinylated primer annealed to the J exon can be extended (dashed line), e.g., through at least part of a V segment. The extension may be eventually blocked by the adapter blocking oligonucleotide but the extension is sufficient to generate a probe with a higher melting temperature than the melting temperature of primers whose extension was blocked as on the right. A pull-down can be performed at an appropriate temperature to preferentially isolate CDR3-containing molecules.

Primers that target a J exon can be oriented to prime extension toward the V exon (e.g., across the D exon if present). The primers can be labeled (e.g., biotinylated) to facilitate isolation (e.g., pull-down) of target regions to which they hybridized, and conditions such as temperature can be set to preferentially isolate VDJ target regions where extension of the primers was not blocked, i.e., rearranged VDJ target regions, which should contain a CDR3 of a rearranged T cell receptor or immunoglobulin coding sequence.

### 3. Epigenetic target region set

The epigenetic target region set may comprise one or more types of target regions likely to differentiate DNA from neoplastic (e.g., tumor or cancer) cells and from healthy cells, e.g., non-neoplastic circulating cells. Exemplary types of such regions are discussed in detail herein. The epigenetic target region set may also comprise one or more control regions, e.g., as described herein.

In some embodiments, the epigenetic target region set has a footprint of at least 100 kbp, e.g., at least 200 kbp, at least 300 kbp, or at least 400 kbp. In some embodiments, the epigenetic target region set has a footprint in the range of 100-20 Mbp, e.g., 100-200 kbp, 200-300 kbp, 300-400 kbp, 400-500 kbp, 500-600 kbp, 600-700 kbp, 700-800 kbp, 800-900 kbp, 900-1,000 kbp, 1-1.5 Mbp, 1.5-2 Mbp, 2-3 Mbp, 3-4 Mbp, 4-5 Mbp, 5-6 Mbp, 6-7 Mbp, 7-8 Mbp, 8-9 Mbp, 9-10 Mbp, or 10-20 Mbp. In some embodiments, the epigenetic target region set has a footprint of at least 20 Mbp.

### a. Hypermethylation variable target regions

In some embodiments, the epigenetic target region set comprises one or more hypermethylation variable target regions. In general, hypermethylation variable target regions refer to regions where an increase in the level of observed methylation, e.g., in a cfDNA sample, indicates an increased likelihood that a sample (e.g., of cfDNA) contains DNA produced by neoplastic cells, such as tumor or cancer cells. For example, hypermethylation of promoters of tumor suppressor genes has been observed repeatedly. See, e.g., Kang et al., Genome Biol. 18:53 (2017) and references cited therein. In another example, as discussed above, hypermethylation variable target regions can include regions that do not necessarily differ in methylation in cancerous tissue relative to DNA from healthy tissue of the same type, but do differ in methylation (e.g., have more methylation) relative to cfDNA that is typical in healthy subjects. Where, for example, the presence of a cancer results in increased cell death such as apoptosis of cells of the tissue type corresponding to the cancer, such a cancer can be detected at least in part using such hypermethylation variable target regions. In some embodiments, the hypermethylation variable target regions are CpG-containing regions that are unmethylated or have low methylation in cfDNA from healthy subjects (e.g., below-average methylation relative to bulk cfDNA).

An extensive discussion of methylation variable target regions in colorectal cancer is provided in Lam et al., Biochim Biophys Acta. 1866:106-20 (2016). These include VIM, SEPT9, ITGA4, OSM4, GATA4 and NDRG4. An exemplary set of hypermethylation variable target regions based on colorectal cancer (CRC) studies is provided in Table 1. Many of these genes likely have relevance to cancers beyond colorectal cancer; for example, TP53 is widely recognized as a critically important tumor suppressor and hypermethylation-based inactivation of this gene may be a common oncogenic mechanism.

**Table 1. Exemplary Hypermethylation Target Regions based on CRC studies.**

| **Gene Name** | **Additional Gene Name** | **Chromosome** |
|---|---|---|
| VIM | | chr10 |
| SEPT9 | | chr17 |
| CYCD2 | CCND2 | chr12 |
| TFPI2 | | chr7 |
| GATA4 | | chr8 |
| RARB2 | RARB | chr3 |
| p16INK4a | CDKN2A | chr9 |
| MGMT | | chr10 |
| APC | | chr5 |
| NDRG4 | | chr16 |
| HLTF | | chr3 |
| HPP1 | TMEFF2 | chr2 |
| hMLH1 | MLH1 | chr3 |
| RASSF1A | RASSF 1 | chr3 |
| CDH13 | | chr16 |
| IGFBP3 | | chr7 |
| ITGA4 | | chr2 |

In some embodiments, the hypermethylation variable target regions comprise a plurality of loci listed in Table 1, e.g., at least 10%, 20%, 30%, 40%, 50%, 60%, 70%, 80%, 90%, or 100% of the loci listed in Table 1. For example, for each locus included as a target region, there may be one or more probes with a hybridization site that binds between the transcription start site and the stop codon (the last stop codon for genes that are alternatively spliced) of the gene, or in the promoter region of the gene. In some embodiments, the one or more probes bind within 300 bp of the transcription start site of a gene in Table 1, e.g., within 200 or 100 bp.

Methylation variable target regions in various types of lung cancer are discussed in detail, e.g., in Ooki et al., Clin. Cancer Res. 23:7141-52 (2017); Belinksy, Annu. Rev. Physiol. 77:453-74 (2015); Hulbert et al., Clin. Cancer Res. 23:1998-2005 (2017); Shi et al., BMC Genomics 18:901 (2017); Schneider et al., BMC Cancer. 11:102 (2011); Lissa et al., Transl Lung Cancer Res 5(5):492-504 (2016); Skvortsova et al., Br. J. Cancer. 94(10):1492-1495 (2006); Kim et al., Cancer Res. 61:3419-3424 (2001); Furonaka et al., Pathology International 55:303-309 (2005); Gomes et al., Rev. Port. Pneumol. 20:20-30 (2014); Kim et al., Oncogene. 20:1765-70 (2001); Hopkins-Donaldson et al., Cell Death Differ. 10:356-64 (2003); Kikuchi et al., Clin. Cancer Res. 11:2954-61 (2005); Heller et al., Oncogene 25:959-968 (2006); Licchesi et al., Carcinogenesis. 29:895-904 (2008); Guo et al., Clin. Cancer Res. 10:7917-24 (2004); Palmisano et al., Cancer Res. 63:4620-4625 (2003); and Toyooka et al., Cancer Res. 61:4556-4560, (2001).

An exemplary set of hypermethylation variable target regions based on lung cancer studies is provided in Table 2. Many of these genes likely have relevance to cancers beyond lung cancer; for example, Casp8 (Caspase 8) is a key enzyme in programmed cell death and hypermethylation-based inactivation of this gene may be a common oncogenic mechanism not limited to lung cancer. Additionally, a number of genes appear in both Tables 1 and 2, indicating generality.

**Table 2. Exemplary Hypermethylation Target Regions based on Lung Cancer studies**

| **Gene Name** | **Chromosome** |
|---|---|
| MARCH11 | chr5 |
| TAC1 | chr7 |
| TCF21 | chr6 |
| SHOX2 | chr3 |
| p16 | chr3 |
| Casp8 | chr2 |
| CDH13 | chr16 |
| MGMT | chr10 |
| MLH1 | chr3 |
| MSH2 | chr2 |
| TSLC1 | chr11 |
| APC | chr5 |
| DKK1 | chr10 |
| DKK3 | chr11 |
| LKB1 | chr11 |
| WIF1 | chr12 |
| RUNX3 | chr1 |
| GATA4 | chr8 |
| GATA5 | chr20 |
| PAX5 | chr9 |
| E-Cadherin | chr16 |
| H-Cadherin | chr16 |

Any of the foregoing embodiments concerning target regions identified in Table 2 may be combined with any of the embodiments described above concerning target regions identified in Table 1. In some embodiments, the hypermethylation variable target regions comprise a plurality of loci listed in Table 1 or Table 2, e.g., at least 10%, 20%, 30%, 40%, 50%, 60%, 70%, 80%, 90%, or 100% of the loci listed in Table 1 or Table 2.

Additional hypermethylation target regions may be obtained, e.g., from the Cancer Genome Atlas. Kang et al., Genome Biology 18:53 (2017), describe construction of a probabilistic method called CancerLocator using hypermethylation target regions from breast, colon, kidney, liver, and lung. In some embodiments, the hypermethylation target regions can be specific to one or more types of cancer. Accordingly, in some embodiments, the hypermethylation target regions include one, two, three, four, or five subsets of hypermethylation target regions that collectively show hypermethylation in one, two, three, four, or five of breast, colon, kidney, liver, and lung cancers.

In some embodiments, where different epigenetic target regions are captured from the first and second subsamples, the epigenetic target regions captured from the first subsample comprise hypermethylation variable target regions.

### b. Hypomethylation variable target regions

Global hypomethylation is a commonly observed phenomenon in various cancers. See, e.g., Hon et al., Genome Res. 22:246-258 (2012) (breast cancer); Ehrlich, Epigenomics 1:239-259 (2009) (review article noting observations of hypomethylation in colon, ovarian, prostate, leukemia, hepatocellular, and cervical cancers). For example, regions such as repeated elements, e.g., LINE1 elements, Alu elements, centromeric tandem repeats, pericentromeric tandem repeats, and satellite DNA, and intergenic regions that are ordinarily methylated in healthy cells may show reduced methylation in tumor cells. Accordingly, in some embodiments, the epigenetic target region set includes hypomethylation variable target regions, where a decrease in the level of observed methylation indicates an increased likelihood that a sample (e.g., of cfDNA) contains DNA produced by neoplastic cells, such as tumor or cancer cells. In another example, as discussed above, hypomethylation variable target regions can include regions that do not necessarily differ in methylation in cancerous tissue relative to DNA from healthy tissue of the same type, but do differ in methylation (e.g., are less methylated) relative to cfDNA that is typical in healthy subjects. Where, for example, the presence of a cancer results in increased cell death such as apoptosis of cells of the tissue type corresponding to the cancer, such a cancer can be detected at least in part using such hypomethylation variable target regions. In some embodiments, the hypomethylation variable target regions are CpG-containing regions that are methylated or have high methylation in cfDNA from healthy subjects (e.g., above-average methylation relative to bulk cfDNA).

In some embodiments, hypomethylation variable target regions include repeated elements and/or intergenic regions. In some embodiments, repeated elements include one, two, three, four, or five of LINE1 elements, Alu elements, centromeric tandem repeats, pericentromeric tandem repeats, and/or satellite DNA.

Exemplary specific genomic regions that show cancer-associated hypomethylation include nucleotides 8403565-8953708 and 151104701-151106035 of human chromosome 1. In some embodiments, the hypomethylation variable target regions overlap or comprise one or both of these regions.

In some embodiments, where different epigenetic target regions are captured from the first and second subsamples, the epigenetic target regions captured from the second subsample comprise hypomethylation variable target regions. In some embodiments, the epigenetic target regions captured from the second subsample comprise hypomethylation variable target regions and the epigenetic target regions captured from the first subsample comprise hypermethylation variable target regions.

### c. CTCF binding regions

CTCF is a DNA-binding protein that contributes to chromatin organization and often colocalizes with cohesin. Perturbation of CTCF binding sites has been reported in a variety of different cancers. *See, e.g.,* Katainen et al., Nature Genetics, doi:10.1038/ng.3335, published online 8 June 2015; Guo et al., Nat. Commun. 9:1520 (2018). CTCF binding results in recognizable patterns in cfDNA that can be detected by sequencing, e.g., through fragment length analysis. Details regarding sequencing-based fragment length analysis are provided in Snyder et al., Cell 164:57-68 (2016); WO 2018/009723; and US20170211143A1.

Thus, perturbations of CTCF binding result in variation in the fragmentation patterns of cfDNA. As such, CTCF binding sites are a type of fragmentation variable target regions.

There are many known CTCF binding sites. See, e.g., the CTCFBSDB (CTCF Binding Site Database), available on the Internet at insulatordb.uthsc.edu/; Cuddapah et al., Genome Res. 19:24-32 (2009); Martin et al., Nat. Struct. Mol. Biol. 18:708-14 (2011); Rhee et al., Cell. 147:1408-19 (2011). Exemplary CTCF binding sites are at nucleotides 56014955-56016161 on chromosome 8 and nucleotides 95359169-95360473 on chromosome 13.

Accordingly, in some embodiments, the epigenetic target region set includes CTCF binding regions. In some embodiments, the CTCF binding regions comprise at least 10, 20, 50, 100, 200, or 500 CTCF binding regions, or 10-20, 20-50, 50-100, 100-200, 200-500, or 500-1000 CTCF binding regions, e.g., such as CTCF binding regions described above or in one or more of CTCFBSDB or the Cuddapah et al., Martin et al., or Rhee et al. articles cited above.

In some embodiments, at least some of the CTCF sites can be methylated or unmethylated, wherein the methylation state is correlated with the whether or not the cell is a cancer cell. In some embodiments, the epigenetic target region set comprises at least 100 bp, at least 200 bp, at least 300 bp, at least 400 bp, at least 500 bp, at least 750 bp, at least 1000 bp upstream and downstream regions of the CTCF binding sites.

### d. Transcription start sites

Transcription start sites may also show perturbations in neoplastic cells. For example, nucleosome organization at various transcription start sites in healthy cells of the hematopoietic lineage-which contributes substantially to cfDNA in healthy individuals-may differ from nucleosome organization at those transcription start sites in neoplastic cells. This results in different cfDNA patterns that can be detected by sequencing, as discussed generally in Snyder et al., Cell 164:57-68 (2016); WO 2018/009723; and US20170211143A1. In another example, transcription start sites may not necessarily differ epigenetically in cancerous tissue relative to DNA from healthy tissue of the same type, but do differ epigenetically (e.g., with respect to nucleosome organization) relative to cfDNA that is typical in healthy subjects. Where, for example, the presence of a cancer results in increased cell death, such as apoptosis, of cells of the tissue type corresponding to the cancer, such a cancer can be detected at least in part using such differences in transcription start sites.

Thus, perturbations of transcription start sites also result in variation in the fragmentation patterns of cfDNA. As such, transcription start sites are also a type of fragmentation variable target regions.

Human transcriptional start sites are available from DBTSS (DataBase of Human Transcription Start Sites), available on the Internet at dbtss.hgc.jp and described in Yamashita et al., Nucleic Acids Res. 34(Database issue): D86-D89 (2006).

Accordingly, in some embodiments, the epigenetic target region set includes transcriptional start sites. In some embodiments, the transcriptional start sites comprise at least 10, 20, 50, 100, 200, or 500 transcriptional start sites, or 10-20, 20-50, 50-100, 100-200, 200-500, or 500-1000 transcriptional start sites, e.g., such as transcriptional start sites listed in DBTSS. In some embodiments, at least some of the transcription start sites can be methylated or unmethylated, wherein the methylation state is correlated with whether or not the cell is a cancer cell. In some embodiments, the epigenetic target region set comprises at least 100 bp, at least 200 bp, at least 300 bp, at least 400 bp, at least 500 bp, at least 750 bp, at least 1000 bp upstream and downstream regions of the transcription start sites.

### e. Focal amplifications

Although focal amplifications are somatic mutations, they can be detected by sequencing based on read frequency in a manner analogous to approaches for detecting certain epigenetic changes such as changes in methylation. As such, regions that may show focal amplifications in cancer can be included in the epigenetic target region set and may comprise one or more of AR, BRAF, CCND1, CCND2, CCNE1, CDK4, CDK6, EGFR, ERBB2, FGFR1, FGFR2, KIT, KRAS, MET, MYC, PDGFRA, PIK3CA, and RAF1. For example, in some embodiments, the epigenetic target region set comprises at least 2, 3, 4, 5, 6, 7, 8, 9, 10, 11, 12, 13, 14, 15, 16, 17, or 18 of the foregoing targets.

### f. Methylation control regions

It can be useful to include control regions to facilitate data validation. In some embodiments, the epigenetic target region set includes control regions that are expected to be methylated or unmethylated in essentially all samples, regardless of whether the DNA is derived from a cancer cell or a normal cell. In some embodiments, the epigenetic target region set includes control hypomethylated regions that are expected to be hypomethylated in essentially all samples. In some embodiments, the epigenetic target region set includes control hypermethylated regions that are expected to be hypermethylated in essentially all samples.

### 4. Sequence-variable target region set

In some embodiments, the sequence-variable target region set comprises a plurality of regions known to undergo somatic mutations in cancer.

In some aspects, the sequence-variable target region set targets a plurality of different genes or genomic regions ("panel") selected such that a determined proportion of subjects having a cancer exhibits a genetic variant or tumor marker in one or more different genes or genomic regions in the panel. The panel may be selected to limit a region for sequencing to a fixed number of base pairs. The panel may be selected to sequence a desired amount of DNA, e.g., by adjusting the affinity and/or amount of the probes as described elsewhere herein. The panel may be further selected to achieve a desired sequence read depth. The panel may be selected to achieve a desired sequence read depth or sequence read coverage for an amount of sequenced base pairs. The panel may be selected to achieve a theoretical sensitivity, a theoretical specificity, and/or a theoretical accuracy for detecting one or more genetic variants in a sample.

Probes for detecting the panel of regions can include those for detecting genomic regions of interest (hotspot regions). Information about chromatin structure can be taken into account in designing probes, and/or probes can be designed to maximize the likelihood that particular sites (e.g., KRAS codons 12 and 13) can be captured, and may be designed to optimize capture based on analysis of cfDNA coverage and fragment size variation impacted by nucleosome binding patterns and GC sequence composition. Regions used herein can also include non-hotspot regions optimized based on nucleosome positions and GC models.

Examples of listings of genomic locations of interest may be found in Table 3 and Table 4. In some embodiments, a sequence-variable target region set used in the methods of the present disclosure comprises at least a portion of at least 5, at least 10, at least 15, at least 20, at least 25, at least 30, at least 35, at least 40, at least 45, at least 50, at least 55, at least 60, at least 65, or 70 of the genes of Table 3. In some embodiments, a sequence-variable target region set used in the methods of the present disclosure comprises at least 5, at least 10, at least 15, at least 20, at least 25, at least 30, at least 35, at least 40, at least 45, at least 50, at least 55, at least 60, at least 65, or 70 of the SNVs of Table 3. In some embodiments, a sequence-variable target region set used in the methods of the present disclosure comprises at least 1, at least 2, at least 3, at least 4, at least 5, or 6 of the fusions of Table 3. In some embodiments, a sequence-variable target region set used in the methods of the present disclosure comprise at least a portion of at least 1, at least 2, or 3 of the indels of Table 3. In some embodiments, a sequence-variable target region set used in the methods of the present disclosure comprises at least a portion of at least 5, at least 10, at least 15, at least 20, at least 25, at least 30, at least 35, at least 40, at least 45, at least 50, at least 55, at least 60, at least 65, at least 70, or 73 of the genes of Table 4. In some embodiments, a sequence-variable target region set used in the methods of the present disclosure comprises at least 5, at least 10, at least 15, at least 20, at least 25, at least 30, at least 35, at least 40, at least 45, at least 50, at least 55, at least 60, at least 65, at least 70, or 73 of the SNVs of Table 4. In some embodiments, a sequence-variable target region set used in the methods of the present disclosure comprises at least 1, at least 2, at least 3, at least 4, at least 5, or 6 of the fusions of Table 4. In some embodiments, a sequence-variable target region set used in the methods of the present disclosure comprises at least a portion of at least 1, at least 2, at least 3, at least 4, at least 5, at least 6, at least 7, at least 8, at least 9, at least 10, at least 11, at least 12, at least 13, at least 14, at least 15, at least 16, at least 17, or 18 of the indels of Table 4. Each of these genomic locations of interest may be identified as a backbone region or hot-spot region for a given panel. An example of a listing of hot-spot genomic locations of interest may be found in Table 5. In some embodiments, a sequence-variable target region set used in the methods of the present disclosure comprises at least a portion of at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, or at least 20 of the genes of Table 5. Each hot-spot genomic region is listed with several characteristics, including the associated gene, chromosome on which it resides, the start and stop position of the genome representing the gene's locus, the length of the gene's locus in base pairs, the exons covered by the gene, and the critical feature (e.g., type of mutation) that a given genomic region of interest may seek to capture.

**Table 3**

| **Point Mutations (SNVs)** | | | | | | **Fusions** |
|---|---|---|---|---|---|---|
| AKT1 | ALK | APC | AR | ARAF | ARID1A | ALK |
| ATM | BRAF | BRCA1 | BRCA2 | CCND1 | CCND2 | FGFR2 |
| CCNE1 | CDH1 | CDK4 | CDK6 | CDKN2A | CDKN2B | FGFR3 |
| CTNNB1 | EGFR | ERBB2 | ESR1 | EZH2 | FBXW7 | NTRK1 |
| FGFR1 | FGFR2 | FGFR3 | GATA3 | GNA11 | GNAQ | RET |
| GNAS | HNF1A | HRAS | IDH1 | IDH2 | JAK2 | ROS1 |
| JAK3 | KIT | KRAS | MAP2K 1 | MAP2K2 | MET | |
| MLH1 | MPL | MYC | NF1 | NFE2L2 | NOTCH1 | |
| NPM1 | NRAS | NTRK1 | PDGFRA | PIK3CA | PTEN | |
| PTPN11 | RAF1 | RB1 | RET | RHEB | RHOA | |
| RIT1 | ROS1 | SMAD4 | SMO | SRC | STK11 | |
| TERT | TP53 | TSC1 | VHL | | | |

**Table 4**

| **Point Mutations (SNVs)** | | | | | | **Fusions** |
|---|---|---|---|---|---|---|
| AKT1 | ALK | APC | AR | ARAF | ARID1A | ALK |
| ATM | BRAF | BRCA1 | BRCA2 | CCND1 | CCND2 | FGFR2 |
| CCNE1 | CDH1 | CDK4 | CDK6 | CDKN2A | DDR2 | FGFR3 |
| CTNNB1 | EGFR | ERBB2 | ESR1 | EZH2 | FBXW7 | NTRK1 |
| FGFR1 | FGFR2 | FGFR3 | GATA3 | GNA11 | GNAQ | RET |
| GNAS | HNF1A | HRAS | IDH1 | IDH2 | JAK2 | ROS1 |
| JAK3 | KIT | KRAS | MAP2K 1 | MAP2K2 | MET | |
| MLH1 | MPL | MYC | NF1 | NFE2L2 | NOTCH1 | |
| NPM1 | NRAS | NTRK1 | PDGFRA | PIK3CA | PTEN | |
| PTPN11 | RAF1 | RB1 | RET | RHEB | RHOA | |
| RIT1 | ROS1 | SMAD4 | SMO | MAPK1 | STK11 | |
| TERT | TP53 | TSC1 | VHL | MAPK3 | MTOR | |
| NTRK3 | | | | | | |

**Table 5**

| **Gene** | **Chromosome** | **Start Position** | **Stop Position** | **Length (bp)** | **Exons Covered** | **Critical Feature** |
|---|---|---|---|---|---|---|
| ALK | chr2 | 29446405 | 29446655 | 250 | intron 19 | Fusion |
| ALK | chr2 | 29446062 | 29446197 | 135 | intron 20 | Fusion |
| ALK | chr2 | 29446198 | 29446404 | 206 | 20 | Fusion |
| ALK | chr2 | 29447353 | 29447473 | 120 | intron 19 | Fusion |
| ALK | chr2 | 29447614 | 29448316 | 702 | intron 19 | Fusion |
| ALK | chr2 | 29448317 | 29448441 | 124 | 19 | Fusion |
| ALK | chr2 | 29449366 | 29449777 | 411 | intron 18 | Fusion |
| ALK | chr2 | 29449778 | 29449950 | 172 | 18 | Fusion |
| BRAF | chr7 | 140453064 | 140453203 | 139 | 15 | BRAF V600 |
| CTNNB1 | chr3 | 41266007 | 41266254 | 247 | 3 | S37 |
| EGFR | chr7 | 55240528 | 55240827 | 299 | 18 and 19 | G719 and deletions |
| EGFR | chr7 | 55241603 | 55241746 | 143 | 20 | Insertions/T790M |
| EGFR | chr7 | 55242404 | 55242523 | 119 | 21 | L858R |
| ERBB2 | chr17 | 37880952 | 37881174 | 222 | 20 | Insertions |
| ESR1 | chr6 | 152419857 | 152420111 | 254 | 10 | V534, P535, L536, Y537, D538 |
| FGFR2 | chr10 | 123279482 | 123279693 | 211 | 6 | S252 |
| GATA3 | chr10 | 8111426 | 8111571 | 145 | 5 | SS / Indels |
| GATA3 | chr10 | 8115692 | 8116002 | 310 | 6 | SS / Indels |
| GNAS | chr20 | 57484395 | 57484488 | 93 | 8 | R844 |
| IDH1 | chr2 | 209113083 | 209113394 | 311 | 4 | R132 |
| IDH2 | chr15 | 90631809 | 90631989 | 180 | 4 | R140, R172 |
| KIT | chr4 | 55524171 | 55524258 | 87 | 1 | |
| KIT | chr4 | 55561667 | 55561957 | 290 | 2 | |
| KIT | chr4 | 55564439 | 55564741 | 302 | 3 | |
| KIT | chr4 | 55565785 | 55565942 | 157 | 4 | |
| KIT | chr4 | 55569879 | 55570068 | 189 | 5 | |
| KIT | chr4 | 55573253 | 55573463 | 210 | 6 | |
| KIT | chr4 | 55575579 | 55575719 | 140 | 7 | |
| KIT | chr4 | 55589739 | 55589874 | 135 | 8 | |
| KIT | chr4 | 55592012 | 55592226 | 214 | 9 | |
| KIT | chr4 | 55593373 | 55593718 | 345 | 10 and 11 | 557, 559, 560, 576 |
| KIT | chr4 | 55593978 | 55594297 | 319 | 12 and 13 | V654 |
| KIT | chr4 | 55595490 | 55595661 | 171 | 14 | T670, S709 |
| KIT | chr4 | 55597483 | 55597595 | 112 | 15 | D716 |
| KIT | chr4 | 55598026 | 55598174 | 148 | 16 | L783 |
| KIT | chr4 | 55599225 | 55599368 | 143 | 17 | C809, R815, D816, L818, D820, S821F, N822, Y823 |
| KIT | chr4 | 55602653 | 55602785 | 132 | 18 | A829P |
| KIT | chr4 | 55602876 | 55602996 | 120 | 19 | |
| KIT | chr4 | 55603330 | 55603456 | 126 | 20 | |
| KIT | chr4 | 55604584 | 55604733 | 149 | 21 | |
| KRAS | chr12 | 25378537 | 25378717 | 180 | 4 | A146 |
| KRAS | chr12 | 25380157 | 25380356 | 199 | 3 | Q61 |
| KRAS | chr12 | 25398197 | 25398328 | 131 | 2 | G12/G13 |
| MET | chr7 | 116411535 | 116412255 | 720 | 13, 14, intron 13, intron 14 | MET exon 14 SS |
| NRAS | chr1 | 115256410 | 115256609 | 199 | 3 | Q61 |
| NRAS | chr1 | 115258660 | 115258791 | 131 | 2 | G12/G13 |
| PIK3CA | chr3 | 178935987 | 178936132 | 145 | 10 | E545K |
| PIK3CA | chr3 | 178951871 | 178952162 | 291 | 21 | H1047R |
| PTEN | chr10 | 89692759 | 89693018 | 259 | 5 | R130 |
| SMAD4 | chr18 | 48604616 | 48604849 | 233 | 12 | D537 |
| TERT | chr5 | 1294841 | 1295512 | 671 | promoter | chr5:1295228 |
| TP53 | chr17 | 7573916 | 7574043 | 127 | 11 | Q331, R337, R342 |
| TP53 | chr17 | 7577008 | 7577165 | 157 | 8 | R273 |
| TP53 | chr17 | 7577488 | 7577618 | 130 | 7 | R248 |
| TP53 | chr17 | 7578127 | 7578299 | 172 | 6 | R213/Y220 |
| TP53 | chr17 | 7578360 | 7578564 | 204 | 5 | R175 / Deletions |
| TP53 | chr17 | 7579301 | 7579600 | 299 | 4 | |
| | | | | 12574 (total target region) | | |
| | | | | 16330 (total probe coverage) | | |

Additionally or alternatively, suitable target region sets are available from the literature. For example, Gale et al., PLoS One 13: e0194630 (2018), describes a panel of 35 cancer-related gene targets that can be used as part or all of a sequence-variable target region set. These 35 targets are AKT1, ALK, BRAF, CCND1, CDK2A, CTNNB1, EGFR, ERBB2, ESR1, FGFR1, FGFR2, FGFR3, FOXL2, GATA3, GNA11, GNAQ, GNAS, HRAS, IDH1, IDH2, KIT, KRAS, MED12, MET, MYC, NFE2L2, NRAS, PDGFRA, PIK3CA, PPP2R1A, PTEN, RET, STK11, TP53, and U2AF1.

In some embodiments, the sequence-variable target region set comprises target regions from at least 10, 20, 30, or 35 cancer-related genes, such as the cancer-related genes listed above.

In some embodiments, the sequence-variable target region set has a footprint of at least 50 kbp, e.g., at least 100 kbp, at least 200 kbp, at least 300 kbp, or at least 400 kbp. In some embodiments, the sequence-variable target region set has a footprint in the range of 100-2000 kbp, e.g., 100-200 kbp, 200-300 kbp, 300-400 kbp, 400-500 kbp, 500-600 kbp, 600-700 kbp, 700-800 kbp, 800-900 kbp, 900-1,000 kbp, 1-1.5 Mbp or 1.5-2 Mbp. In some embodiments, the sequence-variable target region set has a footprint of at least 2 Mbp.

### H. Subjects

In some embodiments, the DNA (e.g., cfDNA) is obtained from a subject having a cancer. In some embodiments, the DNA (e.g., cfDNA) is obtained from a subject suspected of having a cancer. In some embodiments, the DNA (e.g., cfDNA) is obtained from a subject having a tumor. In some embodiments, the DNA (e.g., cfDNA) is obtained from a subject suspected of having a tumor. In some embodiments, the DNA (e.g., cfDNA) is obtained from a subject having neoplasia. In some embodiments, the DNA (e.g., cfDNA) is obtained from a subject suspected of having neoplasia. In some embodiments, the DNA (e.g., cfDNA) is obtained from a subject in remission from a tumor, cancer, or neoplasia (e.g., following chemotherapy, surgical resection, radiation, or a combination thereof). In any of the foregoing embodiments, the cancer, tumor, or neoplasia or suspected cancer, tumor, or neoplasia may be of the lung, colon, rectum, kidney, breast, prostate, or liver. In some embodiments, the cancer, tumor, or neoplasia or suspected cancer, tumor, or neoplasia is of the lung. In some embodiments, the cancer, tumor, or neoplasia or suspected cancer, tumor, or neoplasia is of the colon or rectum. In some embodiments, the cancer, tumor, or neoplasia or suspected cancer, tumor, or neoplasia is of the breast. In some embodiments, the cancer, tumor, or neoplasia or suspected cancer, tumor, or neoplasia is of the prostate. In any of the foregoing embodiments, the subject may be a human subject.

### I. Pooling of DNA from samples or subsamples or portions thereof

In some embodiments, the methods herein comprise preparing a pool comprising at least a portion of the DNA of a second partitioned subsample (such as a hypomethylated partition) and at least a portion of the DNA of a first partitioned subsample (such as a hypermethylated partition). Target regions, e.g., including intronic target regions, exonic VDJ target regions, epigenetic target regions and/or sequence-variable target regions, may be captured from a pool. The steps of capturing a target region set from at least an aliquot or portion of a sample or subsample described elsewhere herein encompass capture steps performed on a pool comprising DNA from first and second subsamples. A step of amplifying DNA in a pool may be performed before capturing target regions from the pool. The capturing step may have any of the features described for capturing steps elsewhere herein.

In some embodiments, sequence-variable target regions are captured from a second portion of a partitioned subsample. The second portion may include some, a majority, substantially all, or all of the DNA of the subsample that was not included in the pool. The regions captured from the pool and from the subsample may be combined and analyzed in parallel.

The epigenetic target regions may show differences in methylation levels and/or fragmentation patterns depending on whether they originated from a tumor or from healthy cells, or what type of tissue they originated from, as discussed elsewhere herein. The sequence-variable target regions may show differences in sequence depending on whether they originated from a tumor or from healthy cells.

Analysis of epigenetic target regions from the hypomethylated partition may be less informative in some applications than analysis of sequence-variable target regions from the hypermethylated and hypomethylated partitions and epigenetic target regions from the hypermethylated partition. As such, in methods where sequence-variable target regions and epigenetic target regions are being captured, the latter may be captured to a lesser extent than one or more of the sequence-variable target regions from the hypermethylated and hypomethylated partitions and epigenetic target regions from the hypermethylated partition. For example, sequence-variable target regions can be captured from the portion of the hypomethylated partition not pooled with the hypermethylated partition, and the pool can be prepared with some (e.g., a majority, substantially all, or all) of the DNA from the hypermethylated partition and none or some (e.g., a minority) of the DNA from the hypomethylated partition. Such approaches can reduce or eliminate sequencing of epigenetic target regions from the hypomethylated partition, thereby reducing the amount of sequencing data that suffices for further analysis.

In some embodiments, including a minority of the DNA of the hypomethylated partition in the pool facilitates quantification of one or more epigenetic features (e.g., methylation or other epigenetic feature(s) discussed in detail elsewhere herein), e.g., on a relative basis.

In some embodiments, the pool comprises a minority of the DNA of the hypomethylated partition, e.g., less than about 50% of the DNA of the hypomethylated partition, such as less than or equal to about 45%, 40%, 35%, 30%, 25%, 20%, 15%, 10%, or 5% of the DNA of the hypomethylated partition. In some embodiments, the pool comprises about 5%-25% of the DNA of the hypomethylated partition. In some embodiments, the pool comprises about 10%-20% of the DNA of the hypomethylated partition. In some embodiments, the pool comprises about 10% of the DNA of the hypomethylated partition. In some embodiments, the pool comprises about 15% of the DNA of the hypomethylated partition. In some embodiments, the pool comprises about 20% of the DNA of the hypomethylated partition.

In some embodiments, the pool comprises a portion of the hypermethylated partition, which may be at least about 50% of the DNA of the hypermethylated partition. For example, the pool may comprise at least about 55%, 60%, 65%, 70%, 75%, 80%, 85%, 90%, or 95% of the DNA of the hypermethylated partition. In some embodiments, the pool comprises 50-55%, 55-60%, 60-65%, 65-70%, 70-75%, 75-80%, 80-85%, 85-90%, 90-95%, or 95-100% of the DNA of the hypermethylated partition. In some embodiments, the second pool comprises all or substantially all of the hypermethylated partition.

In some embodiments, the methods comprise preparing a first pool comprising at least a portion of the DNA of the hypomethylated partition. In some embodiments, the methods comprise preparing a second pool comprising at least a portion of the DNA of the hypermethylated partition. In some embodiments, the first pool further comprises a portion of the DNA of the hypermethylated partition. In some embodiments, the second pool further comprises a portion of the DNA of the hypomethylated partition. In some embodiments, the first pool comprises a majority of the DNA of the hypomethylated partition, and optionally and a minority of the DNA of the hypermethylated partition. In some embodiments, the second pool comprises a majority of the DNA of the hypermethylated partition and a minority of the DNA of the hypomethylated partition. In some embodiments involving an intermediately methylated partition, the second pool comprises at least a portion of the DNA of the intermediately methylated partition, e.g., a majority of the DNA of the intermediately methylated partition. In some embodiments, the first pool comprises a majority of the DNA of the hypomethylated partition, and the second pool comprises a majority of the DNA of the hypermethylated partition and a majority of the DNA of the intermediately methylated partition.

In some embodiments, the methods comprise capturing at least a first set of target regions from the first pool, e.g., wherein the first pool is as set forth in any of the embodiments above. In some embodiments, the first set comprises sequence-variable target regions. In some embodiments, the first set comprises hypomethylation variable target regions and/or fragmentation variable target regions. In some embodiments, the first set comprises sequence-variable target regions and fragmentation variable target regions. In some embodiments, the first set comprises sequence-variable target regions, hypomethylation variable target regions and fragmentation variable target regions. A step of amplifying DNA in the first pool may be performed before this capture step. In some embodiments, capturing the first set of target regions from the first pool comprises contacting the DNA of the first pool with a first set of target-specific probes. In some embodiments, the first set of target-specific probes comprises target-binding probes specific for the sequence-variable target regions. In some embodiments, the first set of target-specific probes comprises target-binding probes specific for the sequence-variable target regions, hypomethylation variable target regions and/or fragmentation variable target regions.

In some embodiments, the methods comprise capturing a second set of target regions or plurality of sets of target regions from the second pool, e.g., wherein the first pool is as set forth in any of the embodiments above. In some embodiments, the second plurality comprises epigenetic target regions, such as hypermethylation variable target regions and/or fragmentation variable target regions. In some embodiments, the second plurality comprises sequence-variable target regions and epigenetic target regions, such as hypermethylation variable target regions and/or fragmentation variable target regions. A step of amplifying DNA in the second pool may be performed before this capture step. In some embodiments, capturing the second plurality of sets of target regions from the second pool comprises contacting the DNA of the first pool with a second set of target-specific probes, wherein the second set of target-specific probes comprises target-binding probes specific for the sequence-variable target regions and target-binding probes specific for the epigenetic target regions. In some embodiments, the first set of target regions and the second set of target regions are not identical. For example, the first set of target regions may comprise one or more target regions not present in the second set of target regions. Alternatively or in addition, the second set of target regions may comprise one or more target regions not present in the first set of target regions. In some embodiments, at least one hypermethylation variable target region is captured from the second pool but not from the first pool. In some embodiments, a plurality of hypermethylation variable target regions are captured from the second pool but not from the first pool. In some embodiments, the first set of target regions comprises sequence-variable target regions and/or the second set of target regions comprises epigenetic target regions. In some embodiments, the first set of target regions comprises sequence-variable target regions, and fragmentation variable target regions; and the second set of target regions comprises epigenetic target regions, such as hypermethylation variable target regions and fragmentation variable target regions. In some embodiments, the first set of target regions comprises sequence-variable target regions, fragmentation variable target regions, and comprises hypomethylation variable target regions; and the second set of target regions comprises epigenetic target regions, such as hypermethylation variable target regions and fragmentation variable target regions.

In some embodiments, the first pool comprises a majority of the DNA of the hypomethylated partition and a portion of the DNA of the hypermethylated partition (e.g., about half), and the second pool comprises a portion of the DNA of the hypermethylated partition (e.g., about half). In some such embodiments, the first set of target regions comprises sequence-variable target regions and/or the second set of target regions comprises epigenetic target regions. The sequence-variable target regions and/or the epigenetic target regions may be as set forth in any of the embodiments described elsewhere herein.

### J. Sequencing

In general, sample nucleic acids flanked by adapters with or without prior amplification can be subject to sequencing. Sequencing methods include, for example, Sanger sequencing, high-throughput sequencing, pyrosequencing, sequencing-by-synthesis, single-molecule sequencing, nanopore sequencing, semiconductor sequencing, sequencing-by-ligation, sequencing-by-hybridization, Digital Gene Expression (Helicos), Next generation sequencing (NGS), Single Molecule Sequencing by Synthesis (SMSS) (Helicos), massively-parallel sequencing, Clonal Single Molecule Array (Solexa), shotgun sequencing, Ion Torrent, Oxford Nanopore, Roche Genia, Maxim-Gilbert sequencing, primer walking, and sequencing using PacBio, SOLiD, Ion Torrent, or Nanopore platforms. Sequencing reactions can be performed in a variety of sample processing units, which may multiple lanes, multiple channels, multiple wells, or other mean of processing multiple sample sets substantially simultaneously. Sample processing unit can also include multiple sample chambers to enable processing of multiple runs simultaneously.

The sequencing reactions can be performed on one or more forms of nucleic acids at least one of which is known to contain markers of cancer or of other disease. The sequencing reactions can also be performed on any nucleic acid fragments present in the sample. In some embodiments, sequence coverage of the genome may be less than 5%, 10%, 15%, 20%, 25%, 30%, 40%, 50%, 60%, 70%, 80%, 90%, 95%, 99%, 99.9% or 100%. In some embodiments, the sequence reactions may provide for sequence coverage of at least 5%, 10%, 15%, 20%, 25%, 30%, 40%, 50%, 60%, 70%, or 80% of the genome. Sequence coverage can performed on at least 5, 10, 20, 70, 100, 200 or 500 different genes, or at most 5000, 2500, 1000, 500 or 100 different genes.

Simultaneous sequencing reactions may be performed using multiplex sequencing. In some cases, cell-free nucleic acids may be sequenced with at least 1000, 2000, 3000, 4000, 5000, 6000, 7000, 8000, 9000, 10000, 50000, 100,000 sequencing reactions. In other cases cell-free nucleic acids may be sequenced with less than 1000, 2000, 3000, 4000, 5000, 6000, 7000, 8000, 9000, 10000, 50000, 100,000 sequencing reactions. Sequencing reactions may be performed sequentially or simultaneously. Subsequent data analysis may be performed on all or part of the sequencing reactions. In some cases, data analysis may be performed on at least 1000, 2000, 3000, 4000, 5000, 6000, 7000, 8000, 9000, 10000, 50000, 100,000 sequencing reactions. In other cases, data analysis may be performed on less than 1000, 2000, 3000, 4000, 5000, 6000, 7000, 8000, 9000, 10000, 50000, 100,000 sequencing reactions. An exemplary read depth is 1000-50000 reads per locus (base).

### 1. Differential depth of sequencing

In some embodiments, primer-extended products or nucleic acids hybridized to primer-extended products, or nucleic acids corresponding to a sequence-variable target region set are sequenced to a greater depth of sequencing than nucleic acids corresponding to an epigenetic target region set. For example, the depth of sequencing for nucleic acids corresponding to primer-extended products or sequence variant target region sets may be at least 1.25-, 1.5-, 1.75-, 2-, 2.25-, 2.5-, 2.75-, 3-, 3.5-, 4-, 4.5-, 5-, 6-, 7-, 8-, 9-, 10-, 11-, 12-, 13-, 14-, or 15-fold greater, or 1.25- to 1.5-, 1.5- to 1.75-, 1.75- to 2-, 2- to 2.25-, 2.25- to 2.5-, 2.5- to 2.75-, 2.75- to 3-, 3- to 3.5-, 3.5- to 4-, 4- to 4.5-, 4.5- to 5-, 5- to 5.5-, 5.5- to 6-, 6- to 7-, 7- to 8-, 8- to 9-, 9- to 10-, 10-to 11-, 11- to 12-, 13- to 14-, 14- to 15-fold, or 15- to 100-fold greater, than the depth of sequencing for nucleic acids corresponding to an epigenetic target region set. In some embodiments, said depth of sequencing is at least 2-fold greater. In some embodiments, said depth of sequencing is at least 5-fold greater. In some embodiments, said depth of sequencing is at least 10-fold greater. In some embodiments, said depth of sequencing is 4- to 10-fold greater. In some embodiments, said depth of sequencing is 4- to 100-fold greater.

In some embodiments, the captured cfDNA corresponding to primer-extended products, a sequence-variable target region set, and/or to an epigenetic target region set are sequenced concurrently, e.g., in the same sequencing cell (such as the flow cell of an Illumina sequencer) and/or in the same composition, which may be a pooled composition resulting from recombining separately captured sets or a composition obtained by capturing the cfDNA corresponding to the primer-extended products, sequence-variable target region set, and/or the captured cfDNA corresponding to an epigenetic target region set in the same vessel.

### K. Analysis

In some embodiments, a method described herein comprises detecting one or more structural variations relative to a reference sequence, such as a human genome reference sequence (e.g., hg19 or grch38). The one or more structural variations may comprise a rearrangement, such as any of the types of rearrangements described elsewhere herein. The one or more structural variations may include mutations such as, for example, copy number variants or variations (CNVs)/aberrations, insertions or deletions (indels), gene fusions, transversions, translocations, frame shifts, duplications, and repeat expansions. A structural variation can be a germline or somatic structural variation. In some embodiments, a reference sequence for purposes of comparison is a wild type genomic sequence of the species of the subject providing a test sample, typically the human genome.

In some embodiments, a method described herein comprises identifying the presence of DNA produced by a tumor (or neoplastic cells, or cancer cells).

The present methods can be used to diagnose presence of conditions, particularly cancer, in a subject, to characterize conditions (e.g., staging cancer or determining heterogeneity of a cancer), monitor response to treatment of a condition, effect prognosis risk of developing a condition or subsequent course of a condition. The present disclosure can also be useful in determining the efficacy of a particular treatment option. Successful treatment options may increase the amount of copy number variation or rare mutations detected in subject's blood if the treatment is successful as more cancers may die and shed DNA. In other examples, this may not occur. In another example, perhaps certain treatment options may be correlated with genetic profiles of cancers over time. This correlation may be useful in selecting a therapy.

Additionally, if a cancer is observed to be in remission after treatment, the present methods can be used to monitor residual disease or recurrence of disease.

The types and number of cancers that may be detected may include blood cancers, brain cancers, lung cancers, skin cancers, nose cancers, throat cancers, liver cancers, bone cancers, lymphomas, pancreatic cancers, skin cancers, bowel cancers, rectal cancers, thyroid cancers, bladder cancers, kidney cancers, mouth cancers, stomach cancers, solid state tumors, heterogeneous tumors, homogenous tumors and the like. Type and/or stage of cancer can be detected from genetic variations including mutations, rare mutations, indels, copy number variations, transversions, translocations, recombination, inversion, deletions, aneuploidy, partial aneuploidy, polyploidy, chromosomal instability, chromosomal structure alterations, gene fusions, chromosome fusions, gene truncations, gene amplification, gene duplications, chromosomal lesions, DNA lesions, abnormal changes in nucleic acid chemical modifications, abnormal changes in epigenetic patterns, and abnormal changes in nucleic acid 5-methylcytosine.

Genetic data can also be used for characterizing a specific form of cancer. Cancers are often heterogeneous in both composition and staging. Genetic profile data may allow characterization of specific sub-types of cancer that may be important in the diagnosis or treatment of that specific sub-type. This information may also provide a subject or practitioner clues regarding the prognosis of a specific type of cancer and allow either a subject or practitioner to adapt treatment options in accord with the progress of the disease. Some cancers can progress to become more aggressive and genetically unstable. Other cancers may remain benign, inactive or dormant. The system and methods of this disclosure may be useful in determining disease progression.

Further, the methods of the disclosure may be used to characterize the heterogeneity of an abnormal condition in a subject. Such methods can include, e.g., generating a genetic profile of extracellular polynucleotides derived from the subject, wherein the genetic profile comprises a plurality of data resulting from copy number variation and rare mutation analyses. In some embodiments, an abnormal condition is cancer. In some embodiments, the abnormal condition may be one resulting in a heterogeneous genomic population. In the example of cancer, some tumors are known to comprise tumor cells in different stages of the cancer. In other examples, heterogeneity may comprise multiple foci of disease. Again, in the example of cancer, there may be multiple tumor foci, perhaps where one or more foci are the result of metastases that have spread from a primary site.

The present methods can be used to generate or profile, fingerprint or set of data that is a summation of genetic information derived from different cells in a heterogeneous disease. This set of data may comprise copy number variation, epigenetic variation, and rearrangement or other mutation analyses alone or in combination.

The present methods can be used to diagnose, prognose, monitor or observe cancers, or other diseases. In some embodiments, the methods herein do not involve the diagnosing, prognosing or monitoring a fetus and as such are not directed to non-invasive prenatal testing. In other embodiments, these methodologies may be employed in a pregnant subject to diagnose, prognose, monitor or observe cancers or other diseases in an unborn subject whose DNA and other polynucleotides may co-circulate with maternal molecules.

An exemplary method for identification of rearrangements through NGS comprises the following steps:
1. Preparing an extracted DNA sample (e.g., extracted blood plasma DNA from a human sample) by ligating adapters to the DNA and amplifying one or more target regions using LP-PCR.
2. Contacting the amplified, adapter-ligated DNA with primers that anneal to at least one target region and that completely tile wild type sequences with primers.
3. Contacting the primer-annealed DNA with a 5' to 3' exonuclease negative, strand displacement negative DNA polymerase and deoxynucleoside triphosphates comprising a low percentage of a deoxynucleoside triphosphate linked to a capture moiety, such as biotin, to extend primers bound to target regions comprising a rearrangement.
4. Capturing primer-extended products and the DNA hybridized to them by contacting the DNA with a solid support comprising a binding partner of the capture moiety.
5. Enriching and/or eluting the primer-extended products and/or the DNA hybridized to them.
6. Re-amplifying the captured DNA and assaying in multiplex on an NGS instrument.
7. Bioinformatics analysis of NGS data, with the molecular tags being used to identify unique molecules.

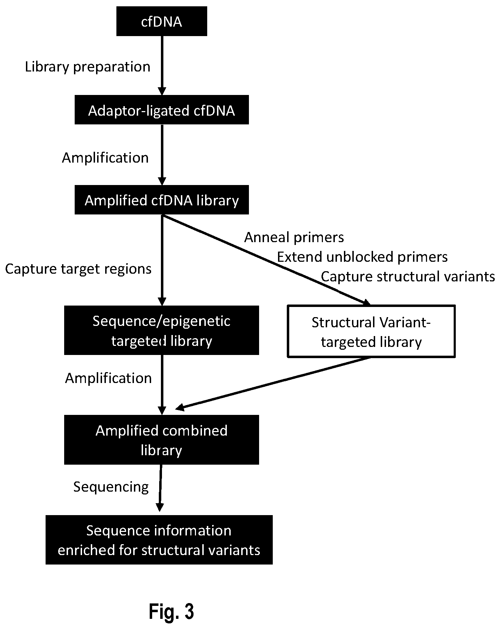

An exemplary workflow is shown in Fig. 3, wherein a cfDNA sample undergoes library preparation including adaptor ligation, then amplification, to provide an amplified cfDNA library. Then sequence-variable and/or epigenetic target regions are captured from one portion of the amplified cfDNA library, providing a sequence/epigenetic targeted library; and primers are annealed to another portion of the amplified cfDNA library, followed by extension of unblocked primers and capture of structural variants, providing a structural variant-targeted library. The structural variant-targeted library and the sequence/epigenetic targeted library are combined; one or both may be amplified before or after combination, resulting in an amplified combined library. Sequencing the amplified combined library provides sequence information which is enriched for structural variants (in addition to information about the sequence-variable and/or epigenetic target regions).

In some embodiments of methods described herein, molecular tags consist of nucleotides that are not altered by a procedure that affects a first nucleobase in the DNA differently from a second nucleobase in the DNA, such as any of those described herein (e.g., mC along with A, T, and G where the procedure is bisulfite conversion or any other conversion that does not affect mC; hmC along with A, T, and G where the procedure is a conversion that does not affect hmC; etc.). In some embodiments of methods described herein, the molecular tags do not comprise nucleotides that are altered by a procedure that affects a first nucleobase in the DNA differently from a second nucleobase in the DNA, such as any of those described herein (e.g., the tags do not comprise unmodified C where the procedure is bisulfite conversion or any other conversion that affects C; the tags do not comprise mC where the procedure is a conversion that affects mC; the tags do not comprise hmC where the procedure is a conversion that affects hmC; etc.).

### III. Additional features of certain disclosed methods

### A. Samples

A sample can be any biological sample isolated from a subject. A sample can be a bodily sample. Samples can include body tissues, such as known or suspected solid tumors, whole blood, platelets, serum, plasma, stool, red blood cells, white blood cells or leucocytes, endothelial cells, tissue biopsies, cerebrospinal fluid synovial fluid, lymphatic fluid, ascites fluid, interstitial or extracellular fluid, the fluid in spaces between cells, including gingival crevicular fluid, bone marrow, pleural effusions, cerebrospinal fluid, saliva, mucous, sputum, semen, sweat, urine. Samples are preferably body fluids, particularly blood and fractions thereof, and urine. A sample can be in the form originally isolated from a subject or can have been subjected to further processing to remove or add components, such as cells, or enrich for one component relative to another. Thus, a preferred body fluid for analysis is plasma or serum containing cell-free nucleic acids.

In some embodiments, a population of nucleic acids is obtained from a serum, plasma or blood sample from a subject suspected of having neoplasia, a tumor, or cancer or previously diagnosed with neoplasia, a tumor, or cancer. The population includes nucleic acids having varying levels of rearrangements, sequence variation, epigenetic variation, and/or post-replication or transcriptional modifications. Post-replication modifications include modifications of cytosine, particularly at the 5-position of the nucleobase, e.g., 5-methylcytosine, 5-hydroxymethylcytosine, 5-formylcytosine and 5-carboxylcytosine.

A sample can be isolated or obtained from a subject and transported to a site of sample analysis. The sample may be preserved and shipped at a desirable temperature, e.g., room temperature, 4°C, -20°C, and/or -80°C. A sample can be isolated or obtained from a subject at the site of the sample analysis. The subject can be a human, a mammal, an animal, a companion animal, a service animal, or a pet. The subject may have a cancer. The subject may not have cancer or a detectable cancer symptom. The subject may have been treated with one or more cancer therapy, e.g., any one or more of chemotherapies, antibodies, vaccines or biologies. The subject may be in remission. The subject may or may not be diagnosed of being susceptible to cancer or any cancer-associated genetic mutations/disorders.

In some embodiments, the sample comprises plasma. The volume of plasma obtanied can depend on the desired read depth for sequenced regions. Exemplary volumes are 0.4-40 ml, 5-20 ml, 10-20 ml. For examples, the volume can be 0.5 mL, 1 mL, 5 mL 10 mL, 20 mL, 30 mL, or 40 mL. A volume of sampled plasma may be 5 to 20 mL.

A sample can comprise various amount of nucleic acid that contains genome equivalents. For example, a sample of about 30 ng DNA can contain about 10,000 (10⁴) haploid human genome equivalents and, in the case of cfDNA, about 200 billion (2x10¹¹) individual polynucleotide molecules. Similarly, a sample of about 100 ng of DNA can contain about 30,000 haploid human genome equivalents and, in the case of cfDNA, about 600 billion individual molecules.

A sample can comprise nucleic acids from different sources, e.g., from cells and cell-free of the same subject, from cells and cell-free of different subjects. A sample can comprise nucleic acids carrying mutations. For example, a sample can comprise DNA carrying germline mutations and/or somatic mutations. Germline mutations refer to mutations existing in germline DNA of a subject. Somatic mutations refer to mutations originating in somatic cells of a subject, e.g., cancer cells. A sample can comprise DNA carrying cancer-associated mutations (e.g., cancer-associated somatic mutations). A sample can comprise an epigenetic variant (i.e. a chemical or protein modification), wherein the epigenetic variant associated with the presence of a genetic variant such as a cancer-associated mutation. In some embodiments, the sample comprises an epigenetic variant associated with the presence of a genetic variant, wherein the sample does not comprise the genetic variant.

Exemplary amounts of cell-free nucleic acids in a sample before amplification range from about 1 fg to about 1 µg, e.g., 1 pg to 200 ng, 1 ng to 100 ng, 10 ng to 1000 ng. For example, the amount can be up to about 600 ng, up to about 500 ng, up to about 400 ng, up to about 300 ng, up to about 200 ng, up to about 100 ng, up to about 50 ng, or up to about 20 ng of cell-free nucleic acid molecules. The amount can be at least 1 fg, at least 10 fg, at least 100 fg, at least 1 pg, at least 10 pg, at least 100 pg, at least 1 ng, at least 10 ng, at least 100 ng, at least 150 ng, or at least 200 ng of cell-free nucleic acid molecules. The amount can be up to 1 femtogram (fg), 10 fg, 100 fg, 1 picogram (pg), 10 pg, 100 pg, 1 ng, 10 ng, 100 ng, 150 ng, or 200 ng of cell-free nucleic acid molecules. The method can comprise obtaining 1 femtogram (fg) to 200 ng-

Cell-free nucleic acids are nucleic acids not contained within or otherwise bound to a cell or in other words nucleic acids remaining in a sample after removing intact cells. Cell- free nucleic acids include DNA, RNA, and hybrids thereof, including genomic DNA, mitochondrial DNA, siRNA, miRNA, circulating RNA (cRNA), tRNA, rRNA, small nucleolar RNA

(snoRNA), Piwi-interacting RNA (piRNA), long non-coding RNA (long ncRNA), or fragments of any of these. Cell-free nucleic acids can be double-stranded, single-stranded, or a hybrid thereof. A cell-free nucleic acid can be released into bodily fluid through secretion or cell death processes, e.g., cellular necrosis and apoptosis. Some cell-free nucleic acids are released into bodily fluid from cancer cells e.g., circulating tumor DNA, (ctDNA). Others are released from healthy cells. In some embodiments, cfDNA is cell-free fetal DNA (cffDNA) In some embodiments, cell free nucleic acids are produced by tumor cells. In some embodiments, cell free nucleic acids are produced by a mixture of tumor cells and non-tumor cells.

Cell-free nucleic acids have an exemplary size distribution of about 100-500 nucleotides, with molecules of 110 to about 230 nucleotides representing about 90% of molecules, with a mode of about 168 nucleotides and a second minor peak in a range between 240 to 440 nucleotides.

Cell-free nucleic acids can be isolated from bodily fluids through a fractionation or partitioning step in which cell-free nucleic acids, as found in solution, are separated from intact cells and other non-soluble components of the bodily fluid. Partitioning may include techniques such as centrifugation or filtration. Alternatively, cells in bodily fluids can be lysed and cell-free and cellular nucleic acids processed together. Generally, after addition of buffers and wash steps, nucleic acids can be precipitated with an alcohol. Further clean up steps may be used such as silica based columns to remove contaminants or salts. Non-specific bulk carrier nucleic acids, such as C 1 DNA, DNA or protein for bisulfite sequencing, hybridization, and/or ligation, may be added throughout the reaction to optimize certain aspects of the procedure such as yield.

After such processing, samples can include various forms of nucleic acid including double stranded DNA, single stranded DNA, and single stranded RNA. In some embodiments, single stranded DNA and RNA can be converted to double stranded forms so they are included in subsequent processing and analysis steps.

DNA molecules can be linked to adapters at either one end or both ends. Typically, double-stranded molecules are blunt ended by treatment with a polymerase with a 5'-3' polymerase and a 3 '-5' exonuclease (or proof-reading function), in the presence of all four standard nucleotides. Klenow large fragment and T4 polymerase are examples of suitable polymerase. The blunt ended DNA molecules can be ligated with at least partially double stranded adapter (e.g., a Y shaped or bell-shaped adapter). Alternatively, complementary nucleotides can be added to blunt ends of sample nucleic acids and adapters to facilitate ligation. Contemplated herein are both blunt end ligation and sticky end ligation. In blunt end ligation, both the nucleic acid molecules and the adapter tags have blunt ends. In sticky-end ligation, typically, the nucleic acid molecules bear an "A" overhang and the adapters bear a "T" overhang.

### B. Amplification

Sample nucleic acids flanked by adapters can be amplified by PCR and other amplification methods. Amplification is typically primed by primers that anneal or bind to primer binding sites in adapters flanking a DNA molecule to be amplified. Amplification methods can involve cycles of denaturation, annealing and extension, resulting from thermocycling or can be isothermal as in transcription-mediated amplification. Other amplification methods include the ligase chain reaction, strand displacement amplification, nucleic acid sequence based amplification, and self-sustained sequence based replication.

In some embodiments, the present methods perform dsDNA ligations with T-tailed and C-tailed adapters, which result in amplification of at least 50, 60, 70 or 80% of double stranded nucleic acids before linking to adapters. Preferably the present methods increase the amount or number of amplified molecules relative to control methods performed with T-tailed adapters alone by at least 10, 15 or 20%.

### C. Capture moieties

As discussed above, nucleic acids in a sample can be subject to a capture step, in which molecules having target regions are captured for subsequent analysis. Target capture can involve use of oligonucleotides labeled with a capture moiety, such as biotin, and a second moiety or binding partner that binds to the capture moiety, such as streptavidin. In some embodiments, a capture moiety and binding partner can have higher and lower capture yields for different sets of target regions, such as those of the sequence-variable target region set and the epigenetic target region set, respectively, as discussed elsewhere herein. Methods comprising capture moieties are further described in, for example, U.S. patent 9,850,523, issuing December 26, 2017.

Capture moieties include, without limitation, biotin, avidin, streptavidin, a nucleic acid comprising a particular nucleotide sequence, a hapten recognized by an antibody, and magnetically attractable particles. The extraction moiety can be a member of a binding pair, such as biotin/streptavidin or hapten/antibody. In some embodiments, a capture moiety that is attached to an analyte is captured by its binding pair which is attached to an isolatable moiety, such as a magnetically attractable particle or a large particle that can be sedimented through centrifugation. The capture moiety can be any type of molecule that allows affinity separation of nucleic acids bearing the capture moiety from nucleic acids lacking the capture moiety. Exemplary capture moieties are biotin which allows affinity separation by binding to streptavidin linked or linkable to a solid phase or an oligonucleotide, which allows affinity separation through binding to a complementary oligonucleotide linked or linkable to a solid phase.

### D. Collections of target-specific probes

In some embodiments, a collection of target-specific probes is used in a method comprising a sequence-variable target set and/or epigenetic target set, as described herein. In some embodiments, the collection of target-specific probes comprises target-binding probes specific for a sequence-variable target region set and target-binding probes specific for an epigenetic target region set. In some embodiments, the capture yield of the target-binding probes specific for the sequence-variable target region set is higher (e.g., at least 2-fold higher) than the capture yield of the target-binding probes specific for the epigenetic target region set. In some embodiments, the collection of target-specific probes is configured to have a capture yield specific for the sequence-variable target region set higher (e.g., at least 2-fold higher) than its capture yield specific for the epigenetic target region set.

In some embodiments, the capture yield of the target-binding probes specific for the sequence-variable target region set is at least 1.25-, 1.5-, 1.75-, 2-, 2.25-, 2.5-, 2.75-, 3-, 3.5-, 4-, 4.5-, 5-, 6-, 7-, 8-, 9-, 10-, 11-, 12-, 13-, 14-, or 15-fold higher than the capture yield of the target-binding probes specific for the epigenetic target region set. In some embodiments, the capture yield of the target-binding probes specific for the sequence-variable target region set is 1.25- to 1.5-, 1.5- to 1.75-, 1.75- to 2-, 2- to 2.25-, 2.25- to 2.5-, 2.5- to 2.75-, 2.75- to 3-, 3- to 3.5-, 3.5- to 4-, 4- to 4.5-, 4.5- to 5-, 5- to 5.5-, 5.5- to 6-, 6- to 7-, 7- to 8-, 8- to 9-, 9- to 10-, 10-to 11-, 11- to 12-, 13- to 14-, or 14- to 15-fold higher than the capture yield of the target-binding probes specific for the epigenetic target region set.

In some embodiments, the collection of target-specific probes is configured to have a capture yield specific for the sequence-variable target region set at least 1.25-, 1.5-, 1.75-, 2-, 2.25-, 2.5-, 2.75-, 3-, 3.5-, 4-, 4.5-, 5-, 6-, 7-, 8-, 9-, 10-, 11-, 12-, 13-, 14-, or 15-fold higher than its capture yield for the epigenetic target region set. In some embodiments, the collection of target-specific probes is configured to have a capture yield specific for the sequence-variable target region set is 1.25- to 1.5-, 1.5- to 1.75-, 1.75- to 2-, 2- to 2.25-, 2.25- to 2.5-, 2.5- to 2.75-, 2.75- to 3-, 3- to 3.5-, 3.5- to 4-, 4- to 4.5-, 4.5- to 5-, 5- to 5.5-, 5.5- to 6-, 6- to 7-, 7- to 8-, 8- to 9-, 9- to 10-, 10- to 11-, 11- to 12-, 13- to 14-, or 14- to 15-fold higher than its capture yield specific for the epigenetic target region set.

The collection of probes can be configured to provide higher capture yields for the sequence-variable target region set in various ways, including concentration, different lengths and/or chemistries (e.g., that affect affinity), and combinations thereof. Affinity can be modulated by adjusting probe length and/or including nucleotide modifications as discussed below.

In some embodiments, the target-specific probes specific for the sequence-variable target region set are present at a higher concentration than the target-specific probes specific for the epigenetic target region set. In some embodiments, concentration of the target-binding probes specific for the sequence-variable target region set is at least 1.25-, 1.5-, 1.75-, 2-, 2.25-, 2.5-, 2.75-, 3-, 3.5-, 4-, 4.5-, 5-, 6-, 7-, 8-, 9-, 10-, 11-, 12-, 13-, 14-, or 15-fold higher than the concentration of the target-binding probes specific for the epigenetic target region set. In some embodiments, the concentration of the target-binding probes specific for the sequence-variable target region set is 1.25- to 1.5-, 1.5- to 1.75-, 1.75- to 2-, 2- to 2.25-, 2.25- to 2.5-, 2.5- to 2.75-, 2.75- to 3-, 3- to 3.5-, 3.5- to 4-, 4- to 4.5-, 4.5- to 5-, 5- to 5.5-, 5.5- to 6-, 6- to 7-, 7- to 8-, 8- to 9-, 9- to 10-, 10- to 11-, 11- to 12-, 13- to 14-, or 14- to 15-fold higher than the concentration of the target-binding probes specific for the epigenetic target region set. In such embodiments, concentration may refer to the average mass per volume concentration of individual probes in each set.

In some embodiments, the target-specific probes specific for the sequence-variable target region set have a higher affinity for their targets than the target-specific probes specific for the epigenetic target region set. Affinity can be modulated in any way known to those skilled in the art, including by using different probe chemistries. For example, certain nucleotide modifications, such as cytosine 5-methylation (in certain sequence contexts), modifications that provide a heteroatom at the 2' sugar position, and LNA nucleotides, can increase stability of double-stranded nucleic acids, indicating that oligonucleotides with such modifications have relatively higher affinity for their complementary sequences. See, e.g., Severin et al., Nucleic Acids Res. 39: 8740-8751 (2011); Freier et al., Nucleic Acids Res. 25: 4429-4443 (1997); US Patent No. 9,738,894. Also, longer sequence lengths will generally provide increased affinity. Other nucleotide modifications, such as the substitution of the nucleobase hypoxanthine for guanine, reduce affinity by reducing the amount of hydrogen bonding between the oligonucleotide and its complementary sequence. In some embodiments, the target-specific probes specific for the sequence-variable target region set have modifications that increase their affinity for their targets. In some embodiments, alternatively or additionally, the target-specific probes specific for the epigenetic target region set have modifications that decrease their affinity for their targets. In some embodiments, the target-specific probes specific for the sequence-variable target region set have longer average lengths and/or higher average melting temperatures than the target-specific probes specific for the epigenetic target region set. These embodiments may be combined with each other and/or with differences in concentration as discussed above to achieve a desired fold difference in capture yield, such as any fold difference or range thereof described above.

In some embodiments, the target-specific probes comprise a capture moiety. The capture moiety may be any of the capture moieties described herein, e.g., biotin. In some embodiments, the target-specific probes are linked to a solid support, e.g., covalently or non-covalently such as through the interaction of a binding pair of capture moieties. In some embodiments, the solid support is a bead, such as a magnetic bead.

In some embodiments, the target-specific probes specific for the sequence-variable target region set and/or the target-specific probes specific for the epigenetic target region set comprise a capture moiety as discussed above, e.g., probes comprising capture moieties and sequences selected to tile across a panel of regions, such as genes.

In some embodiments, the target-specific probes are provided in a single composition. The single composition may be a solution (liquid or frozen). Alternatively, it may be a lyophilizate.

Alternatively, the target-specific probes may be provided as a plurality of compositions, e.g., comprising a first composition comprising probes specific for the epigenetic target region set and a second composition comprising probes specific for the sequence-variable target region set. These probes may be mixed in appropriate proportions to provide a combined probe composition with any of the foregoing fold differences in concentration and/or capture yield. Alternatively, they may be used in separate capture procedures (e.g., with aliquots of a sample or sequentially with the same sample) to provide first and second compositions comprising captured epigenetic target regions and sequence-variable target regions, respectively.

### 1. Probes specific for epigenetic target regions

The probes for the epigenetic target region set may comprise probes specific for one or more types of target regions likely to differentiate DNA from neoplastic (e.g., tumor or cancer) cells from healthy cells, e.g., non-neoplastic circulating cells. Exemplary types of such regions are discussed in detail herein, e.g., in the sections above concerning captured sets. The probes for the epigenetic target region set may also comprise probes for one or more control regions, e.g., as described herein.

In some embodiments, the probes for the epigenetic target region set have a footprint of at least 100 kbp, e.g., at least 200 kbp, at least 300 kbp, or at least 400 kbp. In some embodiments, the epigenetic target region set has a footprint in the range of 100-20 Mbp, e.g., 100-200 kbp, 200-300 kbp, 300-400 kbp, 400-500 kbp, 500-600 kbp, 600-700 kbp, 700-800 kbp, 800-900 kbp, 900-1,000 kbp, 1-1.5 Mbp, 1.5-2 Mbp, 2-3 Mbp, 3-4 Mbp, 4-5 Mbp, 5-6 Mbp, 6-7 Mbp, 7-8 Mbp, 8-9 Mbp, 9-10 Mbp, or 10-20 Mbp. In some embodiments, the epigenetic target region set has a footprint of at least 20 Mbp.

### a. Hypermethylation variable target regions

In some embodiments, the probes for the epigenetic target region set comprise probes specific for one or more hypermethylation variable target regions. The hypermethylation variable target regions may be any of those set forth above. For example, in some embodiments, the probes specific for hypermethylation variable target regions comprise probes specific for a plurality of loci listed in Table 1, e.g., at least 10%, 20%, 30%, 40%, 50%, 60%, 70%, 80%, 90%, or 100% of the loci listed in Table 1. In some embodiments, the probes specific for hypermethylation variable target regions comprise probes specific for a plurality of loci listed in Table 2, e.g., at least 10%, 20%, 30%, 40%, 50%, 60%, 70%, 80%, 90%, or 100% of the loci listed in Table 2. In some embodiments, the probes specific for hypermethylation variable target regions comprise probes specific for a plurality of loci listed in Table 1 or Table 2, e.g., at least 10%, 20%, 30%, 40%, 50%, 60%, 70%, 80%, 90%, or 100% of the loci listed in Table 1 or Table 2. In some embodiments, for each locus included as a target region, there may be one or more probes with a hybridization site that binds between the transcription start site and the stop codon (the last stop codon for genes that are alternatively spliced) of the gene. In some embodiments, the one or more probes bind within 300 bp of the listed position, e.g., within 200 or 100 bp. In some embodiments, a probe has a hybridization site overlapping the position listed above. In some embodiments, the probes specific for the hypermethylation target regions include probes specific for one, two, three, four, or five subsets of hypermethylation target regions that collectively show hypermethylation in one, two, three, four, or five of breast, colon, kidney, liver, and lung cancers.

### b. Hypomethylation variable target regions

In some embodiments, the probes for the epigenetic target region set comprise probes specific for one or more hypomethylation variable target regions. The hypomethylation variable target regions may be any of those set forth above. For example, the probes specific for one or more hypomethylation variable target regions may include probes for regions such as repeated elements, e.g., LINE1 elements, Alu elements, centromeric tandem repeats, pericentromeric tandem repeats, and satellite DNA, and intergenic regions that are ordinarily methylated in healthy cells may show reduced methylation in tumor cells.

In some embodiments, probes specific for hypomethylation variable target regions include probes specific for repeated elements and/or intergenic regions. In some embodiments, probes specific for repeated elements include probes specific for one, two, three, four, or five of LINE1 elements, Alu elements, centromeric tandem repeats, pericentromeric tandem repeats, and/or satellite DNA.

Exemplary probes specific for genomic regions that show cancer-associated hypomethylation include probes specific for nucleotides 8403565-8953708 and/or 151104701-151106035 of human chromosome 1. In some embodiments, the probes specific for hypomethylation variable target regions include probes specific for regions overlapping or comprising nucleotides 8403565-8953708 and/or 151104701-151106035 of human chromosome 1.

### c. CTCF binding regions

In some embodiments, the probes for the epigenetic target region set include probes specific for CTCF binding regions. In some embodiments, the probes specific for CTCF binding regions comprise probes specific for at least 10, 20, 50, 100, 200, or 500 CTCF binding regions, or 10-20, 20-50, 50-100, 100-200, 200-500, or 500-1000 CTCF binding regions, e.g., such as CTCF binding regions described above or in one or more of CTCFBSDB or the Cuddapah et al., Martin et al., or Rhee et al. articles cited above. In some embodiments, the probes for the epigenetic target region set comprise at least 100 bp, at least 200 bp at least 300 bp, at least 400 bp, at least 500 bp, at least 750 bp, or at least 1000 bp upstream and downstream regions of the CTCF binding sites.

### d. Transcription start sites

In some embodiments, the probes for the epigenetic target region set include probes specific for transcriptional start sites. In some embodiments, the probes specific for transcriptional start sites comprise probes specific for at least 10, 20, 50, 100, 200, or 500 transcriptional start sites, or 10-20, 20-50, 50-100, 100-200, 200-500, or 500-1000 transcriptional start sites, e.g., such as transcriptional start sites listed in DBTSS. In some embodiments, the probes for the epigenetic target region set comprise probes for sequences at least 100 bp, at least 200 bp, at least 300 bp, at least 400 bp, at least 500 bp, at least 750 bp, or at least 1000 bp upstream and downstream of the transcriptional start sites.

### e. Focal amplifications

As noted above, although focal amplifications are somatic mutations, they can be detected by sequencing based on read frequency in a manner analogous to approaches for detecting certain epigenetic changes such as changes in methylation. As such, regions that may show focal amplifications in cancer can be included in the epigenetic target region set, as discussed above. In some embodiments, the probes specific for the epigenetic target region set include probes specific for focal amplifications. In some embodiments, the probes specific for focal amplifications include probes specific for one or more of AR, BRAF, CCND1, CCND2, CCNE1, CDK4, CDK6, EGFR, ERBB2, FGFR1, FGFR2, KIT, KRAS, MET, MYC, PDGFRA, PIK3CA, and RAF1. For example, in some embodiments, the probes specific for focal amplifications include probes specific for one or more of at least 2, 3, 4, 5, 6, 7, 8, 9, 10, 11, 12, 13, 14, 15, 16, 17, or 18 of the foregoing targets.

### f. Control regions

It can be useful to include control regions to facilitate data validation. In some embodiments, the probes specific for the epigenetic target region set include probes specific for control methylated regions that are expected to be methylated in essentially all samples. In some embodiments, the probes specific for the epigenetic target region set include probes specific for control hypomethylated regions that are expected to be hypomethylated in essentially all samples.

### 2. Probes specific for sequence-variable target regions

The probes for the sequence-variable target region set may comprise probes specific for a plurality of regions known to undergo somatic mutations in cancer. The probes may be specific for any sequence-variable target region set described herein. Exemplary sequence-variable target region sets are discussed in detail herein, e.g., in the sections above concerning captured sets.

In some embodiments, the sequence-variable target region probe set has a footprint of at least 0.5 kb, e.g., at least 1 kb, at least 2 kb, at least 5 kb, at least 10 kb, at least 20 kb, at least 30 kb, or at least 40 kb. In some embodiments, the epigenetic target region probe set has a footprint in the range of 0.5-100 kb, e.g., 0.5-2 kb, 2-10 kb, 10-20 kb, 20-30 kb, 30-40 kb, 40-50 kb, 50-60 kb, 60-70 kb, 70-80 kb, 80-90 kb, and 90-100 kb. In some embodiments, the sequence-variable target region probe set has a footprint of at least 50 kbp, e.g., at least 100 kbp, at least 200 kbp, at least 300 kbp, or at least 400 kbp. In some embodiments, the sequence-variable target region probe set has a footprint in the range of 100-2000 kbp, e.g., 100-200 kbp, 200-300 kbp, 300-400 kbp, 400-500 kbp, 500-600 kbp, 600-700 kbp, 700-800 kbp, 800-900 kbp, 900-1,000 kbp, 1-1.5 Mbp or 1.5-2 Mbp. In some embodiments, the sequence-variable target region set has a footprint of at least 2 Mbp.

In some embodiments, probes specific for the sequence-variable target region set comprise probes specific for at least a portion of at least 5, at least 10, at least 15, at least 20, at least 25, at least 30, at least 35, at least 40, at least 45, at least 50, at least 55, at least 60, at least 65, or at 70 of the genes of Table 3. In some embodiments, probes specific for the sequence-variable target region set comprise probes specific for the at least 5, at least 10, at least 15, at least 20, at least 25, at least 30, at least 35, at least 40, at least 45, at least 50, at least 55, at least 60, at least 65, or 70 of the SNVs of Table 3. In some embodiments, probes specific for the sequence-variable target region set comprise probes specific for at least 1, at least 2, at least 3, at least 4, at least 5, or 6 of the fusions of Table 3. In some embodiments, probes specific for the sequence-variable target region set comprise probes specific for at least a portion of at least 1, at least 2, or 3 of the indels of Table 3. In some embodiments, probes specific for the sequence-variable target region set comprise probes specific for at least a portion of at least 5, at least 10, at least 15, at least 20, at least 25, at least 30, at least 35, at least 40, at least 45, at least 50, at least 55, at least 60, at least 65, at least 70, or 73 of the genes of Table 4. In some embodiments, probes specific for the sequence-variable target region set comprise probes specific for at least 5, at least 10, at least 15, at least 20, at least 25, at least 30, at least 35, at least 40, at least 45, at least 50, at least 55, at least 60, at least 65, at least 70, or 73 of the SNVs of Table 4. In some embodiments, probes specific for the sequence-variable target region set comprise probes specific for at least 1, at least 2, at least 3, at least 4, at least 5, or 6 of the fusions of Table 4. In some embodiments, probes specific for the sequence-variable target region set comprise probes specific for at least a portion of at least 1, at least 2, at least 3, at least 4, at least 5, at least 6, at least 7, at least 8, at least 9, at least 10, at least 11, at least 12, at least 13, at least 14, at least 15, at least 16, at least 17, or 18 of the indels of Table 4. In some embodiments, probes specific for the sequence-variable target region set comprise probes specific for at least a portion of at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, or at least 20 of the genes of Table 5.

In some embodiments, the probes specific for the sequence-variable target region set comprise probes specific for target regions from at least 10, 20, 30, or 35 cancer-related genes, such as AKT1, ALK, BRAF, CCND1, CDK2A, CTNNB1, EGFR, ERBB2, ESR1, FGFR1, FGFR2, FGFR3, FOXL2, GATA3, GNA11, GNAQ, GNAS, HRAS, IDH1, IDH2, KIT, KRAS, MED12, MET, MYC, NFE2L2, NRAS, PDGFRA, PIK3CA, PPP2R1A, PTEN, RET, STK11, TP53, and U2AF1.

### E. Computer Systems

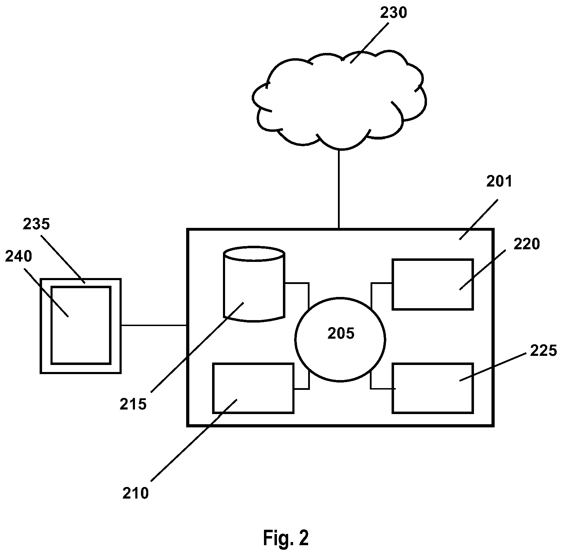

Methods of the present disclosure can be implemented using, or with the aid of, computer systems. FIG. 2 shows a computer system 201 that is programmed or otherwise configured to implement the methods of the present disclosure. The computer system 201 can regulate various aspects sample preparation, sequencing, and/or analysis. In some examples, the computer system 201 is configured to perform sample preparation and sample analysis, including nucleic acid sequencing, e.g., according to any of the methods disclosed herein.

The computer system 201 includes a central processing unit (CPU, also "processor" and "computer processor" herein) 205, which can be a single core or multi core processor, or a plurality of processors for parallel processing. The computer system 201 also includes memory or memory location 210 (e.g., random-access memory, read-only memory, flash memory), electronic storage unit 215 (e.g., hard disk), communication interface 220 (e.g., network adapter) for communicating with one or more other systems, and peripheral devices 225, such as cache, other memory, data storage, and/or electronic display adapters. The memory 210, storage unit 215, interface 220, and peripheral devices 225 are in communication with the CPU 205 through a communication network or bus (solid lines), such as a motherboard. The storage unit 215 can be a data storage unit (or data repository) for storing data. The computer system 201 can be operatively coupled to a computer network 230 with the aid of the communication interface 220. The computer network 230 can be the Internet, an internet and/or extranet, or an intranet and/or extranet that is in communication with the Internet. The computer network 230 in some cases is a telecommunication and/or data network. The computer network 230 can include one or more computer servers, which can enable distributed computing, such as cloud computing. The computer network 230, in some cases with the aid of the computer system 0, can implement a peer-to-peer network, which may enable devices coupled to the computer system 201 to behave as a client or a server.

The CPU 205 can execute a sequence of machine-readable instructions, which can be embodied in a program or software. The instructions may be stored in a memory location, such as the memory 210. Examples of operations performed by the CPU 205 can include fetch, decode, execute, and writeback.

The storage unit 215 can store files, such as drivers, libraries, and saved programs. The storage unit 215 can store programs generated by users and recorded sessions, as well as output(s) associated with the programs. The storage unit 215 can store user data, e.g., user preferences and user programs. The computer system 201 in some cases can include one or more additional data storage units that are external to the computer system 201, such as located on a remote server that is in communication with the computer system 201 through an intranet or the Internet. Data may be transferred from one location to another using, for example, a communication network or physical data transfer (e.g., using a hard drive, thumb drive, or other data storage mechanism).

The computer system 201 can communicate with one or more remote computer systems through the network 230. For embodiment, the computer system 201 can communicate with a remote computer system of a user (e.g., operator). Examples of remote computer systems include personal computers (e.g., portable PC), slate or tablet PC's (e.g., Apple^{®} iPad, Samsung^{®} Galaxy Tab), telephones, Smart phones (e.g., Apple^{®} iPhone, Android-enabled device, Blackberry^{®}), or personal digital assistants. The user can access the computer system 201 via the network 230.

Methods as described herein can be implemented by way of machine (e.g., computer processor) executable code stored on an electronic storage location of the computer system 201, such as, for example, on the memory 210 or electronic storage unit 215. The machine executable or machine-readable code can be provided in the form of software. During use, the code can be executed by the processor 205. In some cases, the code can be retrieved from the storage unit 215 and stored on the memory 210 for ready access by the processor 205. In some situations, the electronic storage unit 215 can be precluded, and machine-executable instructions are stored on memory 210.

In an aspect, the present disclosure provides a non-transitory computer-readable medium comprising computer-executable instructions which, when executed by at least one electronic processor, perform at least a portion of a method comprising: collecting cfDNA from a test subject; capturing a plurality of sets of target regions from the cfDNA, wherein the plurality of target region sets comprises an intronic target region, exonic VDJ target region, sequence-variable target region set, and/or an epigenetic target region set, whereby a captured set of cfDNA molecules is produced; sequencing the captured cfDNA molecules, wherein the captured cfDNA molecules of the intronic, exonic VDJ, and sequence-variable target region sets are sequenced to a greater depth of sequencing than the captured cfDNA molecules of the epigenetic target region set; obtaining a plurality of sequence reads generated by a nucleic acid sequencer from sequencing the captured cfDNA molecules; mapping the plurality of sequence reads to one or more reference sequences to generate mapped sequence reads; and processing the mapped sequence reads corresponding to the sequence-variable target region set and to the epigenetic target region set to determine the likelihood that the subject has cancer.

The code can be pre-compiled and configured for use with a machine have a processer adapted to execute the code or can be compiled during runtime. The code can be supplied in a programming language that can be selected to enable the code to execute in a pre-compiled or as-compiled fashion.

Aspects of the systems and methods provided herein, such as the computer system 201, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming.

All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical, and electromagnetic waves, such as those used across physical interfaces between local devices, through wired and optical landline networks, and over various air-links. The physical elements that carry such waves, such as wired or wireless links, optical links, or the like, also may be considered as media bearing the software. As used herein, unless restricted to non-transitory, tangible "storage" media, terms such as computer or machine "readable medium" refer to any medium that participates in providing instructions to a processor for execution.

Hence, a machine-readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards, paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 201 can include or be in communication with an electronic display that comprises a user interface (UI) for providing, for example, one or more results of sample analysis. Examples of UIs include, without limitation, a graphical user interface (GUI) and web-based user interface.

Additional details relating to computer systems and networks, databases, and computer program products are also provided in, for example, Peterson, Computer Networks: A Systems Approach, Morgan Kaufmann, 5th Ed. (2011), Kurose, Computer Networking: A Top-Down Approach, Pearson, 7th Ed. (2016), Elmasri, Fundamentals of Database Systems, Addison Wesley, 6th Ed. (2010), Coronel, Database Systems: Design, Implementation, & Management, Cengage Learning, 11th Ed. (2014), Tucker, Programming Languages, McGraw-Hill Science/Engineering/Math, 2nd Ed. (2006), and Rhoton, Cloud Computing Architected: Solution Design Handbook, Recursive Press (2011).

### F. Applications

### 1. Structural Variation Detection, Cancer, and Other Diseases

The present methods can be used to detect structural variations, including rearrangements, such as any of the types of rearrangements described elsewhere herein. In some embodiments, the methods can be used to detect structural variations and other types of mutations, such as single nucleotide variations. For example, detection of a structural variation (e.g., rearrangement) can be based on observation of a sequence read, a unique sequence, or a plurality of sequence reads or unique sequences that comprise a rearranged sequence. More generally, the sequence information may comprise sequence reads of the nucleic acids generated by a nucleic acid sequencer. In some embodiments, the nucleic acid sequencer performs pyrosequencing, single-molecule sequencing, nanopore sequencing, semiconductor sequencing, sequencing-by-synthesis, sequencing-by-ligation or sequencing-by-hybridization on the nucleic acids to generate sequencing reads. In some embodiments, the method further comprises grouping the sequence reads into families of sequence reads, each family comprising sequence reads generated from a nucleic acid in the sample.

The present methods can be used to diagnose presence of conditions, particularly cancer, in a subject, to characterize conditions (e.g., staging cancer or determining heterogeneity of a cancer), monitor response to treatment of a condition, effect prognosis risk of developing a condition or subsequent course of a condition. The present disclosure can also be useful in determining the efficacy of a particular treatment option. Successful treatment options may increase the amount of copy number variation or rare mutations detected in subject's blood if the treatment is successful as more cancers may die and shed DNA. In other examples, this may not occur. In another example, perhaps certain treatment options may be correlated with genetic profiles of cancers over time. This correlation may be useful in selecting a therapy.

In some embodiments, the present methods are used for screening for a cancer, or in a method for screening cancer. For example, the sample can be a sample from a subject who has not been previously diagnosed with cancer. In some embodiments, the subject may or may not have cancer. In some embodiments, the subject may or may not have an early-stage cancer. In some embodiments, the subject has one or more risk factors for cancer, such as tobacco use (e.g., smoking), being overweight or obese, having a high body mass index (BMI), being of advanced age, poor nutrition, high alcohol consumption, or a family history of cancer.

In some embodiments, the subject has used tobacco, e.g., for at least 1, 5, 10, or 15 years. In some embodiments, the subject has a high BMI, e.g., a BMI of 25 or greater, 26 or greater, 27 or greater, 28 or greater, 29 or greater, or 30 or greater. In some embodiments, the subject is at least 40, 45, 50, 55, 60, 65, 70, 75, or 80 years old. In some embodiments, the subject has poor nutrition, e.g., high consumption of one or more of red meat and/or processed meat, trans fat, saturated fat, and refined sugars, and/or low consumption of fruits and vegetables, complex carbohydrates, and/or unsaturated fats. High and low consumption can be defined, e.g., as exceeding or falling below, respectively, recommendations in Dietary Guidelines for Americans 2020-2025, available at www.dietaryguidelines.gov/sites/default/files/2021-03/Dietary_Guidelines_for_Americans-2020-2025.pdf . In some embodiments, the subject has high alcohol consumption, e.g., at least three, four, or five drinks per day on average (where a drink is about one ounce or 30 mL of 80-proof hard liquor or the equivalent). In some embodiments, the subject has a family history of cancer, e.g., at least one, two, or three blood relatives were previously diagnosed with cancer. In some embodiments, the relatives are at least third-degree relatives (e.g., great-grandparent, great aunt or uncle, first cousin), at least second-degree relatives (e.g., grandparent, aunt or uncle, or half-sibling), or first-degree relatives (e.g., parent or full sibling).

Additionally, if a cancer is observed to be in remission after treatment, the present methods can be used to monitor residual disease or recurrence of disease.

In some embodiments, the methods and systems disclosed herein may be used to identify customized or targeted therapies to treat a given disease or condition in patients based on the classification of a nucleic acid variant as being of somatic or germline origin. Typically, the disease under consideration is a type of cancer. Non-limiting examples of such cancers include biliary tract cancer, bladder cancer, transitional cell carcinoma, urothelial carcinoma, brain cancer, gliomas, astrocytomas, breast carcinoma, metaplastic carcinoma, cervical cancer, cervical squamous cell carcinoma, rectal cancer, colorectal carcinoma, colon cancer, hereditary nonpolyposis colorectal cancer, colorectal adenocarcinomas, gastrointestinal stromal tumors (GISTs), endometrial carcinoma, endometrial stromal sarcomas, esophageal cancer, esophageal squamous cell carcinoma, esophageal adenocarcinoma, ocular melanoma, uveal melanoma, gallbladder carcinomas, gallbladder adenocarcinoma, renal cell carcinoma, clear cell renal cell carcinoma, transitional cell carcinoma, urothelial carcinomas, Wilms tumor, leukemia, acute lymphocytic leukemia (ALL), acute myeloid leukemia (AML), chronic lymphocytic leukemia (CLL), chronic myeloid leukemia (CML), chronic myelomonocytic leukemia (CMML), liver cancer, liver carcinoma, hepatoma, hepatocellular carcinoma, cholangiocarcinoma, hepatoblastoma, Lung cancer, non-small cell lung cancer (NSCLC), mesothelioma, B-cell lymphomas, non-Hodgkin lymphoma, diffuse large B-cell lymphoma, Mantle cell lymphoma, T cell lymphomas, non-Hodgkin lymphoma, precursor T-lymphoblastic lymphoma/leukemia, peripheral T cell lymphomas, multiple myeloma, nasopharyngeal carcinoma (NPC), neuroblastoma, oropharyngeal cancer, oral cavity squamous cell carcinomas, osteosarcoma, ovarian carcinoma, pancreatic cancer, pancreatic ductal adenocarcinoma, pseudopapillary neoplasms, acinar cell carcinomas. Prostate cancer, prostate adenocarcinoma, skin cancer, melanoma, malignant melanoma, cutaneous melanoma, small intestine carcinomas, stomach cancer, gastric carcinoma, gastrointestinal stromal tumor (GIST), uterine cancer, or uterine sarcoma. Type and/or stage of cancer can be detected from genetic variations including mutations, rare mutations, indels, rearrangements, copy number variations, transversions, translocations, recombinations, inversion, deletions, aneuploidy, partial aneuploidy, polyploidy, chromosomal instability, chromosomal structure alterations, gene fusions, chromosome fusions, gene truncations, gene amplification, gene duplications, chromosomal lesions, DNA lesions, abnormal changes in nucleic acid chemical modifications, abnormal changes in epigenetic patterns, and abnormal changes in nucleic acid 5-methylcytosine.

Genetic data can also be used for characterizing a specific form of cancer. Cancers are often heterogeneous in both composition and staging. Genetic profile data may allow characterization of specific sub-types of cancer that may be important in the diagnosis or treatment of that specific sub-type. This information may also provide a subject or practitioner clues regarding the prognosis of a specific type of cancer and allow either a subject or practitioner to adapt treatment options in accord with the progress of the disease. Some cancers can progress to become more aggressive and genetically unstable. Other cancers may remain benign, inactive or dormant. The system and methods of this disclosure may be useful in determining disease progression.

Further, the methods of the disclosure may be used to characterize the heterogeneity of an abnormal condition in a subject. Such methods can include, e.g., generating a genetic profile of extracellular polynucleotides derived from the subject, wherein the genetic profile comprises a plurality of data resulting from copy number variation and rare mutation analyses. In some embodiments, an abnormal condition is cancer. In some embodiments, the abnormal condition may be one resulting in a heterogeneous genomic population. In the example of cancer, some tumors are known to comprise tumor cells in different stages of the cancer. In other examples, heterogeneity may comprise multiple foci of disease. Again, in the example of cancer, there may be multiple tumor foci, perhaps where one or more foci are the result of metastases that have spread from a primary site.

The present methods can be used to generate or profile, fingerprint or set of data that is a summation of genetic information derived from different cells in a heterogeneous disease. This set of data may comprise copy number variation, epigenetic variation, and mutation analyses alone or in combination.

The present methods can be used to diagnose, prognose, monitor or observe cancers, or other diseases. In some embodiments, the methods herein do not involve the diagnosing, prognosing or monitoring a fetus and as such are not directed to non-invasive prenatal testing. In other embodiments, these methodologies may be employed in a pregnant subject to diagnose, prognose, monitor or observe cancers or other diseases in an unborn subject whose DNA and other polynucleotides may co-circulate with maternal molecules.

Non-limiting examples of other genetic-based diseases, disorders, or conditions that are optionally evaluated using the methods and systems disclosed herein include achondroplasia, alpha-1 antitrypsin deficiency, antiphospholipid syndrome, autism, autosomal dominant polycystic kidney disease, Charcot-Marie-Tooth (CMT), cri du chat, Crohn's disease, cystic fibrosis, Dercum disease, down syndrome, Duane syndrome, Duchenne muscular dystrophy, Factor V Leiden thrombophilia, familial hypercholesterolemia, familial Mediterranean fever, fragile X syndrome, Gaucher disease, hemochromatosis, hemophilia, holoprosencephaly, Huntington's disease, Klinefelter syndrome, Marfan syndrome, myotonic dystrophy, neurofibromatosis, Noonan syndrome, osteogenesis imperfecta, Parkinson's disease, phenylketonuria, Poland anomaly, porphyria, progeria, retinitis pigmentosa, severe combined immunodeficiency (SCID), sickle cell disease, spinal muscular atrophy, Tay-Sachs, thalassemia, trimethylaminuria, Turner syndrome, velocardiofacial syndrome, WAGR syndrome, Wilson disease, or the like.

In some embodiments, a method described herein comprises detecting a presence or absence of DNA originating or derived from a tumor cell at a preselected timepoint following a previous cancer treatment of a subject previously diagnosed with cancer using a set of sequence information obtained as described herein. The method may further comprise determining a cancer recurrence score that is indicative of the presence or absence of the DNA originating or derived from the tumor cell for the test subject.

Where a cancer recurrence score is determined, it may further be used to determine a cancer recurrence status. The cancer recurrence status may be at risk for cancer recurrence, e.g., when the cancer recurrence score is above a predetermined threshold. The cancer recurrence status may be at low or lower risk for cancer recurrence, e.g., when the cancer recurrence score is above a predetermined threshold. In particular embodiments, a cancer recurrence score equal to the predetermined threshold may result in a cancer recurrence status of either at risk for cancer recurrence or at low or lower risk for cancer recurrence.

In some embodiments, a cancer recurrence score is compared with a predetermined cancer recurrence threshold, and the test subject is classified as a candidate for a subsequent cancer treatment when the cancer recurrence score is above the cancer recurrence threshold or not a candidate for therapy when the cancer recurrence score is below the cancer recurrence threshold. In particular embodiments, a cancer recurrence score equal to the cancer recurrence threshold may result in classification as either a candidate for a subsequent cancer treatment or not a candidate for therapy.

The methods discussed above may further comprise any compatible feature or features set forth elsewhere herein, including in the section regarding methods of determining a risk of cancer recurrence in a test subject and/or classifying a test subject as being a candidate for a subsequent cancer treatment.

### 2. Methods of determining a risk of cancer recurrence in a test subject and/or classifying a test subject as being a candidate for a subsequent cancer treatment

In some embodiments, a method provided herein is a method of determining a risk of cancer recurrence in a test subject. In some embodiments, a method provided herein is a method of classifying a test subject as being a candidate for a subsequent cancer treatment.

Any of such methods may comprise collecting DNA (e.g., originating or derived from a tumor cell) from the test subject diagnosed with the cancer at one or more preselected timepoints following one or more previous cancer treatments to the test subject. The subject may be any of the subjects described herein. The DNA may be cfDNA. The DNA may be obtained from a tissue sample.

Any of such methods may comprise capturing a plurality of sets of target regions from DNA from the subject, wherein the plurality of target region sets comprise a rearrangement, a sequence-variable target region set, and/or an epigenetic target region set, whereby a captured set of DNA molecules is produced. The capturing step may be performed according to any of the embodiments described elsewhere herein.

In any of such methods, the previous cancer treatment may comprise surgery, administration of a therapeutic composition, and/or chemotherapy.

Any of such methods may comprise sequencing the captured DNA molecules, whereby a set of sequence information is produced. The captured DNA molecules of the intronic or exonic target regions and sequence-variable target region set may be sequenced to a greater depth of sequencing than the captured DNA molecules of the epigenetic target region set.

Any of such methods may comprise detecting a presence or absence of DNA originating or derived from a tumor cell at a preselected timepoint using the set of sequence information. The detection of the presence or absence of DNA originating or derived from a tumor cell may be performed according to any of the embodiments thereof described elsewhere herein.

Methods of determining a risk of cancer recurrence in a test subject may comprise determining a cancer recurrence score that is indicative of the presence or absence, or amount, of the DNA originating or derived from the tumor cell for the test subject. The cancer recurrence score may further be used to determine a cancer recurrence status. The cancer recurrence status may be at risk for cancer recurrence, e.g., when the cancer recurrence score is above a predetermined threshold. The cancer recurrence status may be at low or lower risk for cancer recurrence, e.g., when the cancer recurrence score is above a predetermined threshold. In particular embodiments, a cancer recurrence score equal to the predetermined threshold may result in a cancer recurrence status of either at risk for cancer recurrence or at low or lower risk for cancer recurrence.

Methods of classifying a test subject as being a candidate for a subsequent cancer treatment may comprise comparing the cancer recurrence score of the test subject with a predetermined cancer recurrence threshold, thereby classifying the test subject as a candidate for the subsequent cancer treatment when the cancer recurrence score is above the cancer recurrence threshold or not a candidate for therapy when the cancer recurrence score is below the cancer recurrence threshold. In particular embodiments, a cancer recurrence score equal to the cancer recurrence threshold may result in classification as either a candidate for a subsequent cancer treatment or not a candidate for therapy. In some embodiments, the subsequent cancer treatment comprises chemotherapy or administration of a therapeutic composition.

Any of such methods may comprise determining a disease-free survival (DFS) period for the test subject based on the cancer recurrence score; for example, the DFS period may be 1 year, 2 years, 3, years, 4 years, 5 years, or 10 years.

In some embodiments, the set of sequence information comprises intronic or exonic target region sequences and/or sequence-variable target region sequences and determining the cancer recurrence score may comprise determining at least a first subscore indicative of the amount of rearrangements, SNVs, insertions/deletions, CNVs and/or fusions present in sequence-variable target region sequences.

In some embodiments, a number of mutations in the sequence-variable target regions chosen from 1, 2, 3, 4, or 5 is sufficient for the first subscore to result in a cancer recurrence score classified as positive for cancer recurrence. In some embodiments, the number of mutations is chosen from 1, 2, or 3.

In some embodiments, the set of sequence information comprises epigenetic target region sequences, and determining the cancer recurrence score comprises determining a second subscore indicative of the amount of molecules (obtained from the epigenetic target region sequences) that represent an epigenetic state different from DNA found in a corresponding sample from a healthy subject (e.g., cfDNA found in a blood sample from a healthy subject, or DNA found in a tissue sample from a healthy subject where the tissue sample is of the same type of tissue as was obtained from the test subject). These abnormal molecules (i.e., molecules with an epigenetic state different from DNA found in a corresponding sample from a healthy subject) may be consistent with epigenetic changes associated with cancer, e.g., methylation of hypermethylation variable target regions and/or perturbed fragmentation of fragmentation variable target regions, where "perturbed" means different from DNA found in a corresponding sample from a healthy subject.

In some embodiments, a proportion of molecules corresponding to the hypermethylation variable target region set and/or fragmentation variable target region set that indicate hypermethylation in the hypermethylation variable target region set and/or abnormal fragmentation in the fragmentation variable target region set greater than or equal to a value in the range of 0.001%-10% is sufficient for the second subscore to be classified as positive for cancer recurrence. The range may be 0.001%-1%, 0.005%-1%, 0.01%-5%, 0.01%-2%, or 0.01%-1%.

In some embodiments, any of such methods may comprise determining a fraction of tumor DNA from the fraction of molecules in the set of sequence information that indicate one or more features indicative of origination from a tumor cell. This may be done for molecules corresponding to some or all of the target regions, e.g., including one or both of target regions comprising a rearrangement, hypermethylation variable target regions, and fragmentation variable target regions (hypermethylation of a hypermethylation variable target region and/or abnormal fragmentation of a fragmentation variable target region may be considered indicative of origination from a tumor cell). This may be done for molecules corresponding to sequence variable target regions, e.g., molecules comprising alterations consistent with cancer, such as SNVs, indels, CNVs, and/or fusions. The fraction of tumor DNA may be determined based on a combination of molecules corresponding to epigenetic target regions and molecules corresponding to sequence variable target regions.

Determination of a cancer recurrence score may be based at least in part on the fraction of tumor DNA, wherein a fraction of tumor DNA greater than a threshold in the range of 10⁻¹¹ to 1 or 10⁻¹⁰ to 1 is sufficient for the cancer recurrence score to be classified as positive for cancer recurrence. In some embodiments, a fraction of tumor DNA greater than or equal to a threshold in the range of 10⁻¹⁰ to 10⁻⁹, 10⁻⁹ to 10⁻⁸, 10⁻⁸ to 10⁻⁷, 10⁻⁷ to 10⁻⁶, 10⁻⁶ to 10⁻⁵, 10⁻⁵ to 10⁻⁴, 10⁻⁴ to 10⁻³, 10⁻³ to 10⁻², or 10⁻² to 10⁻¹ is sufficient for the cancer recurrence score to be classified as positive for cancer recurrence. In some embodiments, the fraction of tumor DNA greater than a threshold of at least 10⁻⁷ is sufficient for the cancer recurrence score to be classified as positive for cancer recurrence. A determination that a fraction of tumor DNA is greater than a threshold, such as a threshold corresponding to any of the foregoing embodiments, may be made based on a cumulative probability. For example, the sample was considered positive if the cumulative probability that the tumor fraction was greater than a threshold in any of the foregoing ranges exceeds a probability threshold of at least 0.5, 0.75, 0.9, 0.95, 0.98, 0.99, 0.995, or 0.999. In some embodiments, the probability threshold is at least 0.95, such as 0.99.

In some embodiments, the set of sequence information comprises intronic or exonic target region sequences, sequence-variable target region sequences, and epigenetic target region sequences, and determining the cancer recurrence score comprises determining a first subscore indicative of the presence or level of rearrangements, a second subscore indicative of the amount of SNVs, insertions/deletions, CNVs and/or fusions present in sequence-variable target region sequences and a third subscore indicative of the amount of abnormal molecules in epigenetic target region sequences, and combining the first, second, and third subscores to provide the cancer recurrence score. Where the subscores are combined, they may be combined by applying a threshold to each subscore independently (e.g., greater than a predetermined number of rearrangements or mutations (e.g., > 1) in intronic target regions or sequence-variable target regions, respectively, and greater than a predetermined fraction of abnormal molecules (i.e., molecules with an epigenetic state different from the DNA found in a corresponding sample from a healthy subject; e.g., tumor) in epigenetic target regions), or training a machine learning classifier to determine status based on a plurality of positive and negative training samples.

In some embodiments, a value for the combined score in the range of -4 to 2 or -3 to 1 is sufficient for the cancer recurrence score to be classified as positive for cancer recurrence.

In any embodiment where a cancer recurrence score is classified as positive for cancer recurrence, the cancer recurrence status of the subject may be at risk for cancer recurrence and/or the subject may be classified as a candidate for a subsequent cancer treatment.

In some embodiments, the cancer is any one of the types of cancer described elsewhere herein, e.g., colorectal cancer.

### 3. Therapies and Related Administration

**In** certain embodiments, the methods disclosed herein relate to identifying and administering customized therapies to patients given the status of a nucleic acid variant as being of somatic or germline origin. In some embodiments, essentially any cancer therapy (e.g., surgical therapy, radiation therapy, chemotherapy, and/or the like) may be included as part of these methods. Typically, customized therapies include at least one immunotherapy (or an immunotherapeutic agent). Immunotherapy refers generally to methods of enhancing an immune response against a given cancer type. In certain embodiments, immunotherapy refers to methods of enhancing a T cell response against a tumor or cancer.

In certain embodiments, the status of a nucleic acid variant from a sample from a subject as being of somatic or germline origin may be compared with a database of comparator results from a reference population to identify customized or targeted therapies for that subject. Typically, the reference population includes patients with the same cancer or disease type as the test subject and/or patients who are receiving, or who have received, the same therapy as the test subject. A customized or targeted therapy (or therapies) may be identified when the nucleic variant and the comparator results satisfy certain classification criteria (e.g., are a substantial or an approximate match).

In certain embodiments, the customized therapies described herein are typically administered parenterally (e.g., intravenously or subcutaneously). Pharmaceutical compositions containing an immunotherapeutic agent are typically administered intravenously. Certain therapeutic agents are administered orally. However, customized therapies (e.g., immunotherapeutic agents, etc.) may also be administered by methods such as, for example, buccal, sublingual, rectal, vaginal, intraurethral, topical, intraocular, intranasal, and/or intraauricular, which administration may include tablets, capsules, granules, aqueous suspensions, gels, sprays, suppositories, salves, ointments, or the like.

### IV. Kits

Also provided are kits comprising the compositions as described herein. The kits can be useful in performing the methods as described herein. In some embodiments, a kit comprises primers that anneal to at least one target region. In some such embodiments, the primers are configured to tile the entire target region in a wild type sequence. In some embodiments, the kit comprises a 5' to 3' exonuclease negative, strand displacement negative DNA polymersase. In some embodiments, the DNA polymerase is also 3' to 5' exonuclease positive. In some embodiments, the kit comprises deoxynucleoside triphosphates. In some embodiments, the deoxynucleoside triphosphates comprise a low percentage of a deoxynucleoside triphosphate linked to a capture moiety, such as biotin. In some embodiments, the kit comprises a solid support linked to a binding partner of the capture moiety. In some embodiments, the kit comprises adapters. In some embodiments, the kit comprises PCR primers, wherein the PCR primers anneal to a target region or to an adapter. In some embodiments, the kit comprises blocking oligonucleotides. In some embodiments, the kit comprises additional elements elsewhere herein. In some embodiments, the kit comprises instructions for performing a method described herein.

The target regions to which the primers of the kit anneal include J exons, intronic regions, such as the intronic regions of BCR-ABL fusion. and fusions comprising any of ALK, FGFR2, FGFR3, NTRK1, RET, or ROS1; sequence-variable target regions, and epigenetic target regions.

Kits may further comprise a plurality of oligonucleotide probes that selectively hybridize to least 5, 6, 7, 8, 9, 10, 20, 30, 40 or all genes selected from the group consisting of ALK, APC, BRAF, CDKN2A, EGFR, ERBB2, FBXW7, KRAS, MYC, NOTCH1, NRAS, PIK3CA, PTEN, RBI, TP53, MET, AR, ABL1, AKTl, ATM, CDHl, CSFIR, CTNNBl, ERBB4, EZH2, FGFRl, FGFR2, FGFR3, FLT3, GNA11, GNAQ, GNAS, HNF1A, HRAS, IDH1, IDH2, JAK2, JAK3, KDR, KIT, MLH1, MPL, NPM1, PDGFRA, PROC, PTPN11, RET,SMAD4, SMARCB1, SMO, SRC, STK11, VHL, TERT, CCND1, CDK4, CDKN2B, RAF1, BRCA1, CCND2, CDK6, NF1, TP53, ARID 1 A, BRCA2, CCNE1, ESR1, RIT1, GATA3, MAP2K1, RHEB, ROS1, ARAF, MAP2K2, NFE2L2, RHOA, and NTRKl . The number genes to which the oligonucleotide probes can selectively hybridize can vary. For example, the number of genes can comprise 1 , 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, or 54. The kit can include a container that includes the plurality of oligonucleotide probes and instructions for performing any of the methods described herein.

The oligonucleotide probes can selectively hybridize to exon regions of the genes, e.g., of the at least 5 genes. In some cases, the oligonucleotide probes can selectively hybridize to at least 30 exons of the genes, e.g., of the at least 5 genes. In some cases, the multiple probes can selectively hybridize to each of the at least 30 exons. The probes that hybridize to each exon can have sequences that overlap with at least 1 other probe. In some embodiments, the oligoprobes can selectively hybridize to non-coding regions of genes disclosed herein, for example, intronic regions of the genes. The oligoprobes can also selectively hybridize to regions of genes comprising both exonic and intronic regions of the genes disclosed herein.

Any number of exons can be targeted by the oligonucleotide probes. For example, at least 1, 2, 3, 4, 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, , 295, 300, 400, 500, 600, 700, 800, 900, 1,000, or more, exons can be targeted.

The kit can comprise at least 4, 5, 6, 7, or 8 different library adapters having distinct molecular barcodes and identical sample barcodes. The library adapters may not be sequencing adapters. For example, the library adapters do not include flow cell sequences or sequences that permit the formation of hairpin loops for sequencing. The different variations and combinations of molecular barcodes and sample barcodes are described throughout, and are applicable to the kit. Further, in some cases, the adapters are not sequencing adapters. Additionally, the adapters provided with the kit can also comprise sequencing adapters. A sequencing adapter can comprise a sequence hybridizing to one or more sequencing primers. A sequencing adapter can further comprise a sequence hybridizing to a solid support, e.g., a flow cell sequence. For example, a sequencing adapter can be a flow cell adapter. The sequencing adapters can be attached to one or both ends of a polynucleotide fragment. In some cases, the kit can comprise at least 8 different library adapters having distinct molecular barcodes and identical sample barcodes. The library adapters may not be sequencing adapters. The kit can further include a sequencing adapter having a first sequence that selectively hybridizes to the library adapters and a second sequence that selectively hybridizes to a flow cell sequence. In another example, a sequencing adapter can be hairpin shaped. For example, the hairpin shaped adapter can comprise a complementary double stranded portion and a loop portion, where the double stranded portion can be attached {e.g. , ligated) to a double-stranded polynucleotide. Hairpin shaped sequencing adapters can be attached to both ends of a polynucleotide fragment to generate a circular molecule, which can be sequenced multiple times. A sequencing adapter can be up to 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more bases from end to end. The sequencing adapter can comprise 20-30, 20-40, 30-50, 30-60, 40-60, 40-70, 50-60, 50-70, bases from end to end. In a particular example, the sequencing adapter can comprise 20-30 bases from end to end. In another example, the sequencing adapter can comprise 50-60 bases from end to end. A sequencing adapter can comprise one or more barcodes. For example, a sequencing adapter can comprise a sample barcode. The sample barcode can comprise a pre-determined sequence. The sample barcodes can be used to identify the source of the polynucleotides. The sample barcode can be at least 1, 2, 3, 4, 5, 6, 7, 8, 9, 10, 11, 12, 13, 14, 15, 16, 17, 18, 19, 20, 21, 22, 23, 24, 25, or more (or any length as described throughout) nucleic acid bases, e.g., at least 8 bases. The barcode can be contiguous or non-contiguous sequences, as described above.

The library adapters can be blunt ended and Y-shaped and can be less than or equal to 40 nucleic acid bases in length. Other variations of the can be found throughout and are applicable to the kit.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the disclosure described herein may be employed in practicing the invention. The following claims define the invention.

While the foregoing disclosure has been described in some detail by way of illustration and example for purposes of clarity and understanding, it will be clear to one of ordinary skill in the art from a reading of this disclosure that various changes in form and detail can be made. For example, all the methods, systems, computer readable media, and/or component features, steps, elements, or other aspects thereof can be used in various combinations.

If different versions of a sequence are associated with an accession number at different times, the version associated with the accession number at the effective filing date of this application is meant. The effective filing date means the earlier of the actual filing date or filing date of a priority application referring to the accession number, if applicable. Likewise, if different versions of a publication, website or the like are published at different times, the version most recently published at the effective filing date of the application is meant, unless otherwise indicated.

### EXAMPLES

### Example 1: Analysis of cfDNA to detect the presence/absence of a rearrangement in DNA from a subject

A set of patient samples are analyzed by a blood-based assay at Guardant Health (Redwood City, CA, USA) to detect the presence/absence of mutations. cfDNA is extracted from the plasma of these patients. Adapters are then added to the patient cfDNA by ligation to the 3' ends thereof. The 3' end adapter is used as a priming site for second-strand synthesis using a universal primer and a DNA polymerase. Further adapters are ligated to the 5' ends of the cfDNA. The adapters contain non-unique molecular barcodes. After adapter ligation, the cfDNA is amplified by PCR using primers that anneal to primer binding sites in the adapters.

The amplified DNA is washed and concentrated, and then combined with a buffer and primers 30 to 40 nucleotides in length that are complementary to the one or more intronic target regions of the cfDNA. The primers tile the entire intronic target region or regions with intervening gaps (e.g., about 5 nt) short enough to limit primer extension by a 5' to 3' exonuclease negative, strand displacement negative DNA polymerase when primers are annealed in proximity to each other. A blocking oligonucleotide that is non-extendable and anneals to the exon adjacent to the intronic region can also be included to block extension of the primer with the closest 3' end to the exon. Deoxynucleoside triphosphates in which 1-20% (e.g., 10%) of the deoxycytidine triphosphate is biotinylated and Q5 DNA polymerase (New England Biolabs) are added to the primer-annealed DNA in order to extend primers annealed to a DNA molecule comprising a rearrangement, such as a translocation breakpoint or indel. Primers annealed to a DNA molecule comprising a rearrangement near a breakpoint or junction of the rearrangement are substrates for longer extension because extension is not blocked by a subsequent annealed primer. The mixture is incubated at the recommended temperature for the DNA polymerase to perform primer extension. In this way, primer-extended products are substantially limited to products templated by target regions comprising a rearrangement, as a result of which there was an unblocked annealed primer.

Magnetic beads conjugated with streptavidin are added to the DNA to capture any biotinylated primer-extended products. DNA that does not comprise a rearrangement in the target region, unextended primers, and primer-extended products too short to have incorporated a biotinylated nucleotide are separated from the biotinylated primer-extended products by a series of salt washes comprising increasing concentrations of salt. The resulting enriched biotinylated primer-extended products are eluted from the beads using high salt buffer, and the eluted DNA molecules are cleaned to remove salt. An aliquot of the eluted, cleaned DNA is sequenced using Illumina NovaSeq sequencer. The sequence reads generated by the sequencer are then analyzed using bioinformatic tools/algorithms. The molecular barcodes within the adapters are used to identify unique molecules. The method described in this example, in addition to providing information on the intron comprising a rearrangement, can also provide high-resolution information about the identity and/or location of the rearrangement breakpoint.

### Example 2: Analysis of cfDNA to detect the presence/absence of a rearrangement and epigenetic and sequence variation in DNA from a subject

Samples of cfDNA from healthy subjects and subjects with early-stage colorectal cancer are analyzed as follows. cfDNA is first extracted from the plasma of the subjects, then adapters are added as described in Example 1. After adapter ligation, the cfDNA is amplified and separated into a plurality of aliquots. One aliquot is subjected to the primer tiling and extension and enrichment procedures described in Example 1.

A second aliquot is combined with a salt buffer and biotinylated RNA probes for a sequence-variable target region set and optionally for an epigenetic target region set (e.g., for embodiments in which the sample was partitioned into at least hypermethylated and hypomethylated subsamples and differentially tagged) and incubated overnight. The probes for the sequence-variable region set have a footprint of about 50 kb and the probes for the epigenetic target region set, if used, have a footprint of about 500 kb. The probes for the sequence-variable target region set comprise oligonucleotides targeting at least a subset of genes identified in Tables 3-5 and the probes for the epigenetic target region set comprise oligonucleotides targeting a selection of hypermethylation variable target regions, hypomethylation variable target regions, CTCF binding target regions, transcription start site target regions, focal amplification target regions and methylation control regions.

The biotinylated RNA probes hybridized to the DNA are captured by streptavidin magnetic beads and separated from the DNA that are not captured by a series of salt based washes, thereby enriching the sample for sequences complementary to the probes. After enrichment, aliquots of the sample enriched using the biotinylated RNA probes and of the sample enriched using primer extension procedures of Example 1 are pooled and sequenced using an Illumina NovaSeq sequencer. The sequence reads generated by the sequencer are then analyzed using bioinformatic tools/algorithms. The molecular barcodes within the adapters are used to identify unique molecules as well as for deconvolution of the sample into molecules that were differentially partitioned. The sequence-variable target region sequences are analyzed by detecting genomic alterations such as SNVs, insertions, deletions and fusions that can be called with enough support that differentiates real tumor variants from technical errors (for e.g., PCR errors, sequencing errors). If applicable, the epigenetic target region sequences are analyzed independently to detect methylated cfDNA molecules in regions that have been shown to be differentially methylated in cancer compared to normal cells.

In addition to providing information on the presence of rearrangements in the intronic target regions, the presence of mutations in the sequence-variable target regions, and if applicable the epigenetic status (e.g., level of methylation) of the epigenetic target regions, the method described in this example can also provide high-resolution information about the identity and/or location of rearrangement breakpoints. Finally, the results of the analyses are combined to produce a final tumor present/absent call. For example, mutations in the sequence-variable target regions, rearrangements in the intronic target regions, and if applicable, methylation for the hypermethylation variable target regions are quantified and compared to baseline values from known healthy samples. The samples from subjects with colorectal cancer exhibit substantially higher overall frequencies of mutations, rearrangements, and if applicable, methylation in the analyzed target regions than in corresponding regions in samples from healthy subjects.

### Example 3: Analysis of cfDNA to detect the levels of VDJ recombination in DNA from a subject

A set of patient samples are analyzed by a blood-based assay at Guardant Health (Redwood City, CA, USA) to detect rearranged or recombined VDJ sequences. cfDNA is extracted from the plasma of these patients, adapters are added, and the DNA is amplified as described in Example 1.

The amplified DNA is washed and concentrated, and then combined with a buffer, primers 30 to 40 nucleotides in length that are each complementary to the terminus of a J exon, and blocking oligonucleotides complementary to introns adjacent (in the germline, unrearranged locus) to the termini of J exons to which the primers are complementary. The blocking oligonucleotides thus prevent extension of primers that anneal to a J exon that has not undergone rearrangement and do not prevent extension of primers that anneal to a J exon adjacent to another exon due to VDJ recombination. The mixture is incubated to permit annealing. Deoxynucleoside triphosphates comprising 1-20% (e.g., 10%) of biotinylated deoxycytidine triphosphate and Q5 DNA polymerase (New England Biolabs) are then added to the primer-annealed DNA so that primers annealed to J exons in rearranged sequences are extended.

Magnetic beads conjugated with streptavidin are added to the DNA to capture any biotinylated primer-extended products. DNA that does not comprise VDJ recombination in the target region, primers, and primer-extended products too short to have incorporated a biotinylated nucleotide are separated from the biotinylated primer-extended products by a series of salt washes comprising increasing concentrations of salt. The resulting enriched biotinylated primer-extended products are eluted from the beads using high salt buffer, and the eluted DNA molecules are cleaned to remove salt. An aliquot of the eluted, cleaned DNA is sequenced using Illumina NovaSeq sequencer. The sequence reads generated by the sequencer are then analyzed using bioinformatic tools/algorithms. The molecular barcodes within the adapters are used to identify unique molecules. The results are analyzed to produce one or more recombined VDJ sequences as output.

## Claims

1. A method of analyzing DNA in a sample, the DNA comprising intronic regions, the method comprising:
a) contacting the DNA with a plurality of primers, wherein the plurality of primers comprises at least two primers that anneal in a parallel orientation to a target region, thereby producing primer-annealed DNA; and
b) contacting the primer-annealed DNA with a DNA polymerase and deoxynucleoside triphosphates, wherein the DNA polymerase is a 5' to 3' exonuclease negative, strand displacement negative DNA polymerase, thereby producing a primer-extended sample, wherein for at least half of the primers, when annealed to a wild type target region, extension is blocked by a downstream primer; and
c) detecting a presence or absence of a DNA molecule that comprises a structural variation, wherein the detecting comprises sequencing DNA in the primer-extended sample.

2. The method of claim 1, further comprising:
i) ligating adapters to the DNA, thereby producing adapter-ligated DNA;
ii) amplifying the adapter-ligated DNA, thereby producing amplified adapter-ligated DNA, wherein at least a portion of the the amplified adapter-ligated DNA is the DNA contacted in step (a);
iii) enriching or capturing DNA that anneal to a primer-extended product in the primer-extended sample; and
iv) amplifying the enriched or captured DNA, then sequencing the amplified enriched or captured DNA,
optionally further comprising: (i) capturing sequence-variable target regions from the adapter-ligated DNA and amplifying and sequencing the sequence-variable target regions, and/or (ii) capturing epigenetic target regions from the adapter-ligated DNA and amplifying and sequencing the epigenetic target regions.

3. The method of claim 1 or claim 2, wherein:
(a) the target region comprises: (i) an intronic region, (ii) an exonic VDJ region; and/or (iii) an intergenic region; and/or
(b) extension of at least 70%, at least 80%, or at least 90% of the primers annealed to a wild type target region are blocked by a downstream primer.

4. The method of any one of the preceding claims, wherein:
(a) the DNA comprises a plurality of intronic target regions and the plurality of primers comprises at least two primers that anneal in a parallel orientation to each of the plurality of intronic target regions;
(b) the DNA comprises an exonic VDJ region and the plurality of primers comprises at least two primers that anneal in a parallel orientation to the exonic VDJ target region;
(c) the DNA comprises cell-free DNA (cfDNA) molecules;
(d) the DNA is obtained from a test subject;
(e) the DNA comprises DNA obtained from a tissue sample of a test subject, optionally
wherein the tissue sample is a biopsy, a fine needle aspirate, or a formalin-fixed paraffin-embedded tissue sample; and/or
(f) the DNA comprises a plurality of intronic target regions, and wherein the plurality of primers comprises at least two primers that anneal to each of the plurality of intronic target regions, optionally wherein the plurality of intronic target regions comprises at least 3, 5, 10, 15, or 20 intronic target regions.

5. The method of any one of the preceding claims, wherein:
(a) the plurality of primers comprises at least one primer that anneals to an exon-exon junction region of the DNA;
(b) each of the plurality of primers: (i) comprises at least 20 linked nucleosides; (ii) consists of 20 to 60 linked nucleosides; (iii) consists of 25 to 60 linked nucleosides; (iv) comprises 30 to 40 linked nucleosides; or (v) consists of 30 to 40 linked nucleosides;
(c) each of the plurality of primers comprises a label, optionally wherein: (i) the label is a capture moiety; and/or (ii) the label is biotin; and/or
(d) the primer-annealed DNA comprises single-stranded portions of 0 to 50 linked nucleosides or 0 to 10 linked nucleosides between at least a portion of the pair or pairs of adjacent annealed primers.

6. The method of any of the preceding claims, wherein:
(a) the DNA polymerase is Q5 High Fidelity DNA polymerase and/or a 3' to 5' exonuclease positive DNA polymerase; and/or
(b) the deoxynucleoside triphosphates comprise a modified deoxynucleoside triphosphate, optionally wherein:
(i) the modified deoxynucleoside triphosphate comprises a label;
(ii) the modified deoxynucleoside triphosphate comprises a capture moiety;
(iii) the modified deoxynucleoside triphosphate comprises a biotin;
(iv) the deoxynucleoside triphosphates comprise the modified deoxynucleoside triphosphate and the unmodified version of the modified deoxynucleoside triphosphate, optionally wherein the ratio of the modified:unmodified versions of the modified deoxynucleoside triphosphate is from 1:1 to 1:100;
(v) the modified deoxynucleoside triphosphate is modified deoxycytidine triphosphate;
(vi) the modified deoxynucleoside triphosphate is biotinylated deoxycytidine triphosphate, biotinylated deoxyadenosine triphosphate, or biotinylated deoxyuridine triphosphate; and/or
(vii) the deoxynucleoside triphosphates comprise unmodified deoxyadenosine triphosphate, unmodified deoxyguanosine triphosphate, unmodified deoxycytidine triphosphate, and unmodified thymidine triphosphate.

7. The method of any one of the preceding claims, wherein:
(a) the detecting of any primer-extended products comprises separating nucleic acids in the primer-extended sample by size;
(b) the detecting of any primer-extended products comprises contacting the primer-extended sample with a capture reagent, optionally wherein the capture reagent comprises streptavidin;
(c) the detecting of any primer-extended products comprises amplifying nucleic acids in the primer-extended sample, optionally wherein the detecting of any primer-extended products comprises sequencing the DNA in the primer-extended sample after amplifying nucleic acids in the primer-extended sample;
(d) the primers are resistant to 5' exonucleolysis, and the method further comprises contacting the primer-extended sample with a 5' to 3' exonuclease before the detecting step, optionally wherein the primers resistant to 5' exonucleolysis comprise one or more phosphorothioate linkages; and/or
(e) the method further comprises ligating barcode-containing adapters to the DNA before contacting the DNA with primers.

8. The method of any of the preceding claims, further comprising contacting the DNA with one or more blocking oligonucleotides, optionally wherein:
(a) the one or more blocking oligonucleotides comprise one or more blocking oligonucleotides that anneal to an adapter or to an exonic region of the DNA;
(b) the one or more blocking oligonucleotides comprise a blocking oligonucleotide that anneals to a 3' adapter;
(c) the one or more blocking oligonucleotides comprise a blocking oligonucleotide that anneals to an exon adjacent to the 5' end of an intronic region of the DNA;
(d) the one or more blocking oligonucleotides comprise a blocking primer that anneals to a 5' adapter;
(e) the one or more blocking oligonucleotides comprise blocking oligonucleotides that anneal to an adapter or to an intronic region of the DNA within or adjacent to an exonic VDJ region, optionally wherein the one or more blocking oligonucleotides comprise a blocking oligonucleotide that anneals to a J intronic region;
(f) each blocking oligonucleotide is complementary to an adapter or a region adjacent to a target region of the DNA;
(g) the blocking oligonucleotides comprise a modified nucleoside at the 3' end that blocks extension of the blocking oligonucleotides by the DNA polymerase, optionally wherein the modified nucleoside at the 3' end of the blocking oligonucleotides is an abasic nucleoside, an inverted nucleoside, a phosphorylated nucleoside, or a dideoxynucleoside, optionally wherein: (i) the inverted nucleoside is inverted thymidine; or (ii) the dideoxynucleoside is dideoxycytidine; and/or
(h) the blocking oligonucleotides comprise a modified nucleoside at the 3' end that is not resistant to 3' exonucleolysis, optionally wherein the modified nucleoside at the 3' end of the blocking oligonucleotides is an abasic nucleoside, an inverted nucleoside, a phosphorylated nucleoside, or a dideoxynucleoside, optionally wherein: (i) the inverted nucleoside is inverted thymidine; or (ii) the dideoxynucleoside is dideoxycytidine.

9. The method of any of the preceding claims, wherein:
(a) the plurality of primers comprise at least two primers comprising a portion at the 5' end that is not complementary to the DNA, optionally wherein the portion at the 5' end of the primers that is not complementary to the DNA comprises a capture moiety, optionally wherein the capture moiety is biotin; and/or
(b) the detecting comprises contacting the primer-extended sample with a capture reagent and amplifying nucleic acids bound to the capture reagent, optionally wherein the capture reagent comprises streptavidin.

10. The method of any one of the preceding claims, wherein:
(a) the structural variation comprises a rearrangement, an insertion, or a deletion;
(b) the structural variation comprises a rearrangement, optionally wherein the rearrangement comprises a translocation breakpoint, optionally wherein the target region of the DNA comprises an intronic region of the DNA comprising the translocation breakpoint;
(c) the structural variation comprises a rearrangement wherein the rearrangement comprises a VDJ recombination, optionally wherein the target region of the DNA comprises an exonic VDJ region comprising the VDJ recombination, optionally wherein:
(i) the plurality of primers comprises at least two primers that anneal to a J exon, optionally wherein the plurality of primers comprises at least 3, 4, 5, 6, 7, 8, 9, 10, 11, 12, or 13 primers that anneal to different J exons;
(ii) the exonic VDJ region comprises a CDR3;
(iii) the exonic VDJ region is a part of a T cell receptor coding sequence;
(iv) wherein the method further comprises contacting the DNA with a plurality of blocking oligonucleotides that block extension of primers of the plurality when annealed to germline sequence;
(v) the DNA comprises adapters, the method further comprising contacting the DNA with one or more adapter blocking oligonucleotides that block extension of primers of the plurality across one or more of the adapters; and/or
(vi) the primers of the plurality are labeled, optionally wherein the primers of the plurality are labeled with biotin, optionally wherein complexes comprising extended primers are preferentially isolated relative to unextended primers, optionally wherein preferentially isolating the complexes comprising the extended primers comprises pulling down the complexes at a temperature above the melting temperature of the unextended primers; and/or
(d) the structural variation comprises a rearrangement, wherein the rearrangement comprises a translocation breakpoint, wherein the method comprises measuring the levels of DNA in the sample comprising a translocation breakpoint or VDJ recombination and comparing them to reference levels.

11. The method of any one of the preceding claims, comprising determining a likelihood that the subject has cancer, optionally wherein the cancer is a lymphocytic cancer, optionally wherein the lymphocytic cancer is a leukemia, a lymphoma, or a myeloma.

12. The method of any one of the preceding claims, wherein steps a)-b) are performed on a first aliquot of a sample, and one or more of a conversion step, a partitioning step, and a capture step are performed on the sample or a second aliquot of a sample, optionally comprising:
(a) capturing at least a sequence-variable target region set from the sample or the second aliquot; and/or
(b) capturing at least an epigenetic target region set of DNA from the sample or the second aliquot, optionally wherein:
(i) the epigenetic target region set comprises a hypermethylation variable target region set, optionally wherein the hypermethylation variable target region set comprises differentially methylated regions;
(ii) the epigenetic target region set comprises a hypomethylation variable target region set, optionally wherein the hypomethylation variable target region set comprises regions having a lower degree of methylation in at least one type of tissue than the degree of methylation in cell-free DNA from a healthy subject;
(iii) the epigenetic target region set comprises a methylation control target region set; and/or
(iv) the epigenetic target region set comprise a fragmentation variable target region set, optionally wherein the fragmentation variable target region set comprises transcription start site regions and/or CTCF binding regions.

13. The method of any one of the preceding claims, comprising:
(a) subjecting the sample or a subsample thereof (such as a second aliquot) to a procedure that affects a first nucleobase in the DNA differently from a second nucleobase in the DNA of the sample, wherein the first nucleobase is a modified or unmodified nucleobase, the second nucleobase is a modified or unmodified nucleobase different from the first nucleobase, and the first nucleobase and the second nucleobase have the same base pairing specificity, thereby producing a converted sample, optionally comprising sequencing the captured DNA in a manner that distinguishes the first nucleobase from the second nucleobase; and/or
(b) partitioning the sample into a plurality of subsamples by contacting the DNA with an agent that recognizes a modified nucleobase in the DNA, the plurality comprising a first subsample and a second subsample, wherein the first subsample comprises DNA with a cytosine modification in a greater proportion than the second subsample, and the modified nucleobase recognized by the agent is a modified cytosine or a product of a procedure that affects the first nucleobase in the DNA differently from the second nucleobase in the DNA of the sample.

14. The method of claim 12 or claim 13, wherein:
(a) the partitioning step comprises partitioning on the basis of methylation level, optionally wherein the agent that recognizes a modified nucleobase in the DNA is a methyl binding reagent, optionally wherein: (i) the methyl binding reagent is an antibody; (ii) the methyl binding reagent specifically recognizes 5-methylcytosine; (iii) the methyl binding reagent is immobilized on a solid support; and/or (iv) the partitioning step comprises immunoprecipitation of methylated DNA;
(b) the partitioning step comprises partitioning on the basis of binding to a protein, optionally wherein the protein is a methylated protein, an acetylated protein, an unmethylated protein, an unacetylated protein; and/or optionally wherein the protein is a histone, optionally wherein the partitioning step comprises contacting the DNA of the sample with a binding reagent which is specific for the protein and is immobilized on a solid support;
(c) the method comprises partitioning the sample into a plurality of subsamples by contacting the DNA with an agent that recognizes a modified nucleobase in the DNA, the plurality comprising a first subsample and a second subsample, wherein the first subsample comprises DNA with a cytosine modification in a greater proportion than the second subsample, and the modified nucleobase recognized by the agent is a modified cytosine or a product of a procedure that affects the first nucleobase in the DNA differently from the second nucleobase in the DNA of the sample, wherein the method comprises differentially tagging and pooling the first subsample and second subsample, optionally wherein:
(i) the pool comprises less than or equal to about 45%, 40%, 35%, 30%, 25%, 20%, 15%, 10%, or 5% of the DNA of the second subsample;
(ii) the pool comprises about 70-90%, about 75-85%, or about 80% of the DNA of the second subsample; or
(iii) the pool comprises substantially all of the DNA of the first subsample;
(d) the method comprises partitioning the sample into a plurality of subsamples by contacting the DNA with an agent that recognizes a modified nucleobase in the DNA, the plurality comprising a first subsample and a second subsample, wherein the first subsample comprises DNA with a cytosine modification in a greater proportion than the second subsample, and the modified nucleobase recognized by the agent is a modified cytosine or a product of a procedure that affects the first nucleobase in the DNA differently from the second nucleobase in the DNA of the sample, wherein the plurality of subsamples comprises a third subsample, which comprises DNA with a cytosine modification in a greater proportion than the second subsample but in a lesser proportion than the first subsample, optionally wherein:
(i) the method further comprises differentially tagging the third subsample; and/or
(ii) the first, second, and third subsamples combined after subjecting the first subsample to a procedure that affects the first nucleobase in the DNA differently from the second nucleobase in the DNA of the first subsample, optionally wherein the first, second, and third subsamples are sequenced in the same sequencing cell; and/or
(e) the method comprises subjecting the sample or a subsample thereof (such as a second aliquot) to a procedure that affects a first nucleobase in the DNA differently from a second nucleobase in the DNA of the sample, wherein the first nucleobase is a modified or unmodified nucleobase, the second nucleobase is a modified or unmodified nucleobase different from the first nucleobase, and the first nucleobase and the second nucleobase have the same base pairing specificity, thereby producing a converted sample, wherein:
(i) the procedure to which the first subsample is subjected alters base pairing specificity of the first nucleobase without substantially altering base pairing specificity of the second nucleobase;
(ii) the first nucleobase is a modified or unmodified cytosine and the second nucleobase is a modified or unmodified cytosine;
(iii) the first nucleobase comprises unmodified cytosine (C), optionally wherein the second nucleobase comprises 5-methylcytosine (mC); and/or
(iv) the first nucleobase comprises mC, optionally wherein the second nucleobase comprises 5-hydroxymethylcytosine (5hmC) or C.

15. The method of any one of the preceding claims, wherein:
(a) the detecting comprises generating a plurality of sequencing reads; and the method further comprises mapping the plurality of sequence reads to one or more reference sequences to generate mapped sequence reads, and processing the mapped sequence reads to determine the likelihood that the subject has cancer; and/or
(b) the sample is from a test subject who was previously diagnosed with a cancer and received one or more previous cancer treatments, optionally wherein the DNA is obtained at one or more preselected time points following the one or more previous cancer treatments, and sequencing DNA in the primer-extended sample, whereby a set of sequence information is produced, optionally further comprising detecting a presence or absence of DNA originating or derived from a tumor cell at a preselected timepoint using the set of sequence information, optionally further comprising determining a cancer recurrence score that is indicative of the presence or absence of the DNA originating or derived from the tumor cell for the test subject, optionally further comprising determining a cancer recurrence status based on the cancer recurrence score, wherein the cancer recurrence status of the test subject is determined to be at risk for cancer recurrence when a cancer recurrence score is determined to be at or above a predetermined threshold or the cancer recurrence status of the test subject is determined to be at lower risk for cancer recurrence when the cancer recurrence score is below the predetermined threshold, optionally further comprising comparing the cancer recurrence score of the test subject with a predetermined cancer recurrence threshold, wherein the test subject is classified as a candidate for a subsequent cancer treatment when the cancer recurrence score is above the cancer recurrence threshold or not a candidate for a subsequent cancer treatment when the cancer recurrence score is below the cancer recurrence threshold.

## Patentansprüche

1. Verfahren zur Analyse von DNA in einer Probe, wobei die DNA intronische Regionen umfasst, wobei das Verfahren umfasst:
a) Inkontaktbringen der DNA mit einer Vielzahl von Primern, wobei die Vielzahl von Primern mindestens zwei Primer umfasst, die in einer parallelen Orientierung an eine Zielregion anlagern, wodurch Primer-angelagerte DNA erzeugt wird; und
b) Inkontaktbringen der Primer-angelagerten DNA mit einer DNA-Polymerase und Desoxynucleosidtriphosphaten, wobei die DNA-Polymerase eine 5' zu 3' Exonuklease-negative, Strangverdrängungs-negative DNA-Polymerase ist, wodurch eine Primer-erweiterte Probe hergestellt wird, wobei für mindestens die Hälfte der Primer, wenn sie an eine Wildtyp-Zielregion angelagert sind, die Erweiterung durch einen stromabwärts liegenden Primer blockiert wird; und
c) Nachweisen des Vorhandenseins oder der Abwesenheit eines DNA-Moleküls, das eine Strukturvariation umfasst, wobei der Nachweis die Sequenzierung der DNA in der mit dem Primer erweiterten Probe umfasst.

2. Verfahren nach Anspruch 1, das ferner umfasst:
i) Ligieren von Adaptern an die DNA, wodurch eine Adapter-ligierte DNA hergestellt wird;
ii) Amplifizieren der Adapter-ligierten DNA, wodurch amplifizierte Adapter-ligierte DNA hergestellt wird, wobei mindestens ein Teil der amplifizierten Adapter-ligierten DNA die in Schritt (a) kontaktierte DNA ist;
iii) Anreichern oder Einfangen von DNA, die sich an ein Primer-erweitertes Produkt in der Primer-erweiterten Probe anlagert; und
iv) Amplifizieren der angereicherten oder eingefangenen DNA, dann Sequenzieren der amplifizierten angereicherten oder eingefangenen DNA,
optional ferner umfassend: (i) Einfangen von sequenzvariablen Zielregionen aus der Adapter-ligierten DNA und Amplifizieren und Sequenzieren der sequenzvariablen Zielregionen, und/oder (ii) Einfangen von epigenetischen Zielregionen aus der Adapter-ligierten DNA und Amplifizieren und Sequenzieren der epigenetischen Zielregionen.

3. Verfahren nach Anspruch 1 oder Anspruch 2, wobei:
a) die Zielregion umfasst: (i) eine intronische Region, (ii) eine exonische VDJ-Region; und/oder (iii) eine intergenische Region; und/oder
b) die Erweiterung von mindestens 70 %, mindestens 80 % oder mindestens 90 % der Primer, die an eine Wildtyp-Zielregion angelagert sind, durch einen stromabwärts liegenden Primer blockiert wird.

4. Verfahren nach einem der vorhergehenden Ansprüche, wobei:
(a) die DNA eine Vielzahl von intronischen Zielregionen umfasst und die Vielzahl von Primern mindestens zwei Primer umfasst, die in einer parallelen Orientierung an jede der Vielzahl von intronischen Zielregionen anlagern;
(b) die DNA eine exonische VDJ-Region umfasst und die Vielzahl der Primer mindestens zwei Primer umfasst, die in einer parallelen Ausrichtung an die exonische VDJ-Zielregion anlagern;
(c) die DNA zellfreie DNA- (cfDNA) Moleküle umfasst;
(d) die DNA von einem Testsubjekt gewonnen wird;
(e) die DNA DNA umfasst, die aus einer Gewebeprobe eines Testsubjekts gewonnen wurde, optional wobei die Gewebeprobe eine Biopsie, ein Feinnadelaspirat oder eine formalinfixierte, in Paraffin eingebettete Gewebeprobe ist; und/oder
(f) die DNA eine Vielzahl von intronischen Zielregionen umfasst, und wobei die Vielzahl von Primern mindestens zwei Primer umfasst, die sich an jede der Vielzahl von intronischen Zielregionen anlagern, optional wobei die Vielzahl der intronischen Zielregionen mindestens 3, 5, 10, 15 oder 20 intronische Zielregionen umfasst.

5. Verfahren nach einem der vorhergehenden Ansprüche, wobei:
(a) die Vielzahl von Primern mindestens einen Primer umfasst, der an eine Exon-Exon-Kreuzungsregion der DNA anlagert;
(b) jeder der Vielzahl von Primern: (i) mindestens 20 verknüpfte Nucleoside umfasst; (ii) aus 20 bis 60 verknüpften Nucleosiden besteht; (iii) aus 25 bis 60 verknüpften Nucleosiden besteht; (iv) 30 bis 40 verknüpfte Nucleoside umfasst; oder (v) aus 30 bis 40 verknüpften Nucleosiden besteht;
(c) jeder der Vielzahl von Primern eine Markierung umfasst, optional wobei: (i) die Markierung eine Einfangkomponente ist; und/oder (ii) die Markierung Biotin ist; und/oder
(d) die Primer-angelagerte DNA einzelsträngige Abschnitte von 0 bis 50 verknüpften Nukleosiden oder 0 bis 10 verknüpften Nukleosiden zwischen mindestens einem Teil des Paares oder der Paare von benachbarten angelagerten Primern umfasst.

6. Verfahren nach einem der vorhergehenden Ansprüche, wobei:
(a) die DNA-Polymerase eine Q5 High Fidelity DNA-Polymerase und/oder eine 3'-5'-Exonuklease-positive DNA-Polymerase ist; und/oder
(b) die Desoxynucleosidtriphosphate ein modifiziertes Desoxynucleosidtriphosphat umfassen, wobei optional:
(i) das modifizierte Desoxynucleosidtriphosphat eine Markierung umfasst;
(ii) das modifizierte Desoxynucleosidtriphosphat eine Einfangkomponente umfasst;
(iii) das modifizierte Desoxynukleosidtriphosphat ein Biotin umfasst;
(iv) die Desoxynucleosidtriphosphate das modifizierte Desoxynucleosidtriphosphat und die unmodifizierte Version des modifizierten Desoxynucleosidtriphosphats umfassen, optional wobei das Verhältnis der modifizierten:unmodifizierten Versionen des modifizierten Desoxynucleosidtriphosphats von 1:1 bis 1:100 beträgt;
(v) das modifizierte Desoxynucleosidtriphosphat modifiziertes Desoxycytidintriphosphat ist;
(vi) das modifizierte Desoxynucleosidtriphosphat biotinyliertes Desoxycytidintriphosphat, biotinyliertes Desoxyadenosintriphosphat oder biotinyliertes Desoxyuridintriphosphat ist; und/oder
(vii) die Desoxynucleosidtriphosphate unmodifiziertes Desoxyadenosintriphosphat, unmodifiziertes Desoxyguanosintriphosphat, unmodifiziertes Desoxycytidintriphosphat und unmodifiziertes Thymidintriphosphat umfassen.

7. Verfahren nach einem der vorhergehenden Ansprüche, wobei:
(a) der Nachweis jeglicher Primer-erweiterter Produkte die Trennung der Nukleinsäuren in der Primer-erweiterten Probe nach Größe umfasst;
(b) der Nachweis jeglicher Primer-erweiterter Produkte das In-Kontakt-Bringen der Primer-erweiterten Probe mit einem Einfangreagenz umfasst, optional wobei das Einfangreagenz Streptavidin umfasst;
(c) der Nachweis jeglicher Primer-erweiterter Produkte das Amplifizieren von Nukleinsäuren in der Primer-erweiterten Probe umfasst, optional wobei der Nachweis jeglicher Primer-erweiterter Produkte das Sequenzieren der DNA in der Primer-erweiterten Probe nach der Amplifikation von Nukleinsäuren in der Primer-erweiterten Probe umfasst;
(d) die Primer resistent gegen 5'-Exonukleolyse sind, und das Verfahren ferner das In-Kontakt-Bringen der Primer-erweiterten Probe mit einer 5'- bis 3'-Exonuklease vor dem Nachweisschritt umfasst, optional wobei die gegen 5'-Exonukleolyse resistenten Primer eine oder mehrere Phosphorothioat-Bindungen umfassen; und/oder
(e) das Verfahren ferner das Ligieren von Barcode-enthaltenden Adaptern an die DNA umfasst, bevor die DNA mit Primern in Kontakt gebracht wird.

8. Verfahren nach einem der vorhergehenden Ansprüche, ferner umfassend das In-Kontakt-Bringen der DNA mit einem oder mehreren blockierenden Oligonukleotiden, wobei optional:
(a) das eine oder die mehreren blockierenden Oligonukleotide ein oder mehrere blockierende Oligonukleotide umfassen, die sich an einen Adapter oder an eine exonische Region der DNA anlagern;
(b) das eine oder die mehreren blockierenden Oligonukleotide ein blockierendes Oligonukleotid umfassen, das sich an einen 3'-Adapter anlagert;
(c) das eine oder die mehreren blockierenden Oligonukleotide ein blockierendes Oligonukleotid umfassen, das an ein Exon angrenzend an das 5'-Ende einer intronischen Region der DNA anlagert;
(d) das eine oder die mehreren blockierenden Oligonukleotide einen blockierenden Primer umfassen, der an einen 5'-Adapter anlagert;
(e) das eine oder die mehreren blockierenden Oligonukleotide blockierende Oligonukleotide umfassen, die an einen Adapter oder an eine intronische Region der DNA innerhalb oder benachbart zu einer exonischen VDJ-Region anlagern, optional wobei das eine oder die mehreren blockierenden Oligonukleotide ein blockierendes Oligonukleotid umfassen, das an eine intronische J-Region anlagert;
(f) jedes blockierende Oligonukleotid komplementär zu einem Adapter oder einer an eine Zielregion der DNA angrenzenden Region ist;
(g) die blockierenden Oligonukleotide ein modifiziertes Nukleosid am 3'-Ende umfassen, das die Erweiterung der blockierenden Oligonukleotide durch die DNA-Polymerase blockiert, optional wobei das modifizierte Nukleosid am 3'-Ende der blockierenden Oligonukleotide ein abasisches Nukleosid, ein invertiertes Nukleosid, ein phosphoryliertes Nukleosid oder ein Dideoxynukleosid ist, optional wobei: (i) das invertierte Nukleosid invertiertes Thymidin ist; oder (ii) das Dideoxynukleosid Dideoxycytidin ist; und/oder
(h) die blockierenden Oligonukleotide ein modifiziertes Nukleosid am 3'-Ende umfassen, das nicht resistent gegen 3'-Exonukleolyse ist, optional wobei das modifizierte Nukleosid am 3'-Ende der blockierenden Oligonukleotide ein abasisches Nukleosid, ein invertiertes Nukleosid, ein phosphoryliertes Nukleosid oder ein Dideoxynukleosid ist, optional wobei: (i) das invertierte Nukleosid invertiertes Thymidin ist; oder (ii) das Dideoxynukleosid Dideoxycytidin ist.

9. Verfahren nach einem der vorhergehenden Ansprüche, wobei:
(a) die Vielzahl von Primern mindestens zwei Primer umfasst, die einen Teil am 5'-Ende umfassen, der nicht komplementär zur DNA ist, optional wobei der Teil am 5'-Ende der Primer, der nicht komplementär zur DNA ist, eine Einfangkomponente umfasst, optional wobei die Einfangkomponente Biotin ist; und/oder
(b) das Nachweisen das Inkontaktbringen der mit Primer erweiterten Probe mit einem Einfangreagenz und das Amplifizieren von Nukleinsäuren, die an das Einfangreagenz angelagert sind, umfasst, optional wobei das Einfangreagenz Streptavidin umfasst.

10. Verfahren nach einem der vorhergehenden Ansprüche, wobei:
(a) die Strukturvariation eine Umlagerung, eine Insertion oder eine Deletion umfasst;
(b) die Strukturvariation eine Umlagerung umfasst, optional wobei die Umlagerung einen Translokationsbruchpunkt umfasst, optional wobei die Zielregion der DNA eine intronische Region der DNA umfasst, die den Translokationsbruchpunkt umfasst;
(c) die strukturelle Variation eine Umlagerung umfasst, wobei die Umlagerung eine VDJ-Rekombination umfasst, optional wobei die Zielregion der DNA eine exonische VDJ-Region umfasst, die die VDJ-Rekombination umfasst, optional wobei:
(i) die Vielzahl von Primern mindestens zwei Primer umfasst, die an ein J-Exon anlagern, optional wobei die Vielzahl von Primern mindestens 3, 4, 5, 6, 7, 8, 9, 10, 11, 12 oder 13 Primer umfasst, die an verschiedene J-Exons anlagern;
(ii) die exonische VDJ-Region ein CDR3 umfasst;
(iii) die exonische VDJ-Region ein Teil einer T-Zell-Rezeptor-Codiersequenz ist;
(iv) wobei das Verfahren ferner das In-Kontakt-Bringen der DNA mit einer Vielzahl von blockierenden Oligonukleotiden umfasst, die die Erweiterung der Primer der Vielzahl blockieren, wenn sie an die Keimbahnsequenz angelagert werden;
(v) die DNA Adapter umfasst, wobei das Verfahren ferner das In-Kontakt-Bringen der DNA mit einem oder mehreren Adapter-blockierenden Oligonukleotiden umfasst, die die Erweiterung der Primer der Vielzahl über einen oder mehrere der Adapter blockieren; und/oder
(vi) die Primer der Vielzahl markiert werden, optional wobei die Primer der Vielzahl mit Biotin markiert werden, optional wobei Komplexe, die erweiterte Primer umfassen, relativ zu nicht erweiterten Primern bevorzugt isoliert werden, optional wobei das bevorzugte Isolieren der Komplexe, die die erweiterten Primer umfassen, das Herunterziehen der Komplexe bei einer Temperatur oberhalb der Schmelztemperatur der nicht erweiterten Primer umfasst; und/oder
(d) die strukturelle Variation eine Umlagerung umfasst, wobei die Umlagerung einen Translokationsbruchpunkt umfasst, wobei das Verfahren das Messen der DNA-Mengen in der Probe, die einen Translokationsbruchpunkt oder eine VDJ-Rekombination umfasst, und deren Vergleich mit Referenzmengen umfasst.

11. Verfahren nach einem der vorhergehenden Ansprüche, umfassend das Bestimmen einer Wahrscheinlichkeit, dass das Subjekt Krebs hat, optional wobei der Krebs ein lymphozytärer Krebs ist, optional wobei der lymphozytäre Krebs eine Leukämie, ein Lymphom oder ein Myelom ist.

12. Verfahren nach einem der vorhergehenden Ansprüche, wobei die Schritte a)-b) an einem ersten Aliquot einer Probe durchgeführt werden, und einer oder mehrere von einem Umwandlungsschritt, einem Aufteilungsschritt und einem Einfangschritt an der Probe oder einem zweiten Aliquot einer Probe durchgeführt werden, optional umfassend:
(a) Einfangen mindestens eines sequenzvariablen Zielbereichssatzes aus der Probe oder dem zweiten Aliquot; und/oder
(b) Einfangen von mindestens einem epigenetischen Zielregionensatz von DNA aus der Probe oder dem zweiten Aliquot, wobei optional:
(i) der epigenetische Zielregionensatz einen hypermethylierungsvariablen Zielregionensatz umfasst, optional wobei der hypermethylierungsvariable Zielregionensatz differenziell methylierte Regionen umfasst;
(ii) der epigenetische Zielregionensatz einen hypomethylierungsvariablen Zielregionensatz umfasst, optional wobei der hypomethylierungsvariable Zielregionensatz Regionen umfasst, die in mindestens einem Gewebetyp einen geringeren Methylierungsgrad aufweisen als der Methylierungsgrad in zellfreier DNA von einem gesunden Subjekt;
(iii) der epigenetische Zielregionensatz einen Methylierungskontrollzielregionensatz umfasst; und/oder
(iv) der epigenetische Zielregionensatz einen fragmentierungsvariablen Zielregionensatz umfasst, optional wobei der fragmentationsvariable Zielregionensatz Transkriptionsstartstellenregionen und/oder CTCF-Bindungsregionen umfasst.

13. Verfahren nach einem der vorhergehenden Ansprüche, umfassend:
(a) Unterwerfen der Probe oder einer Teilprobe davon (wie einem zweiten Aliquot) einem Verfahren, das eine erste Nukleobase in der DNA anders beeinflusst als eine zweite Nukleobase in der DNA der Probe, wobei die erste Nukleobase eine modifizierte oder unmodifizierte Nukleobase ist, die zweite Nukleobase eine modifizierte oder unmodifizierte Nukleobase ist, die sich von der ersten Nukleobase unterscheidet, und die erste Nukleobase und die zweite Nukleobase die gleiche Basenpaarungsspezifität aufweisen, dadurch Herstellen einer umgewandelten Probe, optional umfassend das Sequenzieren der eingefangenen DNA auf eine Weise, die die erste Nukleobase von der zweiten Nukleobase unterscheidet; und/oder
(b) Aufteilen der Probe in eine Vielzahl von Teilproben durch Inkontaktbringen der DNA mit einem Mittel, das eine modifizierte Nukleobase in der DNA erkennt, wobei die Vielzahl eine erste Teilprobe und eine zweite Teilprobe umfasst, wobei die erste Teilprobe DNA mit einer Cytosinmodifikation in einem größeren Anteil als die zweite Teilprobe umfasst, und die von dem Mittel erfasste modifizierte Nukleobase ein modifiziertes Cytosin oder ein Produkt eines Verfahrens ist, das die erste Nukleobase in der DNA anders beeinflusst als die zweite Nukleobase in der DNA der Probe.

14. Verfahren nach Anspruch 12 oder Anspruch 13, wobei:
(a) der Aufteilungsschritt eine Aufteilung auf der Basis des Methylierungsniveaus umfasst, optional wobei das Mittel, das eine modifizierte Nukleobase in der DNA erkennt, ein Methylbindungsreagenz ist, optional wobei: (i) das Methylbindungsreagenz ein Antikörper ist; (ii) das Methylbindungsreagenz spezifisch 5-Methylcytosin erkennt; (iii) das Methylbindungsreagenz auf einem festen Träger immobilisiert ist; und/oder (iv) der Aufteilungsschritt die Immunpräzipitation von methylierter DNA umfasst;
(b) der Aufteilungsschritt die Aufteilung auf der Basis der Bindung an ein Protein umfasst, optional wobei das Protein ein methyliertes Protein, ein acetyliertes Protein, ein unmethyliertes Protein, ein unacetyliertes Protein ist; und/oder optional wobei das Protein ein Histon ist, optional wobei der Aufteilungsschritt das Inkontaktbringen der DNA der Probe mit einem Bindungsreagenz umfasst, das für das Protein spezifisch ist und auf einem festen Träger immobilisiert ist;
(c) das Verfahren umfasst das Aufteilen der Probe in eine Vielzahl von Teilproben durch Inkontaktbringen der DNA mit einem Mittel, das eine modifizierte Nukleobase in der DNA erkennt, wobei die Vielzahl eine erste Teilprobe und eine zweite Teilprobe umfasst, wobei die erste Teilprobe DNA mit einer Cytosinmodifikation in einem größeren Anteil als die zweite Teilprobe umfasst, und die von dem Mittel erfasste modifizierte Nukleobase ein modifiziertes Cytosin oder ein Produkt eines Verfahrens ist, das die erste Nukleobase in der DNA anders beeinflusst als die zweite Nukleobase in der DNA der Probe, wobei das Verfahren das differentielle Markieren und Poolen der ersten Teilprobe und der zweiten Teilprobe umfasst, wobei optional:
(i) der Pool weniger als oder gleich etwa 45%, 40%, 35%, 30%, 25%, 20%, 15%, 10% oder 5% der DNA der zweiten Teilprobe umfasst;
(ii) der Pool etwa 70-90%, etwa 75-85% oder etwa 80% der DNA der zweiten Teilprobe umfasst; oder
(iii) der Pool im Wesentlichen die gesamte DNA der ersten Teilprobe umfasst;
(d) das Verfahren umfasst das Aufteilen der Probe in eine Vielzahl von Teilproben durch Inkontaktbringen der DNA mit einem Mittel, das eine modifizierte Nukleobase in der DNA erkennt, wobei die Vielzahl eine erste Teilprobe und eine zweite Teilprobe umfasst, wobei die erste Teilprobe DNA mit einer Cytosinmodifikation in einem größeren Anteil als die zweite Teilprobe umfasst, und die von dem Mittel erfasste modifizierte Nukleobase ein modifiziertes Cytosin oder ein Produkt eines Verfahrens ist, das die erste Nukleobase in der DNA anders beeinflusst als die zweite Nukleobase in der DNA der Probe, wobei die Vielzahl von Teilproben eine dritte Teilprobe umfasst, die DNA mit einer Cytosinmodifikation in einem größeren Anteil als die zweite Teilprobe, aber in einem geringeren Anteil als die erste Teilprobe umfasst, wobei optional:
(i) das Verfahren ferner das differentielle Markieren der dritten Teilprobe umfasst; und/oder
(ii) die erste, zweite und dritte Teilprobe kombiniert werden, nachdem die erste Teilprobe einem Verfahren unterzogen wurde, das die erste Nukleobase in der DNA anders beeinflusst als die zweite Nukleobase in der DNA der ersten Teilprobe, optional wobei die erste, zweite und dritte Teilprobe in derselben Sequenzierzelle sequenziert werden; und/oder
(e) das Verfahren das Unterwerfen der Probe oder einer Teilprobe davon (wie einer zweiten Aliquote) einem Verfahren umfasst, das eine erste Nukleobase in der DNA anders beeinflusst als eine zweite Nukleobase in der DNA der Probe, wobei die erste Nukleobase eine modifizierte oder unmodifizierte Nukleobase ist, die zweite Nukleobase eine modifizierte oder unmodifizierte Nukleobase ist, die sich von der ersten Nukleobase unterscheidet, und die erste Nukleobase und die zweite Nukleobase die gleiche Basenpaarungsspezifität aufweisen, wodurch eine umgewandelte Probe hergestellt wird, wobei:
(i) das Verfahren, dem die erste Teilprobe unterzogen wird, die Basenpaarungsspezifität der ersten Nukleobase verändert, ohne die Basenpaarungsspezifität der zweiten Nukleobase wesentlich zu verändern;
(ii) die erste Nukleobase ein modifiziertes oder unmodifiziertes Cytosin ist und die zweite Nukleobase ein modifiziertes oder unmodifiziertes Cytosin ist;
(iii) die erste Nukleobase ein unmodifiziertes Cytosin (C) umfasst, optional wobei die zweite Nukleobase 5-Methylcytosin (mC) umfasst; und/oder
(iv) die erste Nukleobase mC umfasst, optional wobei die zweite Nukleobase 5-Hydroxymethylcytosin (5hmC) oder C umfasst.

15. Verfahren nach einem der vorhergehenden Ansprüche, wobei:
(a) das Nachweisen das Erzeugen einer Vielzahl von Sequenzierlesungen umfasst; und das Verfahren ferner das Abbilden der Vielzahl von Sequenzlesungen auf eine oder mehrere Referenzsequenzen umfasst, um abgebildete Sequenzlesungen zu erzeugen, und das Verarbeiten der abgebildeten Sequenzlesungen, um die Wahrscheinlichkeit zu bestimmen, dass das Subjekt Krebs hat; und/oder
(b) die Probe von einem Subjekt stammt, bei dem zuvor ein Krebs diagnostiziert wurde und das eine oder mehrere frühere Krebsbehandlungen erhalten hat, optional wobei die DNA zu einem oder mehreren vorgewählten Zeitpunkten nach der einen oder den mehreren früheren Krebsbehandlungen erhalten wird, und Sequenzieren der DNA in der mit Primer erweiterten Probe, wodurch ein Sequenzinformationensatz erzeugt wird, optional ferner umfassend das Nachweisen der Anwesenheit oder Abwesenheit von DNA, die von einer Tumorzelle stammt oder abgeleitet ist, zu einem vorgewählten Zeitpunkt unter Verwendung des Satzes von Sequenzinformationen, optional ferner umfassend das Bestimmen eines Krebsrezidiv-Scores, der das Vorhandensein oder die Abwesenheit der DNA, die von der Tumorzelle stammt oder abgeleitet ist, für da Testsubjekt anzeigt, optional ferner umfassend das Bestimmen eines Krebsrezidivstatus basierend auf dem Krebsrezidiv-Score, wobei der Krebsrezidivstatus des Testsubjekts als Risiko für ein Krebsrezidiv bestimmt wird, wenn ein Krebsrezidiv-Score bestimmt wird, der bei oder über einem vorbestimmten Schwellenwert liegt, oder der Krebsrezidivstatus des Testsubjekts als mit einem geringeren Risiko für ein Krebsrezidiv behaftet bestimmt wird, wenn der Krebsrezidiv-Score unter dem vorbestimmten Schwellenwert liegt, optional ferner umfassend das Vergleichen des Krebsrezidivwertes des Testsubjekts mit einem vorbestimmten Krebsrezidivschwellenwert, wobei das Testsubjekt als Kandidat für eine nachfolgende Krebsbehandlung klassifiziert wird, wenn der Krebsrezidivwert über dem Krebsrezidivschwellenwert liegt, oder nicht als Kandidat für eine nachfolgende Krebsbehandlung klassifiziert wird, wenn der Krebsrezidivwert unter dem Krebsrezidivschwellenwert liegt.

## Revendications

1. Procédé d'analyse d'ADN dans un échantillon, l'ADN comprenant de régions introniques, le procédé comprenant :
(a) la mise en contact de l'ADN avec une pluralité d'amorces, dans lequel la pluralité d'amorces comprend au moins deux amorces qui s'annellent dans une orientation parallèle à une région cible, pour ainsi produire de l'ADN annelé par amorce ; et
(b) la mise en contact de l'ADN annelé par amorce avec une ADN polymérase et des triphosphates de désoxynucléosides, dans lequel l'ADN polymérase est une ADN polymérase négative au déplacement de brin, négative à l'exonucléase en 5' à 3', pour ainsi produire un échantillon étendu par amorce, dans lequel pour au moins la moitié des amorces, lorsqu'elles sont annelées à une région cible de type sauvage, l'extension est bloquée par une amorce amont ; et
(c) la détection d'une présence ou d'une absence d'une molécule d'ADN qui comprend une variation structurelle, dans lequel la détection comprend le séquençage d'ADN dans l'échantillon étendu par amorce.

2. Procédé selon la revendication 1, comprenant en outre :
i) la ligature d'adaptateurs sur l'ADN, pour ainsi produire de l'ADN ligaturé par adaptateur ;
ii) l'amplification de l'ADN ligaturé par adaptateur, pour ainsi produire de l'ADN ligaturé par adaptateur amplifié, dans lequel au moins une portion de l'ADN ligaturé par adaptateur amplifié est l'ADN mis en contact à l'étape (a) ;
iii) l'enrichissement ou la capture d'ADN qui s'annelle à un produit étendu par amorce dans l'échantillon étendu par amorce ; et
iv) l'amplification de l'ADN enrichi ou capturé, puis le séquençage de l'ADN enrichi ou capturé amplifié,
comprenant éventuellement en outre : (i) la capture de régions cibles à séquence variable à partir de l'ADN ligaturé par adaptateur et l'amplification et le séquençage des régions cibles à séquence variable, et/ou (ii) la capture de régions cibles épigénétiques à partir de l'ADN ligaturé par adaptateur et l'amplification et le séquençage des régions cibles épigénétiques.

3. Procédé selon la revendication 1 ou la revendication 2, dans lequel :
(a) la région cible comprend : (i) une région intronique, (ii) une région VDJ exonique ; et/ou (iii) une région intergénique ; et/ou
(b) l'extension d'au moins 70 %, d'au moins 80 %, ou d'au moins 90 % des amorces annelées à une région cible de type sauvage est bloquée par une amorce aval.

4. Procédé selon l'une quelconque des revendications précédentes, dans lequel :
(a) l'ADN comprend une pluralité de régions cibles introniques et la pluralité d'amorces comprend au moins deux amorces qui s'annellent dans une orientation parallèle à chacune de la pluralité de régions cibles introniques ;
(b) l'ADN comprend une région VDJ exonique et la pluralité d'amorces comprend au moins deux amorces qui s'annellent dans une orientation parallèle à la région cible VDJ exonique ;
(c) l'ADN comprend des molécules d'ADN acellulaire (ADNcf) ;
(d) l'ADN est prélevé sur un sujet test ;
(e) l'ADN comprend de l'ADN prélevé sur un échantillon de tissu d'un sujet test, éventuellement dans lequel l'échantillon de tissu est une biopsie, un prélèvement par aspiration à l'aiguille fine, ou un échantillon de tissu inclus en paraffine et fixé au formol ; et/ou
(f) l'ADN comprend une pluralité de régions cibles introniques, et dans lequel la pluralité d'amorces comprend au moins deux amorces qui s'annellent à chacune de la pluralité de régions cibles introniques, éventuellement dans lequel la pluralité de régions cibles introniques comprend au moins 3, 5, 10, 15, ou 20 régions cibles introniques.

5. Procédé selon l'une quelconque des revendications précédentes, dans lequel :
(a) la pluralité d'amorces comprend au moins une amorce qui s'annelle à une région de jonction exon-exon de l'ADN ;
(b) chacune de la pluralité d'amorces : (i) comprend au moins 20 nucléosides liés ; (ii) est constituée par 20 à 60 nucléosides liés ; (iii) est constituée par 25 à 60 nucléosides liés ; (iv) comprend 30 à 40 nucléosides liés ; ou (v) est constituée par 30 à 40 nucléosides liés ;
(c) chacune de la pluralité d'amorces comprend un marqueur, éventuellement dans lequel : (i) le marqueur est une fraction de capture ; et/ou (ii) le marqueur est la biotine ; et/ou
(d) l'ADN annelé par amorce comprend des portions simple brin de 0 à 50 nucléosides liés ou de 0 à 10 nucléosides liés entre au moins une portion de la paire ou de paires d'amorces annelées adjacentes.

6. Procédé selon l'une quelconque des revendications précédentes, dans lequel :
(a) l'ADN polymérase est une ADN polymérase haute fidélité Q5 et/ou une ADN polymérase positive à l'exonucléase en 3' à 5' ; et/ou
(b) les triphosphates de désoxynucléosides comprennent un triphosphate de désoxynucléoside modifié, éventuellement dans lequel
(i) le triphosphate de désoxynucléoside modifié comprend un marqueur ;
(ii) le triphosphate de désoxynucléoside modifié comprend une fraction de capture ;
(iii) le triphosphate de désoxynucléoside modifié comprend une biotine ;
(iv) les triphosphates de désoxynucléosides comprennent le triphosphate de désoxynucléoside modifié et la version non modifiée du triphosphate de désoxynucléoside modifié, éventuellement dans lequel le rapport entre les versions modifiée:non modifiée du triphosphate de désoxynucléoside modifié est de 1:1 à 1:100 ;
(v) le triphosphate de désoxynucléoside modifié est le triphosphate de désoxycytidine modifié ;
(vi) le triphosphate de désoxynucléoside modifié est le triphosphate de désoxycytidine biotinylé, le triphosphate de désoxyadénosine biotinylé, ou le triphosphate de désoxyuridine biotinylé ; et/ou
(vii) les triphosphates de désoxynucléosides comprennent du triphosphate de désoxyadénosine non modifié, du triphosphate de désoxyguanosine non modifié, du triphosphate de désoxycytidine non modifiée, et du triphosphate de thymidine non modifié.

7. Procédé selon l'une quelconque des revendications précédentes, dans lequel :
(a) la détection de tout produit étendu par amorce comprend la séparation d'acides nucléiques dans l'échantillon étendu par amorce par taille ;
(b) la détection de tout produit étendu par amorce comprend la mise en contact de l'échantillon étendu par amorce avec un réactif de capture, éventuellement dans lequel le réactif de capture comprend de la streptavidine ;
(c) la détection de tout produit étendu par amorce comprend l'amplification d'acides nucléiques dans l'échantillon étendu par amorce, éventuellement dans lequel la détection de tout produit étendu par amorce comprend le séquençage de l'ADN dans l'échantillon étendu par amorce après l'amplification d'acides nucléiques dans l'échantillon étendu par amorce ;
(d) les amorces sont résistantes à l'exonucléolyse en 5', et le procédé comprend en outre la mise en contact de l'échantillon étendu par amorce avec une exonucléase en 5' à 3' avant l'étape de détection, éventuellement dans lequel les amorces résistantes à l'exonucléolyse en 5' comprennent une ou plusieurs liaisons phosphorothioate ; et/ou
(e) le procédé comprend en outre la ligature d'adaptateurs contenant un code à barres à l'ADN avant la mise en contact de l'ADN avec des amorces.

8. Procédé selon l'une quelconque des revendications précédentes, comprenant en outre la mise en contact de l'ADN avec un ou plusieurs oligonucléotides de blocage, éventuellement dans lequel :
(a) les un ou plusieurs oligonucléotides de blocage comprennent un ou plusieurs oligonucléotides de blocage qui s'annellent à un adaptateur ou à une région exonique de l'ADN ;
(b) les un ou plusieurs oligonucléotides de blocage comprennent un oligonucléotide de blocage qui s'annelle à un adaptateur 3' ;
(c) les un ou plusieurs oligonucléotides de blocage comprennent un oligonucléotide de blocage qui s'annelle à un exon adjacent à l'extrémité 5' d'une région intronique de l'ADN ;
(d) les un ou plusieurs oligonucléotides de blocage comprennent une amorce de blocage qui s'annelle à un adaptateur 5' ;
(e) les un ou plusieurs oligonucléotides de blocage comprennent des oligonucléotides de blocage qui s'annellent à un adaptateur ou à une région intronique de l'ADN au sein ou à côté d'une région VDJ exonique, éventuellement dans lequel les un ou plusieurs oligonucléotides de blocage comprennent un oligonucléotide de blocage qui s'annelle à une région intronique J ;
(f) chaque oligonucléotide de blocage est complémentaire d'un adaptateur ou d'une région adjacente à une région cible de l'ADN ;
(g) les oligonucléotides de blocage comprennent un nucléoside modifié à l'extrémité 3' qui bloque l'extension des oligonucléotides de blocage par l'ADN polymérase, éventuellement dans lequel le nucléoside modifié à l'extrémité 3' des oligonucléotides de blocage est un nucléoside abasique, un nucléoside inversé, un nucléoside phosphorylé, ou un didésoxynucléoside, éventuellement dans lequel : (i) le nucléoside inversé est la thymidine inversée ; ou (ii) le didésoxynucléoside est la didésoxycytidine ; et/ou
(h) les oligonucléotides de blocage comprennent un nucléoside modifié à l'extrémité 3' qui n'est pas résistant à l'exonucléolyse 3', éventuellement dans lequel le nucléoside modifié à l'extrémité 3' des oligonucléotides de blocage est un nucléoside abasique, un nucléoside inversé, un nucléoside phosphorylé, ou un didésoxynucléoside, éventuellement dans lequel :
(i) le nucléoside inversé est la thymidine inversée ; ou (ii) le didésoxynucléoside est la didésoxycytidine.

9. Procédé selon l'une quelconque des revendications précédentes, dans lequel :
(a) la pluralité d'amorces comprend au moins deux amorces comprenant une portion à l'extrémité 5' qui n'est pas complémentaire de l'ADN, éventuellement dans lequel la portion à l'extrémité 5' des amorces qui n'est pas complémentaire de l'ADN comprend une fraction de capture, éventuellement dans lequel la fraction de capture est la biotine ; et/ou
(b) la détection comprend la mise en contact de l'échantillon étendu par amorce avec un réactif de capture et l'amplification d'acides nucléiques liés au réactif de capture, éventuellement dans lequel le réactif de capture comprend de la streptavidine.

10. Procédé selon l'une quelconque des revendications précédentes, dans lequel :
(a) la variation structurelle comprend un réarrangement, une insertion, ou une délétion ;
(b) la variation structurelle comprend un réarrangement, éventuellement dans lequel le réarrangement comprend un point de cassure de translocation, éventuellement dans lequel la région cible de l'ADN comprend une région intronique de l'ADN comprenant le point de cassure de translocation ;
(c) la variation structurelle comprend un réarrangement dans lequel le réarrangement comprend une recombinaison VDJ, éventuellement dans lequel la région cible de l'ADN comprend une région VDJ exonique comprenant la recombinaison VDJ, éventuellement dans lequel :
(i) la pluralité d'amorces comprend au moins deux amorces qui s'annellent à un exon J, éventuellement dans lequel la pluralité d'amorces comprend au moins 3, 4, 5, 6, 7, 8, 9, 10, 11, 12, ou 13 amorces qui s'annellent à des exons J différents ;
(ii) la région VDJ exonique comprend une CDR3 ;
(iii) la région VDJ exonique fait partie d'une séquence codante de récepteur de lymphocytes T ;
(iv) dans lequel le procédé comprend en outre la mise en contact de l'ADN avec une pluralité d'oligonucléotides de blocage qui bloquent l'extension d'amorces de la pluralité lorsqu'elles sont annelées à une séquence de lignée germinale :
(v) l'ADN comprend des adaptateurs, le procédé comprenant en outre la mise en contact de l'ADN avec un ou plusieurs oligonucléotides de blocage d'adaptateur qui bloquent l'extension d'amorces de la pluralité sur un ou plusieurs des adaptateurs ; et/ou
(vi) les amorces de la pluralité sont marquées, éventuellement dans lequel les amorces de la pluralité sont marquées avec de la biotine, éventuellement dans lequel des complexes comprenant des amorces étendues sont isolés préférentiellement par rapport à des amorces non étendues, éventuellement dans lequel l'isolement préférentiel des complexes comprenant les amorces étendues comprend la mise à froid des complexes à une température au-dessus de la température de fusion des amorces non étendues ; et/ou
(d) la variation structurelle comprend un réarrangement, dans lequel le réarrangement comprend un point de cassure de translocation, dans lequel le procédé comprend la mesure des taux d'ADN dans l'échantillon comprenant un point de cassure de translocation ou une recombinaison VDJ et leur comparaison à des taux de référence.

11. Procédé selon l'une quelconque des revendications précédentes, comprenant la détermination d'une probabilité pour le sujet d'avoir un cancer, éventuellement dans lequel le cancer est un cancer lymphocytaire, éventuellement dans lequel le cancer lymphocytaire est une leucémie, un lymphome, ou un myélome.

12. Procédé selon l'une quelconque des revendications précédentes, dans lequel les étapes a) à b) sont réalisées sur une première aliquote d'un échantillon, et une ou plusieurs parmi une étape de conversion, une étape de division, et une étape de capture sont réalisées sur l'échantillon ou une deuxième aliquote d'un échantillon, comprenant éventuellement :
(a) la capture d'au moins un ensemble de régions cibles à séquence variable à partir de l'échantillon ou de la deuxième aliquote ; et/ou
(b) la capture d'au moins un ensemble de régions cibles épigénétiques d'ADN à partir de l'échantillon ou de la deuxième aliquote, éventuellement dans lequel :
(i) l'ensemble de régions cibles épigénétiques comprend un ensemble de régions cibles à hyperméthylation variable, éventuellement dans lequel l'ensemble de régions cibles à hyperméthylation variable comprend des régions méthylées de manière différentielle ;
(ii) l'ensemble de régions cibles épigénétiques comprend un ensemble de régions cibles à hyperméthylation variable, éventuellement dans lequel l'ensemble de régions cibles à hyperméthylation variable comprend des régions ayant un degré de méthylation dans au moins un type de tissu inférieur au degré de méthylation dans l'ADN acellulaire issu d'un sujet sain ;
(iii) l'ensemble de régions cibles épigénétiques comprend un ensemble de régions cibles à méthylation témoin ; et/ou
(iv) l'ensemble de régions cibles épigénétiques comprend un ensemble de régions cibles à fragmentation variable, éventuellement dans lequel l'ensemble de régions cibles à fragmentation variable comprend des régions de site de début de transcription et/ou des régions de liaison CTCF.

13. Procédé selon l'une quelconque des revendications précédentes, comprenant :
(a) la soumission de l'échantillon ou d'un sous-échantillon de celui-ci (tel qu'une deuxième aliquote) à une procédure qui affecte une première nucléobase dans l'ADN différemment d'une deuxième nucléobase dans l'ADN de l'échantillon, dans lequel la première nucléobase est une nucléobase modifiée ou non modifiée, la deuxième nucléobase est une nucléobase modifiée ou non modifiée différente de la première nucléobase, et la première nucléobase et la deuxième nucléobase ont la même spécificité de mise en paires de base, pour ainsi produire un échantillon converti, comprenant éventuellement le séquençage de l'ADN capturé d'une manière qui distingue la première nucléobase de la deuxième nucléobase ; et/ou
(b) la division de l'échantillon en une pluralité de sous-échantillons par mise en contact de l'ADN avec un agent qui reconnaît une nucléobase modifiée dans l'ADN, la pluralité comprenant un premier sous-échantillon et un deuxième sous-échantillon, dans lequel le premier sous-échantillon comprend de l'ADN avec une modification de cytosine dans une proportion supérieure au deuxième sous-échantillon, et la nucléobase modifiée reconnue par l'agent est une cytosine modifiée ou un produit d'une procédure qui affecte la première nucléobase dans l'ADN différemment de la deuxième nucléobase dans l'ADN de l'échantillon.

14. Procédé selon la revendication 12 ou la revendication 13, dans lequel :
(a) l'étape de division comprend la division sur la base du taux de méthylation, éventuellement dans lequel l'agent qui reconnaît une nucléobase modifiée dans l'ADN est un réactif de liaison méthyle, éventuellement dans lequel : (i) le réactif de liaison méthyle est un anticorps ; (ii) le réactif de liaison méthyle reconnaît spécifiquement la 5-méthylcytosine ; (iii) le réactif de liaison méthyle est immobilisé sur un support solide ; et/ou (iv) l'étape de division comprend une immunoprécipitation d'ADN méthylé ;
(b) l'étape de division comprend la division sur la base d'une liaison à une protéine, éventuellement dans lequel la protéine est une protéine méthylée, une protéine acétylée, une protéine non méthylée, une protéine non acétylée ; et/ou éventuellement dans lequel la protéine est une histone, éventuellement dans lequel l'étape de division comprend la mise en contact de l'ADN de l'échantillon avec un réactif de liaison qui est spécifique de la protéine et est immobilisé sur un support solide ;
(c) le procédé comprend la division de l'échantillon en une pluralité de sous-échantillons par mise en contact de l'ADN avec un agent qui reconnaît une nucléobase modifiée dans l'ADN, la pluralité comprenant un premier sous-échantillon et un deuxième sous-échantillon, dans lequel le premier sous-échantillon comprend de l'ADN avec une modification de cytosine dans une proportion supérieure au deuxième sous-échantillon, et la nucléobase modifiée reconnue par l'agent est une cytosine modifiée ou un produit d'une procédure qui affecte la première nucléobase dans l'ADN différemment de la deuxième nucléobase dans l'ADN de l'échantillon, dans lequel le procédé comprend l'étiquetage différentiel et le regroupement du premier sous-échantillon et du deuxième sous-échantillon, éventuellement dans lequel :
(i) le groupe comprend environ 45 %, 40 %, 35 %, 30 %, 25 %, 20 %, 15 %, 10 %, ou 5 % ou moins de l'ADN du deuxième sous-échantillon ;
(ii) le groupe comprend environ 70 à 90 %, environ 75 à 85 %, ou environ 80 % de l'ADN du deuxième sous-échantillon ; ou
(iii) le groupe comprend sensiblement tout l'ADN du premier sous-échantillon ;
(d) le procédé comprend la division de l'échantillon en une pluralité de sous-échantillons par mise en contact de l'ADN avec un agent qui reconnaît une nucléobase modifiée dans l'ADN, la pluralité comprenant un premier sous-échantillon et un deuxième sous-échantillon, dans lequel le premier sous-échantillon comprend de l'ADN avec une modification de cytosine dans une proportion supérieure au deuxième sous-échantillon, et la nucléobase modifiée reconnue par l'agent est une cytosine modifiée ou un produit d'une procédure qui affecte la première nucléobase dans l'ADN différemment de la deuxième nucléobase dans l'ADN de l'échantillon, dans lequel la pluralité de sous-échantillons comprend un troisième sous-échantillon, qui comprend de l'ADN avec une modification de cytosine dans une proportion supérieure au deuxième sous-échantillon, mais dans une proportion inférieure au premier sous-échantillon, éventuellement dans lequel :
(i) le procédé comprend en outre l'étiquetage différentiel du troisième sous-échantillon ; et/ou
(ii) les premier, deuxième, et troisième sous-échantillons sont combinés après soumission du premier sous-échantillon à une procédure qui affecte la première nucléobase dans l'ADN différemment de la deuxième nucléobase dans l'ADN du premier sous-échantillon, éventuellement dans lequel les premier, deuxième, et troisième sous-échantillons sont séquencés dans la même cellule de séquençage ; et/ou
(e) le procédé comprend la soumission de l'échantillon ou d'un sous-échantillon de celui-ci (tel qu'une deuxième aliquote) à une procédure qui affecte une première nucléobase dans l'ADN différemment d'une deuxième nucléobase dans l'ADN de l'échantillon, dans lequel la première nucléobase est une nucléobase modifiée ou non modifiée, la deuxième nucléobase est une nucléobase modifiée ou non modifiée différente de la première nucléobase, et la première nucléobase et la deuxième nucléobase ont la même spécificité de mise en paires de base, pour ainsi produire un échantillon converti, dans lequel :
(i) la procédure à laquelle le premier sous-échantillon est soumis altère la spécificité de mise en paires de base de la première nucléobase sans altérer sensiblement la spécificité de mise en paires de base de la deuxième nucléobase ;
(ii) la première nucléobase est une cytosine modifiée ou non modifiée et la deuxième nucléobase est une cytosine modifiée ou non modifiée ;
(iii) la première nucléobase comprend une cytosine non modifiée (C), éventuellement dans lequel la deuxième nucléobase comprend une 5-méthylcytosine (mC), et/ou
(iv) la première nucléobase comprend mC, éventuellement dans lequel la deuxième nucléobase comprend une 5-hydroxyméthylcytosine (5hmC) ou C.

15. Procédé selon l'une quelconque des revendications précédentes, dans lequel :
(a) la détection comprend la génération d'une pluralité de lectures de séquence ; et le procédé comprend en outre le mappage de la pluralité de lectures de séquence à une ou plusieurs séquences de référence pour générer des lectures de séquence mappées, et le traitement des lectures de séquence mappées pour déterminer la probabilité pour le sujet d'avoir un cancer ; et/ou
(b) l'échantillon est issu d'un sujet test qui a précédemment reçu un diagnostic de cancer et reçu un ou plusieurs traitements précédents pour le cancer, éventuellement dans lequel l'ADN est obtenu à un ou plusieurs points temporels présélectionnés à la suite des un ou plusieurs traitements précédents pour le cancer, et du séquençage d'ADN dans l'échantillon étendu par amorce, moyennant quoi un ensemble d'informations de séquence est produit, comprenant éventuellement en outre la détection d'une présence ou d'une absence d'ADN provenant ou dérivé d'une cellule tumorale à un point temporel présélectionné à l'aide de l'ensemble d'informations de séquence, comprenant éventuellement en outre la détermination d'un score de récidive de cancer qui est indicatif de la présence ou de l'absence de l'ADN provenant ou dérivé de la cellule tumorale pour le sujet test, comprenant éventuellement en outre la détermination d'un statut de récidive de cancer sur la base du score de récidive de cancer, dans lequel le statut de récidive de cancer du sujet test est déterminé comme présentant un risque de récidive de cancer lorsqu'il est déterminé qu'un score de récidive de cancer est égal ou supérieur à un seuil prédéterminé ou le statut de récidive de cancer du sujet test est déterminé comme présentant un risque plus bas de récidive de cancer lorsque le score de récidive de cancer est inférieur au seuil prédéterminé, comprenant éventuellement en outre la comparaison du score de récidive de cancer du sujet test avec un seuil de récidive de cancer prédéterminé, dans lequel le sujet test est classé comme candidat à un traitement ultérieur pour le cancer lorsque le score de récidive de cancer est supérieur au seuil de récidive de cancer ou non-candidat à un traitement ultérieur pour le cancer lorsque le score de récidive de cancer est inférieur au seuil de récidive de cancer.
